# EUROPEAN PATENT APPLICATION

(11) **EP 4 403 573 A1**
(43) Date of publication of application: **24.07.2024**
(21) Application number: 22869304.0
(22) Date of filing: 15.09.2022
(51) Int. Cl.: C07K 16/28, C07K 7/06, C12N 15/13, A61K 39/395, A61P 35/00

(54) **PHARMACEUTICAL COMPOSITION COMPRISING ANTI-PVRIG/TIGIT BISPECIFIC ANTIBODY**

(30) Priority: 15.09.2021 CN 202111078172
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: YANG, Jianjian, Lianyungang, Jiangsu 222047 (CN); TIAN, Chenmin, Lianyungang, Jiangsu 222047 (CN)
(74) Representative: Regimbeau
(86) International application number: PCT/CN2022/118942
(87) International publication number: WO 2023/040935

(57) **Abstract**

A pharmaceutical composition comprising an anti-PVRIG/TIGIT bispecific antibody.

## Description

The present application claims priority to Chinese Patent Application No. 202111078172.7 filed on Sep. 15, 2021.

### TECHNICAL FIELD

The present disclosure belongs to the field of pharmaceutical formulations and particularly relates to a pharmaceutical composition comprising a bispecific antibody and use thereof as a medicament.

### BACKGROUND

The statements herein merely provide background information related to the present disclosure and may not necessarily constitute the prior art.

PVRIG, also known as CD112R, is a protein expressed on the cell surface and belongs to the B7/CD28 superfamily, just like TIGIT, CD96, CD226, etc., and it plays an important role in the immune system.

PVRIG comprises an extracellular region, a transmembrane region, and an intracellular region. When its ligand PVRL2 (also known as CD112) binds to PVRIG, the ITIM domain of PVRIG intracellular region will be activated, thus enabling PVRIG to play the role of immunosuppression.

PVRIG is mainly expressed on the surface of CD4+ T cells, CD8+ T cells, and NK cells. PVRIG and its ligand PVRL2 are highly expressed in many solid tumors, including lung cancer, breast cancer, ovarian cancer, renal cancer, gastric cancer, endometrial cancer, head and neck cancer, and the like. The expression of PVRIG in these cancers is highly correlated with TIGIT and PD-1. Similar to PD-1 and TIGIT, PVRIG-positive T cells are also Eomes-positive and Tbet-negative, indicating that PVRIG is associated with T cell depletion. Thus, PVRIG may represent a new immune checkpoint in addition to PD-1 and TIGIT and plays a redundancy role. *In vitro* cell assays and mouse models show that the knockout or inhibition of mouse PVRIG can effectively inhibit the growth of tumors and generate coordination action with PD-1 and TIGIT inhibitors.

TIGIT is highly expressed on lymphocytes, including tumor-infiltrating lymphocytes (TILs) and Treg infiltrating different types of tumors. It has been proved that engagement of TIGIT signaling to its cognate ligand PVR (also known as CD155) directly inhibits NK cell cytotoxicity through its cytoplasmic ITIM domain. PVR is also widely expressed in tumors, suggesting that the TIGIT-PVR signaling axis may be a dominant immune escape mechanism for cancer.

However, no anti-PVRIG/TIGIT bispecific antibody medicament has entered the clinic trial phase at present. There remains a lack in the prior art of formulations for high-avidity, high-selectivity, and high-bioactivity anti-PVRIG/TIGIT bispecific antibodies capable of inhibiting cancer or tumor growth *in vivo.*

### SUMMARY

The present disclosure provides a pharmaceutical composition comprising an anti-PVRIG/TIGIT bispecific antibody, and the composition has therapeutic activity. In addition, the composition may also have the advantages of good stability and the like. In some embodiments, the present disclosure provides a pharmaceutical composition comprising an anti-PVRIG/TIGIT bispecific antibody and a buffer, wherein the anti-PVRIG/TIGIT bispecific antibody comprises a first antigen-binding domain specifically binding to PVRIG and a second antigen-binding domain specifically binding to TIGIT, and the buffer is a histidine buffer or an acetate buffer.

In some embodiments, the buffer is a histidine-histidine hydrochloride buffer or an acetic acid-sodium acetate buffer.

In some specific embodiments, the buffer is a histidine-histidine hydrochloride buffer.

In some embodiments, provided is the pharmaceutical composition according to any of the above, wherein the pharmaceutical composition has a pH of 5.0-6.5. In some embodiments, the pharmaceutical composition has a pH of 5.5-6.5. In some embodiments, the pharmaceutical composition has a pH of 5.5-6.0. In some embodiments, the pharmaceutical composition has a pH of about 6.0. When a point value is referred to in the present disclosure, it should be understood that the point value includes an error range. This error range is due to factors such as laboratory environment, personnel operations, instruments, methodology, measurement errors, and the like. Taking the pH as an example, when the measurement is about 6.0, it should be understood that an error range is included. As an example, "about 6.0" represents 6.0±0.2 when the formulation is measured using an industrial pH meter.

In some embodiments, the pharmaceutical composition has a pH of 5.0-5.5. In some embodiments, the pharmaceutical composition has a pH of about 5.5.

In some embodiments, the pharmaceutical composition has a pH of 5.0, 5.1, 5.2, 5.3, 5.4, 5.5, 5.6, 5.7, 5.8, 5.9, 6.0, 6.1, 6.2, 6.3, 6.4, or 6.5, or any range between these point values.

Generally, the pH of the pharmaceutical composition obtained by replacing the buffer is almost consistent with the pH of the buffer. Also, it is well known to those skilled in the art that in the process of pharmaceutical formulation, there may sometimes be a pH drift, but the pH drift of the pharmaceutical formulation is generally small (within a range of ±0.3). In some embodiments, the pH drift of the pharmaceutical formulation is within a range of ±0.1.

In some embodiments, provided is the pharmaceutical composition according to any of the above, wherein the anti-PVRIG/TIGIT bispecific antibody is at a concentration of 1 mg/mL to 150 mg/mL. In some embodiments, the anti-PVRIG/TIGIT bispecific antibody is at a concentration of 1 mg/mL to 100 mg/mL. In some embodiments, the anti-PVRIG/TIGIT bispecific antibody is at a concentration of 40 mg/mL to 100 mg/mL. In some embodiments, the anti-PVRIG/TIGIT bispecific antibody is at a concentration of 50 mg/mL to 100 mg/mL. In some embodiments, the anti-PVRIG/TIGIT bispecific antibody is at a concentration of 40 mg/mL to 60 mg/mL. In some embodiments, the anti-PVRIG/TIGIT bispecific antibody is at a concentration of 100 mg/mL. In some embodiments, the anti-PVRIG/TIGIT bispecific antibody is at a concentration of 100±8 mg/mL. In some embodiments, the anti-PVRIG/TIGIT bispecific antibody is at a concentration of 50 mg/mL. In some embodiments, the anti-PVRIG/TIGIT bispecific antibody is at a concentration of 50±5 mg/mL. In some embodiments, the anti-PVRIG/TIGIT bispecific antibody is at a concentration of 1 mg/mL, 10 mg/mL, 20 mg/mL, 30 mg/mL, 40 mg/mL, 45 mg/mL, 50 mg/mL, 55 mg/mL, 60 mg/mL, 65 mg/mL, 70 mg/mL, 80 mg/mL, 90 mg/mL, 100 mg/mL, 110 mg/mL, 120 mg/mL, 130 mg/mL, 140 mg/mL, or 150 mg/mL, or any range between these point values.

In some embodiments, provided is the pharmaceutical composition according to any of the above, wherein the pharmaceutical composition comprises a surfactant. In some embodiments, the surfactant is a nonionic surfactant. In some embodiments, the surfactant is selected from the group consisting of poloxamer (e.g., poloxamer 188), polysorbate (e.g., polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymers of ethylene and propylene glycol, and the like. In some embodiments, the surfactant is polysorbate or poloxamer. In some embodiments, the surfactant is polysorbate 80, polysorbate 20, or poloxamer 188. In some embodiments, the surfactant is polysorbate 80.

In some embodiments, provided is the pharmaceutical composition according to any of the above, wherein the surfactant is at a concentration of 0.05 mg/mL to 1.0 mg/mL. In some embodiments, the surfactant is at a concentration of 0.2 mg/mL to 0.6 mg/mL. In some embodiments, the surfactant is at a concentration of 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL, or any range between these point values. In some embodiments, the surfactant is at a concentration of 0.4 mg/mL. In some embodiments, the surfactant is at a concentration of 0.4±0.1 mg/mL.

In some embodiments, the surfactant is 0.4 mg/mL polysorbate 80.

In some embodiments, provided is the pharmaceutical composition according to any of the above, comprising an osmotic pressure regulator. In some embodiments, the osmotic pressure regulator is a sugar (including monosaccharide, disaccharide, trisaccharide, polysaccharide, sugar alcohol, reducing sugar, non-reducing sugar, and the like), an amino acid (including arginine, glycine, cysteine, histidine, and the like) or a salt (sodium chloride, potassium chloride, calcium chloride, and the like). In some embodiments, the osmotic pressure regulator is a sugar, and the sugar is selected from the group consisting of glucose, sucrose, trehalose, lactose, fructose, maltose, dextran, glycerin, erythritol, glycerol, arabitol, sylitol, sorbitol (also known as sorbol), mannitol, melibiose, melezitose, raffinose, mannotriose, stachyose, maltose, lactulose, maltulose, glucitol, maltitol, lactitol, and iso-maltulose. In some embodiments, the osmotic pressure regulator is selected from one or more of the group consisting of sucrose, trehalose, sorbitol, arginine, glycine and sodium chloride. In some embodiments, the osmotic pressure regulator is a non-reducing disaccharide. In some embodiments, the osmotic pressure regulator is trehalose or sucrose. In some embodiments, the osmotic pressure regulator is sucrose.

In some embodiments, provided is the pharmaceutical composition according to any of the above, wherein the osmotic pressure regulator is at a concentration of 70 mg/mL to 100 mg/mL. In some embodiments, the osmotic pressure regulator is at a concentration of 75 mg/mL to 100 mg/mL. In some embodiments, the osmotic pressure regulator is at a concentration of 75 mg/mL to 90 mg/mL. In some embodiments, the osmotic pressure regulator is at a concentration of 70 mg/mL to 90 mg/mL. In some embodiments, the osmotic pressure regulator is at a concentration of 75 mg/mL to 85 mg/mL. In some embodiments, the osmotic pressure regulator is at a concentration of 75 mg/mL to 80 mg/mL. In some embodiments, the osmotic pressure regulator is at a concentration of 80 mg/mL. In some embodiments, the osmotic pressure regulator is at a concentration of 80±5 mg/mL. In some embodiments, non-limiting examples of the concentration of the osmotic pressure regulator include 60 mg/mL, 65 mg/mL, 70 mg/mL, 75 mg/mL, 80 mg/mL, 85 mg/mL, 90 mg/mL, 95 mg/mL, 100 mg/mL, and any range between these point values.

In some embodiments, the pharmaceutical composition described above is an isotonic formulation. In some embodiments, the osmotic pressure regulator controls the pharmaceutical composition described above to have an osmotic pressure of 280-320 mOsm. In some embodiments, the osmotic pressure is controlled at about 290-300 mOsm. In some embodiments, the osmotic pressure regulator controls the osmotic pressure at 280 mOsm, 290 mOsm, 295 mOsm, 300 mOsm, 305 mOsm, 310 mOsm, 320 mOsm, and any range between these point values.

In some embodiments, the osmotic pressure regulator is 80 mg/mL sucrose.

In some embodiments, the osmotic pressure regulator is 80±5 mg/mL sucrose.

In some embodiments, provided is the pharmaceutical composition according to any of the above, wherein the buffer is at a concentration of 5 mM to 100 mM. In some embodiments, the buffer is at a concentration of 10 mM to 30 mM. In some embodiments, the buffer is at a concentration of 5 mM to 15 mM. In some embodiments, the buffer is at a concentration of 5 mM, 10 mM, 15 mM, 20 mM, 25 mM, 30 mM, 40 mM, 50 mM, 60 mM, 70 mM, 80 mM, 90 mM, or 100 mM, and any range between these point values. In some embodiments, the buffer is 10 mM His-HCl. In some embodiments, the buffer is 10±5 mM His-HCl.

In some embodiments, provided is the pharmaceutical composition according to any of the above, wherein in the anti-PVRIG/TIGIT bispecific antibody, the first antigen-binding domain specifically binding to PVRIG thereof comprises at least one immunoglobulin single variable domain (such as VHH), and the immunoglobulin single variable domain (such as VHH) comprises three complementarity determining regions CDR1, CDR2, and CDR3, wherein the CDR1 is selected from the group consisting of an amino acid sequence set forth in any one of SEQ ID NOs: 7, 10, 13, 16, 19, 22, 25, 28, 31, 34, 37, 40, 43, 46, 49, 52, 55, 58, 61, and 64, and an amino acid sequence having 3, 2, 1, or more amino acid differences compared thereto, and/or the CDR2 is selected from the group consisting of an amino acid sequence set forth in any one of SEQ ID NOs: 8, 11, 14, 17, 20, 23, 26, 29, 32, 35, 38, 41, 44, 47, 50, 53, 56, 59, 62, and 65, and an amino acid sequence having 3, 2, 1, or more amino acid differences compared thereto, and/or the CDR3 is selected from the group consisting of an amino acid sequence set forth in any one of SEQ ID NOs: 9, 12, 15, 18, 21, 24, 27, 30, 33, 36, 39, 42, 45, 48, 51, 54, 57, 60, 63, 66, 150, and 151, and an amino acid sequence having 3, 2, 1, or more amino acid differences compared thereto; wherein SEQ ID NOs: 7-21 are based on the Kabat numbering scheme, SEQ ID NOs: 22-36 are based on the Chothia numbering scheme, SEQ ID NOs: 37-51 are based on the IMGT numbering scheme, and SEQ ID NOs: 52-66 are based on the AbM numbering scheme.

In some embodiments, provided is the pharmaceutical composition according to any of the above, wherein in the anti-PVRIG/TIGIT bispecific antibody, the first antigen-binding domain specifically binding to PVRIG thereof comprises at least one immunoglobulin single variable domain (such as VHH), and the immunoglobulin single variable domain comprises a CDR1, a CDR2, and a CDR3 in a sequence set forth in any one of SEQ ID NOs: 2 and 75-79, a CDR1, a CDR2, and a CDR3 in a sequence set forth in any one of SEQ ID NOs: 3 and 80-84, a CDR1, a CDR2, and a CDR3 in a sequence set forth in any one of SEQ ID NOs: 4 and 86-90, a CDR1, a CDR2, and a CDR3 in a sequence set forth in any one of SEQ ID NOs: 5 and 91-95, or a CDR1, a CDR2, and a CDR3 in a sequence set forth in any one of SEQ ID NOs: 6 and 96-100. In some embodiments, the immunoglobulin single variable domain comprises a CDR1, a CDR2, and a CDR3 in a sequence set forth in any one of SEQ ID NOs: 3 and 80-84. In some embodiments, the immunoglobulin single variable domain comprises a CDR1, a CDR2, and a CDR3 in SEQ ID NO: 81. In some embodiments, the CDR1, CDR2, and CDR3 are defined according to the Kabat, IMGT, Chothia, AbM, or Contact numbering scheme. In some embodiments, the CDR1, CDR2, and CDR3 are determined according to the Kabat numbering scheme. In some embodiments, according to the Kabat numbering scheme, the first antigen-binding domain specifically binding to PVRIG (such as VHH) comprises three complementarity determining regions CDR1, CDR2, and CDR3, wherein the amino acid sequences of the CDR1, CDR2, and CDR3 are set forth in SEQ ID NOs: 7, 8, and 9, respectively; the amino acid sequences of the CDR1, CDR2, and CDR3 are set forth in SEQ ID NOs: 7, 8, and 150, respectively; the amino acid sequences of the CDR1, CDR2, and CDR3 are set forth in SEQ ID NOs: 10, 11, and 12, respectively; the amino acid sequences of the CDR1, CDR2, and CDR3 are set forth in SEQ ID NOs: 10, 11, and 151, respectively; the amino acid sequences of the CDR1, CDR2, and CDR3 are set forth in SEQ ID NOs: 13, 14, and 15, respectively; the amino acid sequences of the CDR1, CDR2, and CDR3 are set forth in SEQ ID NOs: 16, 17, and 18, respectively; or the amino acid sequences of the CDR1, CDR2, and CDR3 are set forth in SEQ ID NOs: 19, 20, and 21, respectively. In some embodiments, according to the Chothia numbering scheme, the first antigen-binding domain specifically binding to PVRIG (such as VHH) comprises three complementarity determining regions CDR1, CDR2, and CDR3, wherein the amino acid sequences of the CDR1, CDR2, and CDR3 are set forth in SEQ ID NOs: 22, 23, and 24, respectively; the amino acid sequences of the CDR1, CDR2, and CDR3 are set forth in SEQ ID NOs: 25, 26, and 27, respectively; the amino acid sequences of the CDR1, CDR2, and CDR3 are set forth in SEQ ID NOs: 28, 29, and 30, respectively; the amino acid sequences of the CDR1, CDR2, and CDR3 are set forth in SEQ ID NOs: 31, 32, and 33, respectively; or the amino acid sequences of the CDR1, CDR2, and CDR3 are set forth in SEQ ID NOs: 34, 35, and 36, respectively. In some embodiments, according to the IMGT numbering scheme, the first antigen-binding domain specifically binding to PVRIG (such as VHH) comprises three complementarity determining regions CDR1, CDR2, and CDR3, wherein the amino acid sequences of the CDR1, CDR2, and CDR3 are set forth in SEQ ID NOs: 37, 38, and 39, respectively; the amino acid sequences of the CDR1, CDR2, and CDR3 are set forth in SEQ ID NOs: 40, 41, and 42, respectively; the amino acid sequences of the CDR1, CDR2, and CDR3 are set forth in SEQ ID NOs: 43, 44, and 45, respectively; the amino acid sequences of the CDR1, CDR2, and CDR3 are set forth in SEQ ID NOs: 46, 47, and 48, respectively; or the amino acid sequences of the CDR1, CDR2, and CDR3 are set forth in SEQ ID NOs: 49, 50, and 51, respectively. In some embodiments, according to the AbM numbering scheme, the first antigen-binding domain specifically binding to PVRIG (such as VHH) comprises three complementarity determining regions CDR1, CDR2, and CDR3, wherein the amino acid sequences of the CDR1, CDR2, and CDR3 are set forth in SEQ ID NOs: 52, 53, and 54, respectively; the amino acid sequences of the CDR1, CDR2, and CDR3 are set forth in SEQ ID NOs: 55, 56, and 57, respectively; the amino acid sequences of the CDR1, CDR2, and CDR3 are set forth in SEQ ID NOs: 58, 59, and 60, respectively; the amino acid sequences of the CDR1, CDR2, and CDR3 are set forth in SEQ ID NOs: 61, 62, and 63, respectively; or the amino acid sequences of the CDR1, CDR2, and CDR3 are set forth in SEQ ID NOs: 64, 65, and 66, respectively.

In some embodiments, provided is the pharmaceutical composition according to any of the above, wherein in the anti-PVRIG/TIGIT bispecific antibody, the first antigen-binding domain specifically binding to PVRIG thereof comprises at least one immunoglobulin single variable domain (such as VHH), and the immunoglobulin single variable domain comprises a CDR1, a CDR2, and a CDR3, the CDR1, CDR2, and CDR3 being defined according to the Kabat numbering scheme, wherein the CDR1 comprises the amino acid sequence of SEQ ID NO: 10, the CDR2 comprises the amino acid sequence of SEQ ID NO: 11, and the CDR3 comprises the amino acid sequence of SEQ ID NO: 151.

In some embodiments, provided is the pharmaceutical composition according to any of the above, wherein in the anti-PVRIG/TIGIT bispecific antibody, the first antigen-binding domain specifically binding to PVRIG thereof (such as VHH) comprises an amino acid sequence set forth in any one of SEQ ID NOs: 2-6, 75-84, and 86-100, or an amino acid sequence having at least 70%, at least 80%, at least 90%, at least 95%, at least 96%, at least 97%, at least 98%, or at least 99% identity to at least one sequence of SEQ ID NOs: 2-6, 75-84, and 86-100. In some embodiments, the first antigen-binding domain of the anti-PVRIG/TIGIT bispecific antibody comprises the amino acid sequence of SEQ ID NO: 81. In some embodiments, the first antigen-binding domain of the anti-PVRIG/TIGIT bispecific antibody comprises a sequence of an amino acid sequence having at least 90%, at least 95%, at least 96%, at least 97%, at least 98%, or at least 99% identity to SEQ ID NO: 81.

In some embodiments, provided is the pharmaceutical composition according to any of the above, wherein in the anti-PVRIG/TIGIT bispecific antibody, the first antigen-binding domain specifically binding to PVRIG is a first antibody which is a VHH; the second antigen-binding domain specifically binding to TIGIT is a second antibody. In some embodiments, the second antibody comprises a heavy chain (HC) and a light chain (LC); the first antibody is located at the N-terminus and/or C-terminus of the heavy chain or light chain of the second antibody.

In some embodiments, provided is the pharmaceutical composition according to any of the above, wherein the anti-PVRIG/TIGIT bispecific antibody comprises 1 second antibody specifically binding to TIGIT and 2 first antibodies specifically binding to PVRIG; the second antibody comprises two HCs and two LCs; in the second antibody, a VH of one HC forms an antigen-binding site with a VL of one LC, and a VH of the other HC forms an antigen-binding site with a VL of the other LC. In some embodiments, one of the first antibodies is located at the N-terminus of the heavy chain or light chain of the second antibody, and the other first antibody is located at the C-terminus of the heavy chain or light chain of the second antibody. In some embodiments, the first antibodies are located at the N-termini of the two heavy chains or two light chains, respectively, of the second antibody; or the first antibodies are located at the C-termini of the two heavy chains or two light chains, respectively, of the second antibody. In some embodiments, the first antibodies are located at the N-termini of the two heavy chains, respectively, of the second antibody. In some embodiments, the first antibodies are located at the C-termini of the two heavy chains, respectively, of the second antibody. In some embodiments, the 2 first antibodies are identical. In some embodiments, the 2 first antibodies are different.

In some embodiments, provided is the pharmaceutical composition according to any of the above, wherein in the anti-PVRIG/TIGIT bispecific antibody, the second antibody may be linked to 1, 2, 3, 4, 5, 6, 7, or 8 first antibodies, and the first antibodies are identical or different, and may all be linked to the N-terminus of the heavy chain of the second antibody, or may all be linked to the C-terminus of the heavy chain of the second antibody, or may all be linked to the N-terminus of the light chain of the second antibody, or may all be linked to the C-terminus of the light chain of the second antibody, or may be linked to the N-terminus of the heavy chain, the C-terminus of the heavy chain, the N-terminus of the light chain, or the C-terminus of the light chain of the second antibody, or any combination thereof.

In some embodiments, provided is the pharmaceutical composition according to any of the above, wherein in the anti-PVRIG/TIGIT bispecific antibody, the first antibody is linked, directly or via a linker, to the N-terminus or C-terminus of the heavy chain of the second antibody. In some embodiments, the linker is selected from the group consisting of: amino acid sequences represented by (GmSn)x or (GGNGT)x or (YGNGT)x, wherein m and n are each independently selected from the group consisting of integers from 1 to 8 (e.g., 1, 2, 3, 4, 5, 6, 7, or 8), and x is independently selected from the group consisting of integers from 1 to 20 (e.g., 1, 2, 3, 4, 5, 6, 7, 8, 9, 10, 11, 12, 13, 14, 15, 16, 17, 18, 19, or 20). In some embodiments, the linker is an amino acid sequence represented by GS, GAP, ASGS (SEQ ID NO: 154), G₄S (SEQ ID NO: 155), (G₄S)₂ (SEQ ID NO: 152), (G₄S)₃ (SEQ ID NO: 156), (G₄S)₄ (SEQ ID NO: 157), (G₄S)₅ (SEQ ID NO: 158), (G₄S)₆ (SEQ ID NO: 159), YGNGT (SEQ ID NO: 160), (YGNGT)₂ (SEQ ID NO: 161), (YGNGT)₃ (SEQ ID NO: 162), (YGNGT)₄ (SEQ ID NO: 163), (YGNGT)₅ (SEQ ID NO: 164), or (YGNGT)₆ (SEQ ID NO: 165).

In some embodiments, provided is the pharmaceutical composition according to any of the above, wherein in the anti-PVRIG/TIGIT bispecific antibody, the heavy chain of the second antibody comprises a heavy chain variable region (VH) and a heavy chain constant region (CH), and the light chain comprises a light chain variable region (VL) and a light chain constant region (CL). In some embodiments, the second antibody is a full-length antibody. In some embodiments, the heavy chain of the second antibody is of an IgG isotype (e.g., IgG1, IgG2, IgG3, or IgG4), e.g., the IgG1 isotype; and/or the light chain of the second antibody is of a Kappa isotype.

In some embodiments, provided is the pharmaceutical composition according to any of the above, wherein in the anti-PVRIG/TIGIT bispecific antibody, the two HCs of the second antibody comprise identical CDRs, and/or the two LCs of the second antibody comprise identical CDRs. In some embodiments, the two HCs of the second antibody comprise identical VH, and/or the two LCs of the second antibody comprise identical VL. In some embodiments, the two HCs of the second antibody have identical amino acid sequences, and/or the two LCs of the second antibody have identical amino acid sequences.

In some embodiments, provided is the pharmaceutical composition according to any of the above, wherein the anti-PVRIG/TIGIT bispecific antibody comprises two first antibodies, wherein the two first antibodies have identical or different amino acid sequences. In some embodiments, the two first antibodies have identical amino acid sequences.

In some embodiments, provided is the pharmaceutical composition according to any of the above, wherein the anti-PVRIG/TIGIT bispecific antibody comprises two first polypeptide chains and two second polypeptide chains, wherein for each polypeptide chain:
a) the first polypeptide chains each independently comprise a VHH as the first antibody and a heavy chain (HC) of the second antibody; and
b) the second polypeptide chains each independently comprise a light chain (LC) of the second antibody;
wherein the VHH is linked, directly or via a linker, to the N-terminus and/or C-terminus of the HC of the second antibody. Alternatively, i) the first polypeptide chains each independently comprise a heavy chain (HC) of the second antibody; and ii) the second polypeptide chains each independently comprise the first antibody and a light chain (LC) of the second antibody; wherein the VHH is linked, directly or via a linker, to the N-terminus and/or C-terminus of the LC of the second antibody. In some embodiments, the anti-PVRIG/TIGIT bispecific antibody comprises two identical first polypeptide chains and two identical second polypeptide chains.

In some embodiments, provided is the pharmaceutical composition according to any of the above, wherein in the anti-PVRIG/TIGIT bispecific antibody, the second antigen-binding domain specifically binding to TIGIT is any anti-TIGIT antibody. TIGIT antibodies in WO2009126688, WO2014089113, WO2015009856, WO2015143343, WO2015174439, WO2016028656, WO2016106302, WO2017053748, WO2017030823, US20160176963, US20130251720, WO2019232484 and WO2019062832 are incorporated herein by reference in their entireties. For example, the TIGIT antibody may be any of CPA.9.083.H4(S241P), CPA.9.086.H4(S241P), CHA.9.547.7.H4(S241P), and CHA.9.547.13.H4(S241P) (see WO2019232484).

In some embodiments, provided is the pharmaceutical composition according to any of the above, wherein in the anti-PVRIG/TIGIT bispecific antibody, the second antigen-binding domain specifically binding to TIGIT is a second antibody. In some embodiments, the anti-TIGIT antibody in WO2019062832 is incorporated herein by reference in its entirety as the second antibody. In some embodiments, in the second antibody, the heavy chain variable region comprises a HCDR1, a HCDR2, and a HCDR3 set forth in SEQ ID NOs: 115, 116, and 117, respectively, and the light chain variable region comprises a LCDR1, a LCDR2, and a LCDR3 set forth in SEQ ID NOs: 118, 119, and 120, respectively; or the heavy chain variable region comprises a HCDR1, a HCDR2, and a HCDR3 set forth in SEQ ID NOs: 121, 122, and 123, respectively, and the light chain variable region comprises a LCDR1, a LCDR2, and a LCDR3 set forth in SEQ ID NOs: 124, 125, and 126, respectively; or the heavy chain variable region comprises a HCDR1, a HCDR2, and a HCDR3 set forth in SEQ ID NOs: 127, 128, and 129, respectively, and the light chain variable region comprises a LCDR1, a LCDR2, and a LCDR3 set forth in SEQ ID NOs: 130, 131, and 132, respectively; or the heavy chain variable region comprises a HCDR1, a HCDR2, and a HCDR3 set forth in SEQ ID NOs: 133, 134, and 135, respectively, and the light chain variable region comprises a LCDR1, a LCDR2, and a LCDR3 set forth in SEQ ID NOs: 136, 137, and 138, respectively; or the heavy chain variable region comprises a HCDR1, a HCDR2, and a HCDR3 set forth in SEQ ID NOs: 139, 140, and 141, respectively, and the light chain variable region comprises a LCDR1, a LCDR2, and a LCDR3 set forth in SEQ ID NOs: 142, 143, and 144, respectively.

In some embodiments, provided is the pharmaceutical composition according to any of the above, wherein in the anti-PVRIG/TIGIT bispecific antibody, the second antigen-binding domain specifically binding to TIGIT comprises a heavy chain variable region (VH) and a light chain variable region (VL), wherein the heavy chain variable region comprises a HCDR1, a HCDR2, and a HCDR3, wherein the HCDR1 comprises the amino acid sequence of SEQ ID NO: 121, the HCDR2 comprises the amino acid sequence of SEQ ID NO: 122, and the HCDR3 comprises the amino acid sequence of SEQ ID NO: 123; and the light chain variable region comprises a LCDR1, a LCDR2, and a LCDR3, wherein the LCDR1 comprises the amino acid sequence of SEQ ID NO: 124, the LCDR2 comprises the amino acid sequence of SEQ ID NO: 125, and the LCDR3 comprises the amino acid sequence of SEQ ID NO: 126. In some embodiments, the second antigen-binding domain specifically binding to TIGIT comprises a VH of an amino acid sequence set forth in any one of SEQ ID NOs: 145-147, or a VH of an amino acid sequence having at least 70%, at least 80%, at least 90%, at least 95%, at least 96%, at least 97%, at least 98%, or at least 99% sequence identity to at least one sequence of SEQ ID NOs: 145-147, and/or comprises a VL of an amino acid sequence set forth in any one of SEQ ID NOs: 148-149, or a VL of an amino acid sequence having at least 70%, at least 80%, at least 90%, at least 95%, at least 96%, at least 97%, at least 98%, or at least 99% sequence identity to at least one sequence of SEQ ID NOs: 148-149. In some embodiments, the heavy chain variable region of the second antigen-binding domain specifically binding to TIGIT comprises the amino acid sequence of SEQ ID NO: 145, and the light chain variable region comprises the amino acid sequence of SEQ ID NO: 149. In some embodiments, the second antigen-binding domain specifically binding to TIGIT comprises a heavy chain (HC) and a light chain (LC). In some embodiments, the second antigen-binding domain specifically binding to TIGIT comprises a HC of an amino acid sequence having at least 70%, at least 80%, at least 90%, at least 95%, at least 96%, at least 97%, at least 98%, or at least 99% sequence identity to SEQ ID NO: 102, and a LC of an amino acid sequence having at least 70%, at least 80%, at least 90%, at least 95%, at least 96%, at least 97%, at least 98%, or at least 99% sequence identity to SEQ ID NO: 103. In some embodiments, the second antigen-binding domain specifically binding to TIGIT has a HC comprising the amino acid sequence of SEQ ID NO: 102, and a LC comprising the amino acid sequence of SEQ ID NO: 103. In some embodiments, the second antigen-binding domain specifically binding to TIGIT comprises a heavy chain variable region set forth in SEQ ID NO: 145, and a light chain variable region set forth in SEQ ID NO: 149. In some embodiments, the second antigen-binding domain specifically binding to TIGIT has a HC comprising the amino acid sequence of SEQ ID NO: 102, and a LC comprising the amino acid sequence of SEQ ID NO: 103.

In some embodiments, provided is the pharmaceutical composition according to any of the above, wherein in the anti-PVRIG/TIGIT bispecific antibody, the first antigen-binding domain specifically binding to PVRIG or the first antibody (such as VHH) comprises a CDR1, a CDR2, and a CDR3 set forth in SEQ ID NOs: 7, 8, and 9, or a CDR1, a CDR2, and a CDR3 set forth in SEQ ID NOs: 7, 8, and 150; the heavy chain variable region of the second antigen-binding domain specifically binding to TIGIT or the second antibody comprises a HCDR1, a HCDR2, and a HCDR3 set forth in SEQ ID NOs: 121, 122, and 123, respectively, and the light chain variable region comprises a LCDR1, a LCDR2, and a LCDR3 set forth in SEQ ID NOs: 124, 125, and 126, respectively.

In some embodiments, provided is the pharmaceutical composition according to any of the above, wherein in the anti-PVRIG/TIGIT bispecific antibody, the first antigen-binding domain specifically binding to PVRIG or the first antibody (such as VHH) comprises a CDR1, a CDR2, and a CDR3 set forth in SEQ ID NOs: 10, 11, and 12, or a CDR1, a CDR2, and a CDR3 set forth in SEQ ID NOs: 10, 11, and 151; the heavy chain variable region of the second antigen-binding domain specifically binding to TIGIT or the second antibody comprises a HCDR1, a HCDR2, and a HCDR3 set forth in SEQ ID NOs: 121, 122, and 123, respectively, and the light chain variable region comprises a LCDR1, a LCDR2, and a LCDR3 set forth in SEQ ID NOs: 124, 125, and 126, respectively.

In some embodiments, provided is the pharmaceutical composition according to any of the above, wherein in the anti-PVRIG/TIGIT bispecific antibody, the first antigen-binding domain specifically binding to PVRIG or the first antibody has a VHH comprising an amino acid sequence set forth in any one of SEQ ID NOs: 6, 79, 81, 92, 98, and 99, or a VHH comprising an amino acid sequence having at least 70%, at least 80%, at least 90%, at least 95%, at least 96%, at least 97%, at least 98%, or at least 99% sequence identity to one of SEQ ID NOs: 6, 79, 81, 92, 98, and 99; the second antigen-binding domain specifically binding to TIGIT or the second antibody has a VH comprising an amino acid sequence set forth in any one of SEQ ID NOs: 145-147, or a VH comprising an amino acid sequence having at least 70%, at least 80%, at least 90%, at least 95%, at least 96%, at least 97%, at least 98%, or at least 99% sequence identity to one of SEQ ID NOs: 145-147, and a VL comprising an amino acid sequence set forth in any one of SEQ ID NOs: 148-149, or a VL comprising an amino acid sequence having at least 70%, at least 80%, at least 90%, at least 95%, at least 96%, at least 97%, at least 98%, or at least 99% sequence identity to one of SEQ ID NOs: 148-149. In some embodiments, the second antigen-binding domain specifically binding to TIGIT or the second antibody has a HC comprising the amino acid sequence of SEQ ID NO: 102 or an amino acid sequence having at least 70%, at least 80%, at least 90%, at least 95%, at least 96%, at least 97%, at least 98%, or at least 99% sequence identity to SEQ ID NO: 102, and a LC comprising the amino acid sequence of SEQ ID NO: 103 or an amino acid sequence having at least 70%, at least 80%, at least 90%, at least 95%, at least 96%, at least 97%, at least 98%, or at least 99% sequence identity to SEQ ID NO: 103. In some embodiments, in the anti-PVRIG/TIGIT bispecific antibody, the first antigen-binding domain specifically binding to PVRIG or the first antibody has a VHH set forth in SEQ ID NO: 81, and the second antigen-binding domain specifically binding to TIGIT has a VH set forth in SEQ ID NO: 145 and a VL set forth in SEQ ID NO: 149. In some embodiments, in the anti-PVRIG/TIGIT bispecific antibody, the first antigen-binding domain specifically binding to PVRIG or the first antibody has a VHH set forth in SEQ ID NO: 81, and the second antigen-binding domain specifically binding to TIGIT has a HC set forth in SEQ ID NO: 102 and a LC set forth in SEQ ID NO: 103.

In some embodiments, provided is the pharmaceutical composition according to any of the above, wherein the anti-PVRIG/TIGIT bispecific antibody comprises an Fc region. In some embodiments, the second antigen-binding domain specifically binding to TIGIT or the second antibody comprises an Fc region. In some embodiments, the Fc is an Fc of an IgG isotype, e.g., an Fc region of IgG1, IgG2, IgG3, or IgG4. In some embodiments, the Fc comprises one or more amino acid mutations (mutation sites are based on the EU index) selected from the group consisting of the following:
i) mutations that alter the number of cysteine residues in the hinge region of CH1 to facilitate assembly of the light and heavy chains or to increase or decrease the stability of the antibody;
ii) mutations that enhance binding to FcyRIIIa to result in enhanced ADCC and mutations that attenuate binding to FcyRIIb, such as 236A, 239D, 239E, 332E, 332D, 239D/332E, 267D, 267E, 328F, 267E/328F, 236A/332E, 239D/332E/330Y, 239D, 332E/330L, 299T, 297N, or any combination thereof;
iii) mutations that increase the biological half-life, such as T252L, T254S, T256F, 428L, 434A, 434S 428L/434S, or any combination thereof;
iv) one or more amino acid mutations at positions 234, 235, 236, 237, 297, 318, 320 and 322, or any combination thereof, to alter the affinity of the antibody for an effector ligand while retaining the antigen-binding ability of the parent antibody;
v) one or more amino acid mutations at positions 329, 331 and 322, or any combination thereof, such that the antibody has altered C1q binding and/or the complement dependent cytotoxicity (CDC) is reduced or eliminated;
vi) one or more amino acid mutations within 231-239 or any combination thereof, such that the antibody's ability to fix complement is altered;
vii) one or more amino acid mutations in 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439, or any combination thereof, to increase the capacity for ADCC and/or to increase the affinity of the antibody for the Fcy receptor;
viii) amino acid mutations of S228P, F234A, L235A, and/or K447A; and
ix) amino acid mutations of S354C, E356D, M358L, and/or T366W.

In some embodiments, provided is the pharmaceutical composition according to any of the above, wherein the anti-PVRIG/TIGIT bispecific antibody comprises a first polypeptide chain set forth in SEQ ID NO: 104, and a second polypeptide chain set forth in SEQ ID NO: 103; a first polypeptide chain set forth in SEQ ID NO: 105, and a second polypeptide chain set forth in SEQ ID NO: 103; a first polypeptide chain set forth in SEQ ID NO: 102, and a second polypeptide chain set forth in SEQ ID NO: 106; a first polypeptide chain set forth in SEQ ID NO: 102, and a second polypeptide chain set forth in SEQ ID NO: 107; or a first polypeptide chain set forth in any one of SEQ ID NOs: 108-112 and 114, and a second polypeptide chain set forth in SEQ ID NO: 103; or a variant having at least 70%, at least 80%, at least 90%, at least 95%, at least 96%, at least 97%, at least 98%, or at least 99% sequence identity to the first polypeptide chain and/or the second polypeptide chain described above.

In some embodiments, provided is the pharmaceutical composition according to any of the above, wherein the anti-PVRIG/TIGIT bispecific antibody comprises a first polypeptide chain and a second polypeptide chain, wherein the first polypeptide chain comprises the amino acid sequence of SEQ ID NO: 109, and the second polypeptide chain comprises the amino acid sequence of SEQ ID NO: 103. In some embodiments, the anti-PVRIG/TIGIT bispecific antibody comprises two identical first polypeptide chains and two identical second polypeptide chains.

In some embodiments, provided is the pharmaceutical composition according to any of the above, wherein the anti-PVRIG/TIGIT bispecific antibody is an anti-PVRIG/TIGIT bispecific antibody that competes for binding to the bispecific antibody according to any of the above.

In some embodiments, provided is the pharmaceutical composition according to any of the above, wherein the anti-PVRIG/TIGIT bispecific antibody has at least one of the following characteristics:
(a) having a KD value for binding to human PVRIG of less than 1 × 10⁻⁷ M;
(b) blocking the interaction of PVRIG with its ligand (e.g., PVRL2);
(c) relieving the inhibition of dendritic cells against T cells and activating the T cells;
(d) relieving the inhibition of tumor cells against NK cells; and
(e) inhibiting tumor growth.

In some embodiments, the anti-PVRIG/TIGIT bispecific antibody may have a KD value of less than 1 × 10⁻⁷ M, 1 × 10⁻⁸ M, 1 × 10⁻⁹ M, or 1 × 10⁻¹⁰ M. In some embodiments, the anti-PVRIG/TIGIT bispecific antibody is capable of inhibiting tumor growth by at least about 10%, at least about 20%, at least about 30%, at least about 40%, at least about 50%, at least about 60%, at least about 70%, or at least about 80%.

In some embodiments, provided is the pharmaceutical composition according to any of the above, which comprises the following components:
(a) 1 mg/mL to 100 mg/mL of the anti-PVRIG/TIGIT bispecific antibody according to any of the above;
(b) 0.2 mg/mL to 0.6 mg/mL polysorbate 80;
(c) 75 mg/mL to 90 mg/mL sucrose; and
(d) 10 mM to 30 mM histidine buffer; the pharmaceutical composition has a pH of 5.5 to 6.0.

In some embodiments, the pharmaceutical composition comprises the following components:
(a) 50 mg/mL to 100 mg/mL of the anti-PVRIG/TIGIT bispecific antibody according to any of the above;
(b) 0.2 mg/mL to 0.6 mg/mL polysorbate 80;
(c) 80 mg/mL sucrose; and (d) 10 mM histidine-histidine hydrochloride buffer; the pharmaceutical composition has a pH of 5.5 to 6.0;

In some embodiments, the pharmaceutical composition comprises the following components:
(a) 50 mg/mL of the anti-PVRIG/TIGIT bispecific antibody, wherein the anti-PVRIG/TIGIT bispecific antibody comprises a first polypeptide chain having the amino acid sequence of SEQ ID NO: 109 and a second polypeptide chain having the amino acid sequence of SEQ ID NO: 103; (b) 0.4 mg/mL polysorbate 80; (c) 80 mg/mL sucrose; and (d) 10 mM histidine-histidine hydrochloride buffer; the pharmaceutical composition has a pH of 6.0.

In some embodiments, the pharmaceutical composition comprises the following components:
(a) 50 mg/mL to 100 mg/mL of the anti-PVRIG/TIGIT bispecific antibody according to any of the above;
(b) 0.2 mg/mL to 0.6 mg/mL polysorbate 80;
(c) 80±5 mg/mL sucrose; and
(d) 10±5 mM histidine-histidine hydrochloride buffer; the pharmaceutical composition has a pH of 5.5 to 6.0.

In some embodiments, the pharmaceutical composition comprises the following components:
(a) 1 mg/mL to 100 mg/mL of the anti-PVRIG/TIGIT bispecific antibody; (b) 0.2 mg/mL to 0.6 mg/mL polysorbate 80; (c) 70 mg/mL to 90 mg/mL sucrose; and (d) 10 mM to 30 mM histidine buffer; the pharmaceutical composition has a pH of 5.5 to 6.5.

In some embodiments, the pharmaceutical composition comprises the following components:
(a) 50 mg/mL to 100 mg/mL of the anti-PVRIG/TIGIT bispecific antibody; (b) 0.2 mg/mL to 0.6 mg/mL polysorbate 80; (c) 75 mg/mL to 80 mg/mL sucrose; and (d) 10 mM histidine-histidine hydrochloride buffer; the pharmaceutical composition has a pH of 5.5 to 6.5.

In some embodiments, the pharmaceutical composition comprises the following components:
(a) 50±5 mg/mL of the anti-PVRIG/TIGIT bispecific antibody, wherein the anti-PVRIG/TIGIT bispecific antibody comprises a first polypeptide chain having the amino acid sequence of SEQ ID NO: 109 and a second polypeptide chain having the amino acid sequence of SEQ ID NO: 103;
(b) 0.4±0.1 mg/mL polysorbate 80;
(c) 80±5 mg/mL sucrose; and
(d) 10±5 mM histidine-histidine hydrochloride buffer; the pharmaceutical composition has a pH of 6.0±0.2.

In some embodiments, the pharmaceutical composition comprises the following components:
(a) 40 mg/mL to 60 mg/mL of the anti-PVRIG/TIGIT bispecific antibody according to any of the above (e.g., 1708-30H2);
(b) 0.2 mg/mL to 0.6 mg/mL polysorbate 80;
(c) 75 mg/mL to 80 mg/mL sucrose; and
(d) 10 mM to 30 mM histidine buffer; the pharmaceutical composition has a pH of 5.5 to 6.0.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 80; (c) 75 mg/mL to 80 mg/mL sucrose; and (d) 10 mM to 30 mM acetate buffer; the pharmaceutical composition has a pH of 5.0 to 5.5.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 80; (c) 80 mg/mL sucrose; and (d) 10 mM acetic acid-sodium acetate buffer, with a pH of 5.0.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 80; (c) 80 mg/mL sucrose; and (d) 10 mM acetic acid-sodium acetate buffer, with a pH of 5.5.

In some embodiments, the pharmaceutical composition comprises the following components:
(a) 50±5 mg/mL of the anti-PVRIG/TIGIT bispecific antibody according to any of the above (e.g., 1708-30H2);
(b) 0.4±0.1 mg/mL polysorbate 80;
(c) 75 mg/mL to 80 mg/mL sucrose; and
(d) 10 mM to 30 mM acetate buffer; the pharmaceutical composition has a pH of 5.0 to 5.5.

In some embodiments, the pharmaceutical composition comprises the following components:
(a) 50±5 mg/mL of the anti-PVRIG/TIGIT bispecific antibody according to any of the above (e.g., 1708-30H2);
(b) 0.4±0.1 mg/mL polysorbate 80;
(c) 80±5 mg/mL sucrose; and
(d) 10±5 mM acetic acid-sodium acetate buffer, with a pH of 5.5±0.2.

In some embodiments, the pharmaceutical composition comprises the following components:
(a) 50±5 mg/mL of the anti-PVRIG/TIGIT bispecific antibody according to any of the above (e.g., 1708-30H2); (b) 0.4±0.1 mg/mL polysorbate 80; (c) 80±5 mg/mL sucrose; and (d) 10±5 mM acetic acid-sodium acetate buffer, with a pH of 5.5.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 80; (c) 80 mg/mL sucrose; and (d) 10 mM histidine-histidine hydrochloride buffer, with a pH of 5.5.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 80; (c) 80 mg/mL sucrose; and (d) 10 mM histidine-histidine hydrochloride buffer, with a pH of 6.0.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 80; (c) 80 mg/mL sucrose; and (d) 10 mM histidine-histidine hydrochloride buffer, with a pH of 6.5.

In some embodiments, the pharmaceutical composition comprises the following components:
(a) 100 mg/mL of the anti-PVRIG/TIGIT bispecific antibody according to any of the above (e.g., 1708-30H2); (b) 0.4 mg/mL polysorbate 80; (c) 80 mg/mL sucrose; and (d) 10 mM histidine-histidine hydrochloride buffer, with a pH of 6.0.

In some embodiments, the pharmaceutical composition comprises the following components:
(a) 50 mg/mL of the anti-PVRIG/TIGIT bispecific antibody according to any of the above (e.g., 1708-30H2); (b) 0.2 mg/mL polysorbate 80; (c) 80 mg/mL sucrose; and (d) 10 mM histidine-histidine hydrochloride buffer, with a pH of 6.5.

In some embodiments, the pharmaceutical composition comprises the following components:
(a) 50 mg/mL of the anti-PVRIG/TIGIT bispecific antibody according to any of the above (e.g., 1708-30H2); (b) 0.6 mg/mL polysorbate 80; (c) 80 mg/mL sucrose; and (d) 10 mM histidine-histidine hydrochloride buffer, with a pH of 6.5.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 20; (c) 80 mg/mL sucrose; and (d) 10 mM histidine-histidine hydrochloride buffer, with a pH of 6.5.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL poloxamer 188; (c) 80 mg/mL sucrose; and (d) 10 mM histidine-histidine hydrochloride buffer, with a pH of 6.5.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 20; (c) 80 mg/mL sucrose; and (d) 10 mM histidine-histidine hydrochloride buffer, with a pH of 5.5.

In some embodiments, the pharmaceutical composition comprises the following components:
(a) 50 mg/mL of the anti-PVRIG/TIGIT bispecific antibody according to any of the above (e.g., 1708-30H2); (b) 0.4 mg/mL polysorbate 20; (c) 75 mg/mL sucrose; and (d) 10 mM histidine-histidine hydrochloride buffer, with a pH of 5.5.

In some embodiments, provided is the pharmaceutical composition described above, wherein the pharmaceutical composition is a liquid formulation. In some embodiments, the solvent of the liquid formulation is water.

The present disclosure further provides a lyophilized formulation, wherein the lyophilized formulation can be reconstituted to form the pharmaceutical composition according to any of the above.

The present disclosure further provides a lyophilized formulation, which is a formulation in a lyophilized form of the pharmaceutical composition according to any of the above.

The present disclosure further provides a method for preparing the lyophilized formulation, comprising the step of lyophilizing the pharmaceutical composition according to any of the above. In some embodiments, the lyophilization according to any one of the above comprises steps of pre-freezing, primary drying, and secondary drying in sequence.

The present disclosure further provides a lyophilized formulation, wherein the lyophilized formulation is obtained by lyophilizing the pharmaceutical composition according to any of the above.

The present disclosure further provides a reconstituted solution, wherein the reconstituted solution is obtained by reconstituting the lyophilized formulation according to any of the above.

The present disclosure further provides a reconstituted solution, which is a formulation in a reconstituted form of the lyophilized formulation according to any of the above.

In some embodiments, the reconstituted solution according to any of the above has identical components and contents to those of the pharmaceutical composition described above.

In some embodiments, the reconstituted solution according to any of the above comprises the following components:
(a) 1 mg/mL to 150 mg/mL of the anti-PVRIG/TIGIT bispecific antibody according to any of the above; (b) 0.05 mg/mL to 1.0 mg/mL polysorbate 80; (c) 70 mg/mL to 100 mg/mL sucrose; and (d) 5 mM to 100 mM histidine buffer; the pharmaceutical composition has a pH of 5.0 to 6.5.

In some embodiments, the reconstituted solution according to any of the above comprises the following components:
(a) 1 mg/mL to 100 mg/mL of the anti-PVRIG/TIGIT bispecific antibody; (b) 0.2 mg/mL to 0.6 mg/mL polysorbate 80; (c) 70 mg/mL to 90 mg/mL sucrose; and (d) 10 mM to 30 mM histidine buffer; the pharmaceutical composition has a pH of 5.5 to 6.5. In some embodiments, the reconstituted solution according to any of the above comprises the following components:
(a) 50 mg/mL to 100 mg/mL of the anti-PVRIG/TIGIT bispecific antibody; (b) 0.2 mg/mL to 0.6 mg/mL polysorbate 80; (c) 75 mg/mL to 80 mg/mL sucrose; and (d) 10 mM histidine-histidine hydrochloride buffer; the pharmaceutical composition has a pH of 5.5 to 6.5.

In some embodiments, the reconstituted solution according to any of the above comprises the following components:
(a) 1 mg/mL to 100 mg/mL of the anti-PVRIG/TIGIT bispecific antibody according to any of the above; (b) 0.2 mg/mL to 0.6 mg/mL polysorbate 80; (c) 75 mg/mL to 90 mg/mL sucrose; and (d) 10 mM to 30 mM histidine buffer; the pharmaceutical composition has a pH of 5.5 to 6.0.

In some embodiments, the reconstituted solution comprises the following components:
(a) 50 mg/mL to 100 mg/mL of the anti-PVRIG/TIGIT bispecific antibody according to any of the above; (b) 0.2 mg/mL to 0.6 mg/mL polysorbate 80; (c) 80 mg/mL sucrose; and (d) 10 mM histidine-histidine hydrochloride buffer; the pharmaceutical composition has a pH of 5.5 to 6.0.

In some embodiments, the reconstituted solution comprises the following components:
(a) 50 mg/mL of the anti-PVRIG/TIGIT bispecific antibody, wherein the anti-PVRIG/TIGIT bispecific antibody comprises a first polypeptide chain having the amino acid sequence of SEQ ID NO: 109 and a second polypeptide chain having the amino acid sequence of SEQ ID NO: 103; (b) 0.4 mg/mL polysorbate 80; (c) 80 mg/mL sucrose; and (d) 10 mM histidine-histidine hydrochloride buffer; the pharmaceutical composition has a pH of 6.0.

In some embodiments, the pharmaceutical composition or reconstituted solution according to any one of the above is a formulation for intravenous, subcutaneous, intraperitoneal, or intramuscular injection; in some embodiments, the pharmaceutical composition or reconstituted solution according to any one of the above is a formulation for intravenous injection.

In some embodiments, the pharmaceutical composition or reconstituted solution according to any one of the above is suitable for intravenous, subcutaneous, intraperitoneal, or intramuscular injection.

In some embodiments, the pharmaceutical composition or reconstituted solution or lyophilized formulation according to any one of the above is for use in the preparation of a medicament for intravenous, subcutaneous, intraperitoneal, or intramuscular injection.

The present disclosure further provides a kit, comprising at least one container, wherein each container independently contains the pharmaceutical composition according to any of the above, the lyophilized formulation according to any of the above, or the reconstituted solution according to any of the above.

In some embodiments, the present disclosure further provides a method for diagnosing, treating, and ameliorating a disorder in a subject, which comprises administering to the subject an effective amount of the pharmaceutical composition according to any of the above, the lyophilized formulation according to any of the above, the reconstituted solution according to any of the above, or the kit according to any of the above.

In some embodiments, the present disclosure further provides a method for activating cytotoxic T cells (CTLs) of a subject, which comprises administering to the subject an effective amount of the pharmaceutical composition according to any of the above, the lyophilized formulation according to any of the above, the reconstituted solution according to any of the above, or the kit according to any of the above, wherein a subset of the CTLs of the subject are activated.

In some embodiments, the present disclosure further provides a method for activating NK cells of a subject, which comprises administering to the subject an effective amount of the pharmaceutical composition according to any of the above, the lyophilized formulation according to any of the above, the reconstituted solution according to any of the above, or the kit according to any of the above, wherein a subset of the NK cells of the subject are activated.

In some embodiments, the present disclosure further provides a method for activating γδT cells of a subject, which comprises administering to the subject an effective amount of the pharmaceutical composition according to any of the above, the lyophilized formulation according to any of the above, the reconstituted solution according to any of the above, or the kit according to any of the above, wherein a subset of the γδT cells of the subject are activated.

In some embodiments, the present disclosure further provides a method for activating Th1 cells of a subject, which comprises administering to the subject an effective amount of the pharmaceutical composition according to any of the above, the lyophilized formulation according to any of the above, the reconstituted solution according to any of the above, or the kit according to any of the above, wherein a subset of the Th1 cells of the subject are activated.

In some embodiments, the present disclosure further provides a method for activating, reducing, or eliminating the cell number and/or activity of at least one type of regulatory T cells (Tregs) in a subject, which comprises administering to the subject an effective amount of the pharmaceutical composition according to any of the above, the lyophilized formulation according to any of the above, the reconstituted solution according to any of the above, or the kit according to any of the above.

In some embodiments, the present disclosure further provides a method for increasing the generation of interferon-γ and/or the secretion of pro-inflammatory cytokines in a subject, which comprises administering to the subject an effective amount of the pharmaceutical composition according to any of the above, the lyophilized formulation according to any of the above, the reconstituted solution according to any of the above, or the kit according to any of the above.

In some embodiments, the present disclosure further provides a method for inhibiting the interaction of PVRIG and PVLR2 in a subject, which comprises administering to the subject an effective amount of the pharmaceutical composition according to any of the above, the lyophilized formulation according to any of the above, the reconstituted solution according to any of the above, or the kit according to any of the above.

In some embodiments, the present disclosure further provides use of the pharmaceutical composition according to any of the above, the lyophilized formulation according to any of the above, the reconstituted solution according to any of the above, or the kit according to any of the above in the preparation of a medicament for treating a disease. In some embodiments, the present disclosure further provides a method for treating a disease, which comprises administering to a subject a therapeutically effective amount of the pharmaceutical composition according to any of the above, the lyophilized formulation according to any of the above, the reconstituted solution according to any of the above, or the kit according to any of the above.

In some embodiments, the present disclosure further provides the pharmaceutical composition according to any of the above, the lyophilized formulation according to any of the above, the reconstituted solution according to any of the above, or the kit according to any of the above for use in the treatment of a disease.

In some specific embodiments, the disease according to any of the above is a proliferative disease (e.g., a tumor). In some specific embodiments, the tumor is selected from the group consisting of the following or combinations thereof: prostate cancer, liver cancer (HCC), colorectal cancer (colon cancer and rectal cancer), ovarian cancer, endometrial cancer, breast cancer (e.g., triple negative breast cancer), pancreatic cancer, stomach/gastric cancer, cervical cancer, head and neck cancer, thyroid cancer, testicular cancer, urothelial cancer, lung cancer (small cell lung cancer or non-small cell lung cancer), melanoma, non-melanoma skin cancer (squamous and basal cell carcinoma), neuroglioma, renal cancer (RCC), lymphoma (NHL or HL), acute myeloid leukemia (AML), T-cell acute lymphoblastic leukemia (T-ALL), diffuse large B-cell lymphoma, testis germ cell tumor, mesothelioma, esophagus cancer (also known as esophageal cancer), Merkel cell cancer, MSI-high cancer, KRAS-mutant tumor, adult T-cell leukemia/lymphoma, and myelodysplastic syndrome (MDS). In some specific embodiments, the disease described above may be a disease associated with the abnormal expression of PVRIG and/or TIGIT. In some specific embodiments, the tumor is selected from the group consisting of the following cancers or combinations thereof: triple negative breast cancer, gastric cancer, lung cancer (small cell lung cancer or non-small cell lung cancer), Merkel cell cancer, MSI-high cancer, KRAS-mutant tumor, adult T cell leukemia/lymphoma, and myelodysplastic syndrome (MDS). In some specific embodiments, the tumor is selected from the group consisting of the following cancers or combinations thereof: triple negative breast cancer, gastric cancer, lung cancer (small cell lung cancer or non-small cell lung cancer), Merkel cell cancer, and MSI-high cancer. In some specific embodiments, a patient with the disease described above has a condition associated with PVRIG and/or TIGIT. In some embodiments, the condition of the subject includes a cancer that expresses or does not express PVRIG and further includes non-metastatic or non-infiltrative and infiltrative or metastatic cancer, wherein PVRIG expression of immune cells, stromal cells, or diseased cells inhibits an anti-tumor response and an anti-infiltration immune response. In some embodiments, the disease is a vascularized tumor. In some embodiments, the tumor is selected from the group consisting of lung cancer, prostate cancer, breast cancer, head and neck cancer, esophagus cancer, gastric cancer, colorectal cancer, bladder cancer, cervical cancer, endometrial cancer, ovarian cancer, liver cancer, melanoma, renal cancer, squamous cell carcinoma, hematological cancer, and any other diseases or disorders characterized by uncontrolled cell growth.

In some embodiments, the disease is infection or sepsis. In some embodiments, the infection is a pathogen infection characterized by different degrees of dysfunction of a virus-specific T cell response, such as HIV, HCV, or HBV In some embodiments, the sepsis is selected from the group consisting of severe sepsis, septic shock, systemic inflammatory response syndrome (SIRS), bacteremia, septicemia, toxemia, and septic syndrome.

### BRIEF DESCRIPTION OF THE DRAWING

FIG. 1 shows the detection results of the activity of anti-PVRIG antibodies in PVRIG reporter gene cells.
FIG. 2 shows the detection results of activating the activity of NK cells by anti-PVRIG antibodies in an NK cell killing experiment.
FIG. 3 shows the detection results of activating the activity of T cells by anti-PVRIG antibodies in an MLR experiment.
FIGs. 4A-4B show the detection results of the activity of humanized anti-PVRIG antibodies in PVRIG reporter gene cells.
FIGs. 5A-5B show the detection results of activating the activity of NK cells by humanized anti-PVRIG antibodies in an NK cell killing experiment.
FIGs. 6A-6E show the detection results of the binding activities of humanized anti-PVRIG/TIGIT bispecific antibodies to a human PVRIG recombinant protein, cells overexpressing human PVRIG, a cynomolgus monkey PVRIG recombinant protein, and cells overexpressing cynomolgus monkey PVRIG, and the activity thereof in blocking the binding of human PVRIG to human PVRL2, respectively.
FIGs. 7A-7E show the detection results of the binding activities of humanized anti-PVRIG/TIGIT bispecific antibodies to a human TIGIT recombinant protein, cells overexpressing human TIGIT, a cynomolgus monkey TIGIT recombinant protein, and cells overexpressing cynomolgus monkey TIGIT, and the activity thereof in blocking the binding of human TIGIT to human PVR, respectively.
FIG. 8 shows the detection results of activating the activity of T cells by humanized anti-PVRIG/TIGIT bispecific antibodies in an MLR experiment.
FIGs. 9A-9B show the effect of anti-PVRIG/TIGIT bispecific antibodies on mouse body weight and tumor volume, respectively, in mouse subcutaneous xenograft tumor models of human melanoma A375 mixed with human PBMCs.
FIGs. 10A-10B show the effect of anti-PVRIG/TIGIT bispecific antibodies on mouse body weight and tumor volume, respectively, in mouse subcutaneous xenograft tumor models of human melanoma A375 mixed with human PBMCs.

### DETAILED DESCRIPTION

### Terminology

In order to facilitate the understanding of the present disclosure, some technical and scientific terms are specifically defined below. Unless otherwise explicitly defined herein, all other technical and scientific terms used herein have the meanings generally understood by those of ordinary skill in the art to which the present disclosure belongs. The three-letter and single-letter codes for amino acids used herein are as described in J. biol. chem, 243, p3558 (1968).

"PVRIG", "PVRIG protein", or "PVRIG polypeptide" may optionally include any such protein or a variant, a conjugate, or a fragment thereof, including but not limited to known or wild-type PVRIG described herein, as well as any naturally occurring splice variant, amino acid variant or isoform, in particular a soluble extracellular domain (ECD) fragment of PVRIG. ECD is defined herein as in patent application WO2016134333. The complete human PVRIG sequence can be found under GenBank accession No. AAH73861.1.

"TIGIT", "TIGIT protein", or "TIGIT polypeptide" may optionally include any such protein or a variant, a conjugate, or a fragment thereof, including (but not limited to) known or wild-type TIGIT described herein, as well as any naturally occurring splice variant, amino acid variant or isoform. The complete TIGIT sequence can be found under GenBank accession No. AAI01289.1.

"Binding to PVRIG" refers to the ability to interact with PVRIG or an epitope thereof, wherein the PVRIG or the epitope thereof may be derived from humans.

"Binding to TIGIT" refers to the ability to interact with TIGIT or an epitope thereof, wherein the TIGIT or the epitope thereof may be derived from humans. "Antigen-binding site" refers to a continuous or discontinuous three-dimensional spatial site on an antigen that is recognized by an antibody of the present disclosure.

"Antibody" or "immunoglobulin" is used in the broadest sense and encompasses a variety of antibody structures, including, but not limited to conventional antibodies (antibodies with a tetrapeptide chain structure formed by linking two heavy chains and two light chains via inter-chain disulfide bonds) and a Fab, an Fv, an sFv, an F(ab')2, a linear antibody, a single-chain antibody, an scFv, an sdAb, an sdFv, a nanobody, a peptibody, a domain antibody (a heavy chain (VH) antibody and a light chain (VL) antibody) and a multispecific antibody (a bispecific antibody, a diabody, a triabody, a tetrabody, a tandem di-scFv, and a tandem tri-scFv) having antigen-binding activity.

The term "antibody" as used in the present disclosure is intended to encompass full-length antibodies, individual chains thereof, any portions (i.e., antigen-binding fragments), domains, or fragments thereof having antigen-binding activity, and multispecific antibodies (including but not limited to antigen-binding domains or fragments, such as VHH domains or VH/VL domains) comprising individual chains thereof and any portions, domains, or fragments thereof having antigen-binding activity. A conventional antibody or immunoglobulin is usually of a tetrapeptide chain structure formed by linking two identical heavy chains and two identical light chains by inter-chain disulfide bonds. According to differences in the amino acid composition and the order of arrangement of the heavy chain constant regions, immunoglobulins can be divided into five classes, otherwise called isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five Ig classes may have a κ (kappa) chain or a λ (lambda) chain. In some embodiments, the antibody of the present disclosure specifically binds to PVRIG and/or TIGIT.

The "antigen-binding fragments" of the present disclosure include, but are not limited to: (i) a Fab fragment consisting of VL, VH, CL, and CH1 domains; (ii) an Fd fragment consisting of the VH and CH1 domains; (iii) an F(ab')2 fragment, a bivalent fragment comprising two linked Fab fragments; (vii) a single-chain Fv molecule (scFv) in which the VH domain and the VL domain are linked via a peptide linker that allows the binding of the two domains to form an antigen-binding site; (Bird et al., 1988, Science, 242: 423-426; Huston et al., 1988, Proc. Natl. Acad. Sci. U.S.A., 85:5879-5883) 242, incorporated herein by reference in their entireties); (iv) "bifunctional antibodies" or "trifunctional antibodies", multivalent or multispecific fragments constructed by gene fusion (Tomlinson et al., 2000, Methods Enzymol., 326: 461-479; WO94/13804; Holliger et al., 1993, PNAS, 90: 6444-6448, all incorporated herein by reference in their entireties); (v) "domain antibodies" or "dAbs" (sometimes referred to as "immunoglobulin single variable domains"), including immunoglobulin single variable domains derived from other species, such as rodents (e.g., as disclosed in WO00/29004), nurse sharks, and camelidae V-HH dAbs; (vi) small molecule immunopharmaceuticals (SMIPs), camelid antibodies, nanobodies, and IgNARs; (vii) humanized antibodies of the above (i) to (vi).

Unless otherwise stated, antibodies of the present disclosure generally use the Kabat numbering scheme. EU numbering in Kabat is also generally used for constant domains and/or Fc domains.

The term "bispecific antibody" refers to an antibody that is capable of specifically binding to two different antigens or two different epitopes of the same antigen. Bispecific antibodies of various structures have been disclosed in the prior art. The bispecific antibodies can be classified into IgG-like bispecific antibodies and antibody-fragment-type bispecific antibodies according to the integrity of IgG molecules. The bispecific antibodies can be classified into bivalent, trivalent, tetravalent, or higher-valent bispecific antibodies according to the number of the antigen-binding regions. The bispecific antibodies can be classified into symmetric bispecific antibodies and asymmetric bispecific antibodies according to whether their structures are horizontally symmetric or asymmetric. Among them, bispecific antibodies based on antibody fragments, such as Fab fragments lacking Fc fragments, formed by linking 2 or more Fab fragments in one molecule, have low immunogenicity, a small molecular weight, and relatively high tumor tissue permeability. Typical antibody structures of this type include bispecific antibodies such as an F(ab)2, an scFv-Fab, an (scFv)2-Fab, and the like, as well as IgG-like bispecific antibodies (e.g., having Fc fragments). This type of antibodies have a large relative molecular weight, and the Fc fragments facilitate the later purification of the antibodies and increase their solubility and stability. The Fc portions may further bind to the receptor FcRn and increase the serum half-life of the antibodies. For example, typical structural models of bispecific antibodies include bispecific antibodies such as VHH-IgG, KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, F(ab)4-CrossMAb, and the like (see Aran F. Labrijn et al., Nature Reviews Drug Discovery, volume 18, pages 585-608 (2019); Chen S1 et al., J Immunol Res., Feb. 11, 2019; 2019: 4516041).

The antibody of the present disclosure may be polyclonal, monoclonal, xenogenic, allogeneic, syngeneic, or modified forms thereof, with the monoclonal antibody being particularly useful in various embodiments. Generally, the antibody of the present disclosure is a recombinant antibody. The "recombinant" used herein generally refers to such products as a cell, a nucleic acid, a protein or a vector, and indicates that the cell, the nucleic acid, the protein or the vector has been modified by the introduction of a heterologous nucleic acid or protein or the alteration of a natural nucleic acid or protein, or that the cell is derived from a cell modified in this way. For example, recombinant cells express genes that are not found within the native (non-recombinant) cellular form or express native genes that are abnormally expressed, lowly expressed, or not expressed at all.

"Monoclonal antibody" refers to a population of antibody molecules produced by a single B cell clone and capable of interacting with a particular epitope of an antigen. "Polyclonal antibody" refers to a population containing antibody molecules produced by multiple B cell clones and capable of interacting with a particular antigen. The monoclonal antibody typically exhibits a single binding affinity for a particular antigen with which it interacts.

Herein, antigens are defined more broadly, and are generally intended to include target molecules specifically recognized by antibodies. Thus, antigens include molecules or mimics used in immunization processes for the production of antibodies or in antibody library screening.

"Homology" or "identity" refers to sequence similarity between two polynucleotide sequences or between two polypeptides. When positions in two sequences to be compared are occupied by identical bases or amino acid residues and if the position of one of two DNA molecules is occupied by adenine, the molecules are homologous at that position. The identity percentage between two sequences is a function of the number of matching or homologous positions shared by the two sequences divided by the number of positions to be compared × 100%. For example, if 6 out of 10 positions are matched or homologous when two sequences are optimally aligned, the two sequences are 60% homologous. Generally, when two sequences are aligned, comparison is performed to obtain the maximum homology percentage.

"Domain" of a polypeptide or protein refers to a folded protein structure that is capable of maintaining its tertiary structure independently of the rest of the protein. In general, a domain is responsible for a single functional property of a protein, and in many cases may be added, removed or transferred to other proteins without loss of functions of the rest of the protein and/or the domain.

"Immunoglobulin domain" refers to a globular region of an antibody chain or a polypeptide substantially consisting of this type of globular regions. In some embodiments, non-limiting examples of "antigen-binding domains" include Fab fragments, Fd fragments, F(ab')2 fragments, scFvs, full-length antibodies, and the like.

"Immunoglobulin variable domain" refers to an immunoglobulin domain substantially consisting of the following: "framework region 1" or "FR1", "framework region 2" or "FR2", "framework region 3" or "FR3", "framework region 4" or "FR4", "complementarity determining region 1" or "CDR1", "complementarity determining region 2" or "CDR2", and "complementarity determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Antibody framework (FR)" refers to a portion of a variable domain, which serves as a framework for the antigen-binding loops (CDRs) of the variable domain.

For the determination or definition of "CDRs", the deterministic depiction of CDRs or identification of residues of binding sites can be accomplished by resolving the structure of the antibody and/or resolving the structure of the antibody-ligand complex. This can be accomplished by any of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions. (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Set (USA), 86: 9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283: 1156-1166). In addition, other CDR boundary definitions may not strictly follow one of the above methods, but still overlap with at least a portion of the Kabat CDRs, although they may be shortened or lengthened based on predictions or experimental results that a particular residue or a particular group of residues do not significantly affect the antigen binding. As used herein, a CDR may refer to a CDR defined by any method known in the art, including combinations of methods. In the methods used herein, CDRs defined according to any of those methods may be used. For any given embodiment comprising more than one CDR, the CDRs may be defined according to any of Kabat, Chothia, extended, AbM, IMGT, contact, and/or conformational definitions.

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain antibodies). Examples of "immunoglobulin single variable domains" include nanobodies (including a VHH, such as a humanized VHH and/or camelized VH, e.g., a camelized human VH), IgNARs, domains, (single-domain) antibodies as VH domains or derived from VH domains (such as dAbs^{™}) and (single-domain) antibodies as VL domains or derived from VL domains (such as dAbs^{™}). Immunoglobulin single variable domains based on and/or derived from heavy chain variable domains (such as VH or VHH domains) are generally preferred. A specific example of an immunoglobulin single variable domain is a "VHH domain" (or simply "VHH") as defined below.

"VHH domain", also known as a heavy chain single-domain antibody, a VHH, a VHH antibody fragment, a VHH antibody, or a nanobody, is a variable domain of an antigen-binding immunoglobulin known as a "heavy chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). The term "VHH domain" is used to distinguish the variable domain from the heavy chain variable domain (which is referred to in the present disclosure as a "VH domain") and the light chain variable domain (which is referred to in the present disclosure as a "VL domain") present in conventional antibodies with a tetrapeptide chain structure. The VHH domain specifically binds to an epitope without the need for an additional antigen-binding domain (unlike the VH or VL domain in conventional antibodies with a tetrapeptide chain structure, in which case the epitope is recognized by the VL domain together with the VH domain). The VHH domain is a small and stable antigen recognition unit formed from a single immunoglobulin domain. The terms "heavy chain single-domain antibody", "VHH domain", "VHH", "VHH domain", "VHH antibody fragment", and "VHH antibody" ("Nanobody" is a trademark of Ablynx N.V, Ghent, Belgium) are used interchangeably. "VHH domains" include, but are not limited to, natural antibodies produced by camelids, antibodies produced by camelids and then humanized, or antibodies obtained by screening with phage display techniques.

As is well known in the art for VH domains and VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. Other numbering schemes or coding rules applicable to VHH include Chothia, IMGT, and AbM.

The total number of amino acid residues in a VHH domain will usually be in the range of 110 to 120, often between 112 and 115. However, it should be noted that smaller and longer sequences may also be suitable for the purposes described in the present disclosure.

Compared with conventional VH and VL domains, scFv or conventional antibody fragments (e.g., Fab- or F(ab')2 fragments), VHH domains, either alone or as part of a larger polypeptide, offer a number of superior significant advantages:
- only a single domain is required to bind to an antigen with high affinity and high selectivity, so that there is no need to have two separate domains present, nor to assure that these two domains are present in the right spatial conformation and configuration (e.g., the use of specially designed linker is generally required for an scFv);
- VHH domains can be expressed from a single gene and do not require post-translational folding or modification;
- VHH domains can be engineered into multivalent and multispecific formats;
- VHH domains are highly soluble and do not have a tendency to aggregate;
- VHH domains are highly stable to heat, pH, proteases, and denaturing agents, and thus may reduce the dependence on refrigeration equipment during preparation, storage, or transportation, thereby saving the cost, time, and environment;
- VHH domains may be prepared and relatively inexpensive, even in terms of the manufacturing scale;
- VHH domains are relatively small compared with conventional antibodies with a tetrapeptide chain structure and antigen-binding fragments thereof (about 15 kDa or 1/10 of conventional IgG in size), and therefore show higher tissue permeability and can be administered at higher doses compared with conventional antibodies with a tetrapeptide chain structure and antigen-binding fragments thereof;
- VHH domains can show so-called cavity-binding properties (particularly due to their extended CDR3 loop, compared with conventional VH domains) and can therefore also access targets and epitopes not accessible to conventional antibodies with a tetrapeptide chain structure and antigen-binding fragments thereof.

Methods for obtaining VHHs that bind to a particular antigen or epitope have been previously disclosed in the following documents: R. van der Linden et al., Journal of Immunological Methods, 240(2000) 185-195; Li et al., J Biol Chem., 287(2012) 13713-13721; Deffar et al., African Journal of Biotechnology Vol. 8(12), pp.2645-2652, 17 June, 2009 and WO94/04678.

"Fc variant" or "variant Fc" refers to a protein comprising amino acid modifications in the Fc domain. The Fc variants of the present disclosure are defined according to the amino acid modifications that compose them. Thus, for example, S228P or 228P is an Fc variant with the substitution proline at position 228 relative to the parent Fc polypeptide, wherein the numbering is according to the EU index. The WT amino acid may be unspecified, in which case the aforementioned variant is referred to as 228P. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a tetrapeptide chain structure. Humanization is also referred to in the present disclosure as "sequence optimization". In addition to humanization, "sequence optimization" may also encompass other modifications to the sequence by one or more mutations providing improved properties of the VHH, such as removal of potential post-translational modification sites. The humanized VHH domain may contain one or more fully human framework region sequences, and in some specific embodiments, may contain the human framework region sequence of IGHV3. Another example of "humanization" includes an antibody produced by grafting mouse CDR sequences into a human antibody variable region framework, i.e., a different type of human germline antibody framework sequence. Therefore, the strong antibody variable antibody reaction induced by a large amount of heterologous protein components contained in the chimeric antibody can be overcome. Methods for humanization include, e.g., protein surface amino acid humanization (resurfacing) and universal framework grafting method for antibody humanization (CDR grafting to a universal framework), i.e., "grafting" CDRs onto other "frameworks" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the "VBase" human species sequence database, as well as in Kabat, E. A. et al., 1991 Sequences of Proteins of Immunological Interest, 5th edition. The humanized antibodies of the present disclosure also include humanized antibodies which are further subjected to CDR affinity maturation by phage display. In addition, in order to avoid the decrease in activity caused by the decrease in immunogenicity, the framework sequence in the human antibody variable region can be subjected to minimum reverse mutation or back mutation to maintain the activity.

"Affinity-matured" antibody has one or more changes in one or more CDRs that result in increased affinity for an antigen as compared to its respective parent antibody. Affinity-matured antibodies can be prepared, for example, by methods known in the art as described below: Marks et al., 1992, Biotechnology 10: 779-783 or Barbas et al., 1994, Proc. Nat. Acad. Sci, USA 91: 3809-3813; Shier et al., 1995, Gene 169: 147-155; Yelton et al., 1995, Immunol. 155: 1994-2004; Jackson et al., 1995, J. Immunol. 154(7): 3310-9; Hawkins et al., 1992, J. Mol. Biol. 226(3): 889896; KS Johnson and RE Hawkins, "Affinity maturation of antibodies using phage display", Oxford University Press 1996.

Typically, the antibody of the present disclosure binds to an antigen to be bound (i.e., PVRIG) with a dissociation constant (KD) of preferably 10⁻⁷ to 10⁻¹⁰ mol/L (M), more preferably 10⁻⁸ to 10⁻¹⁰ mol/L, even more preferably 10⁻⁹ to 10⁻¹⁰ or less, and/or with an association constant (KA) of at least 10⁻⁷ M, preferably at least 10⁻⁸ M, more preferably at least 10⁻⁹ M, or more preferably at least 10⁻¹⁰ M, as measured in a Biacore, KinExA, or Fortibio assay. Any KD value greater than 10⁻⁴ M is generally considered to indicate non-specific binding. Specific binding of an antibody to an antigen or epitope can be measured in any suitable manner known, including, for example, surface plasmon resonance (SPR) assays, Scatchard assay, and/or competitive binding assay (e.g., radioimmunoassay (RIA), enzyme immunoassay (EIA), and sandwich competitive assay) described in the present disclosure.

"Epitope" or "antigenic determinant" used interchangeably herein refers to any portion on an antigen to which an antibody binds. The antigenic determinant generally comprises chemically active surface groups, such as amino acids or sugar side chains, and usually has specific three-dimensional structural characteristics and specific charge characteristics. For example, an epitope typically comprises at least 3, 4, 5, 6, 7, 8, 9, 10, 11, 12, 13, 14 or 15 contiguous or non-contiguous amino acids in a unique spatial conformation, and it may be a "linear" epitope or a "conformational" epitope. In a linear epitope, all points of interaction between a protein and an interacting molecule (e.g., an antibody) exist linearly along the primary amino acid sequence of the protein. In a conformational epitope, the interaction positions are located in amino acid residues that are separated from each other. Epitopes of a given antigen can be identified using a number of epitope mapping techniques well known in the art (e.g., Epitope Mapping Protocols in Methods in Molecular Biology, Vol. 66, G.E.Morris, Ed. (1996), US4708871). Antibodies can be competitively screened for binding to the same epitope using conventional techniques known to those skilled in the art. For example, competition and cross-competition studies can be performed to obtain antibodies that compete or cross-compete with one another for binding to an antigen (see, e.g., WO03/48731 for high throughput screening methods). Therefore, an antibody and an antigen-binding fragment thereof that competes with the antibody molecule of the present disclosure for binding to the same epitope on PVRIG can be obtained using conventional techniques known to those skilled in the art.

"Specific binding" or "selective binding" refers to binding of an antibody to an epitope on a predetermined antigen. Typically, an antibody binds to a predetermined antigen or epitope thereof with an equilibrium dissociation constant (K_{D}) of about less than 10⁻⁷ M or even less and with an affinity that is at least twice as high as its affinity for binding to a non-specific antigen (e.g., BSA) other than the predetermined antigen (or epitope thereof) or a closely related antigen, when determined by surface plasmon resonance (SPR) technique in an instrument using recombinant human PVRIG, TIGIT or an epitope thereof as the analyte and an antibody as the ligand. "Antigen-recognizing antibody" is used interchangeably herein with "specifically bound antibody".

"Binding affinity" is used herein as a measure of the strength of a non-covalent interaction between two molecules (e.g., an antibody or a portion thereof and an antigen) and is used to describe monovalent interaction (intrinsic activity). The binding affinity between two molecules can be quantified by determining the dissociation constant (K_{D}). K_{D} can be determined by measuring the kinetics of complex formation and dissociation by using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods (see Caceci et al., 1984, Byte 9: 340-362; Wong&Lohman, 1993, PNAS 90: 5428-5432). Other standard assays for evaluating the binding ability of an antibody to a target antigen are known in the art and include, for example, ELISA, western blot, RIA and flow cytometry, as well as other assays exemplified elsewhere herein. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to PVRIG). In some embodiments, the anti-PVRIG antibody of the present disclosure is capable of binding to its target with an affinity at least 2-fold, 10-fold, 50-fold, 100-fold, 200-fold, 500-fold, 1,000-fold, or 10,000-fold greater than its affinity for binding to another non-PVRIG molecule, and these amounts here are not meant to be limiting.

"Conservative modifications" are applicable to amino acid and nucleotide sequences. For particular nucleotide sequences, conservative modifications refer to mutual replacement of those nucleic acids encoding identical or substantially identical amino acid sequences, or, in the case of nucleotides not encoding amino acid sequences, to substantially identical nucleotide sequences. For amino acid sequences, "conservative modifications" refer to the replacement of amino acids in a protein with other amino acids having similar characteristics (e.g., charge, side chain size, hydrophobicity/hydrophilicity, backbone conformation, and rigidity) such that changes can be made frequently without altering the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)).

"Amino acid mutations" include amino acid substitutions, deletions, insertions, modifications, and any combination thereof, to obtain a final construct that possesses desired properties, such as enhanced stability and increased activity. Amino acid sequence deletions and insertions include deletions and insertions at the amino terminus and/or the carboxyl terminus. Preferred amino acid mutations are amino acid substitutions. To alter the binding properties of, for example, an anti-PVRIG antibody, non-conservative amino acid substitutions may be made, i.e., one amino acid is replaced with another amino acid having different structural and/or chemical properties. Preferred amino acid substitutions include the replacement of hydrophobic amino acids with hydrophilic amino acids. Amino acid substitutions include the replacement with non-naturally occurring amino acids or with naturally occurring amino acid derivatives of the 20 standard amino acids (e.g., 4-hydroxyproline, 3-methylhistidine, omithine, homoserine and 5-hydroxylysine). Amino acid mutations can be generated using genetic or chemical methods well known in the art, including site-directed mutagenesis, PCR, gene synthesis, chemical modification, and the like. The amino acid mutations may occur in the CDRs, FRs or Fc regions of an antibody.

The engineered antibody or antigen-binding fragment of the present disclosure can be prepared and purified by conventional methods. For example, cDNA sequences encoding the heavy and light chains can be cloned and recombined into an expression vector. Recombinant immunoglobulin expression vectors can be stably transfected into CHO cells. Mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free medium of a bioreactor to produce antibodies. The culture medium with the secreted antibody can be purified and collected by conventional techniques. The antibody can be filtered and concentrated by conventional methods. Soluble mixtures and multimers can also be removed by conventional methods, such as molecular sieves and ion exchange. The resulting product needs to be immediately frozen, e.g., at -70 °C, or lyophilized.

"Optional" or "optionally" means that the event or circumstance subsequently described may, but does not necessarily, occur, and that the description includes instances where the event or circumstance occurs or does not occur. For example, "optionally comprising 1-3 antibody heavy chain variable regions" means that the antibody heavy chain variable region of a particular sequence may, but does not necessarily, exist.

"Pharmaceutical composition" refers to a mixture containing one or more of the antibodies described herein (such as anti-PVRIG/TIGIT bispecific antibodies) or a physiologically and pharmaceutically acceptable salt or prodrug thereof, and other chemical components, wherein the other components are, for example, physiologically and pharmaceutically acceptable carriers and excipients. The pharmaceutical composition is intended to promote the administration to an organism, so as to facilitate the absorption of the active ingredient, thereby exerting biological activity.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as a phosphate buffered saline solution, water, an emulsion such as an oil/water emulsion, and various types of wetting agents. In some embodiments, the diluent for parenteral administration is phosphate buffered saline (PBS) or normal (0.9%) saline. Compositions containing such carriers are formulated by well-known conventional methods (see, e.g., Remington's Pharmaceutical Sciences, 18th edition, A. Gennaro, eds., Mack Publishing Co., Easton, PA, 1990; and R Remington, The Science and Practice of Pharmacy, 20th edition, Mack Publishing, 2000).

"Buffer" refers to a buffer that regulates changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Lyophilized formulation" refers to a formulation or a pharmaceutical composition obtained by lyophilizing a pharmaceutical composition or a formulation in a liquid or solution form.

The pharmaceutical composition described herein can achieve a stable effect and is a pharmaceutical composition in which the antibody therein (such as the anti-PVRIG/TIGIT bispecific antibody) substantially retains its physical stability, and/or chemical stability, and/or biological activity after storage. Preferably, the pharmaceutical composition substantially retains its physical and chemical stability and its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured. Stable formulations include formulations in which no significant change is observed under the following conditions: stored at a refrigeration temperature (2-8 °C) for at least 3 months, preferably 6 months, more preferably 1 year, and even more preferably up to 2 years.

In addition, stable liquid formulations further include liquid formulations that exhibit desirable features after storage at temperatures including 25 °C for periods including 1 month, 3 months, or 6 months. Typical examples for stability are as follows: generally, no more than about 10%, preferably no more than about 5%, of anti-PVRIG/TIGIT bispecific antibodies aggregate or are degraded as measured by SEC-HPLC. The formulation is a pale yellow, nearly colorless and clear liquid, or a colorless and clear liquid, or is clear to slightly opalescent, by visual analysis. The concentration, pH, and osmolality of the formulation have a change of no more than ±10%. Generally, a decrease of no more than about 10%, preferably no more than about 5% is observed. Generally, aggregation of no more than about 10%, preferably no more than about 5% is formed.

In some embodiments, the anti-PVRIG/TIGIT bispecific antibody formulation of the present disclosure has a SEC monomer% greater than or equal to 95% (e.g., greater than 95%, greater than 96%, greater than 97%, greater than 98%, or greater than 99%) and/or a CE-SDS (NR)% greater than or equal to 95% (e.g., greater than 95%, greater than 96%, greater than 97%, greater than 98%, or greater than 99%) after storage at a high temperature of 40 °C for one month.

An anti-PVRIG/TIGIT bispecific antibody "retains its physical stability" in a pharmaceutical formulation if the anti-PVRIG/TIGIT bispecific antibody shows no significant increase in aggregation, precipitation, and/or denaturation upon visual inspection of color and/or clarity, or as determined by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An anti-PVRIG/TIGIT bispecific antibody "retains its chemical stability" in a pharmaceutical formulation if the anti-PVRIG/TIGIT bispecific antibody shows no significant chemical change. Chemical stability can be evaluated by detecting and quantifying chemically changed forms of the protein. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An anti-PVRIG/TIGIT bispecific antibody "retains its biological activity" in a pharmaceutical formulation if the biological activity of the anti-PVRIG/TIGIT bispecific antibody at a given time is within a predetermined range of the biological activity exhibited during the preparation of the pharmaceutical formulation.

"Administrating", "giving", and "treating", when applied to animals, humans, experimental subjects, cells, tissues, organs or biological fluids, refer to contact of an exogenous drug, a therapeutic agent, a diagnostic agent or a composition with the animals, humans, subjects, cells, tissues, organs or biological fluids. "Administering", "giving", and "treating" can refer to, for example, therapeutic, pharmacokinetic, diagnostic, research and experimental methods. The treatment of cells comprises contacting the reagent with the cells and contacting the reagent with fluid, where the fluid is in contact with the cells. "Administering", "giving", and "treating" also refer to *in vitro* and *ex vivo* treatments, e.g., of a cell, by a reagent, diagnosis, a composition, or another cell. "Treating", when applied to humans, veterinary, or research subjects, refers to therapeutic treatment, preventive or prophylactic measures, and research and diagnostic applications.

"Treatment" refers to administering a therapeutic agent, for example, comprising any of the pharmaceutical compositions of the present disclosure, either internally or externally to a subject with one or more symptoms of a disease on which the therapeutic agent is known to have a therapeutic effect. Generally, the therapeutic agent is administered in an amount effective to alleviate one or more symptoms of the disease in the subject or population being treated to induce regression of such symptoms or inhibit the development of such symptoms to any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although embodiments of the present disclosure (e.g., treatment methods or kit) may be ineffective in alleviating symptoms of a disease of interest in each subject, they shall alleviate the symptoms of the disease of interest in a statistically significant number of subjects as determined by any statistical test method known in the art, such as the Student's t-test, chi-square test, U-test by Mann and Whitney, Kruskal-Wallis test (H-test), Jonckheere-Terpstra test, and Wilcoxon test.

"Effective amount" comprises an amount sufficient to ameliorate or prevent a symptom of a medical disorder. An effective amount also refers to an amount sufficient to allow or facilitate diagnosis. The effective amount for a subject may vary depending on factors such as the disorder to be treated, the general health of the subject, the method and dose of administration, and the severity of side effects. An effective amount may be the maximum dose or administration regimen to avoid significant side effects or toxic effects. The subject of the present disclosure may be an animal or a human subject.

The pharmaceutical composition of the present disclosure may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection. Various administration schedules are contemplated herein, including but not limited to, single administration or multiple administrations at multiple time points, bolus administration, and pulse infusion. In some embodiments, the pharmaceutical composition of the present disclosure is administered intravenously.

The pharmaceutical composition of the present disclosure will be formulated, administered, and applied in a manner consistent with Good Medical Practice. Factors considered in this context include the specific disorder being treated, the specific mammal being treated, the clinical state of the individual subject, the cause of the disorder, the site of delivery of the agent, the method of administration, the timing of administration, and other factors known to medical practitioners. Optionally, the pharmaceutical composition may also be formulated with one or more additional agents used for preventing or treating the disorder. The effective amount of such additional agents depends on the amount of the antigen-binding molecule present in the pharmaceutical composition, the type of the disorder or treatment, and other factors. Such additional agents may be used in the same dosages and with routes of administration as described herein, or in about 1 to 99% of the dosages described herein, or in any dosage and by any route that is empirically/clinically determined to be appropriate.

The details of one or more embodiments of the present disclosure are set forth in the specification above. Although any methods and materials similar or identical to those described herein can be used in the practice or testing of the present disclosure, the preferred methods and materials are described below. Other features, objects and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

The present disclosure is further described below with reference to examples, which, however, are not intended to limit the scope of the present disclosure. Experimental procedures without specific conditions indicated in the examples or test examples are generally conducted according to conventional conditions, or according to conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

### Examples-Preparation and Detection of Anti-PVRIG/TIGIT Bispecific Antibodies

PCT/CN2021/080470 (filing date: Mar. 13, 2021; priority Patent Application No.: CN202010174835.4) is incorporated herein by reference in its entirety.

### Example 1: Sequence and Preparation of PVRIG Protein

A his-tagged human PVRIG (h-PVRIG-his) recombinant protein, a mouse IgG2a Fc-tagged human PVRIG (h-PVRIG-mIgG2a Fc) recombinant protein, and a human IgG1 Fc-tagged mouse PVRIG (m-PVRIG-hIgG1 Fc) were purified commercial protein reagents purchased from Acrobiosystems, the sequences of which are shown in Table 1.

**Table 1. Amino acid sequences of recombinant proteins**

| Name | Start and end of amino acid sequence | Genbank accession No. |
|---|---|---|
| h-PVRIG-his | Thr41-Asp171 | Q6DKI7-1 |
| h-PVRIG-mIgG2a Fc | Thr41-Asp171 | Q6DKI7-1 |
| m-PVRIG-hIgG1 Fc | Ser35-Asp165 | A0A1B0GS01-1 |

The sequence of the his-tagged cynomolgus monkey PVRIG (cyno-PVRIG-his) recombinant protein is as follows:

TPEVWVQVQMEATELSSFTVHCGFLGPGSISLVTVSWGGPDGAGGTKLAVLHP
ELGTRQWAPARQARWETQSSISLALEDSGASSPFANTTFCCKFASFPEGSWESCG
SLPPSSDPGLSAPPTPVPILRADHHHHHH (SEQ ID NO: 1)

The recombinant protein was expressed in HEK293 cells by transient transfection using conventional methods, and the supernatant was collected and purified by Ni-NTA. Detection was performed, and cyno-PVRIG-his was obtained.

### Example 2: Production of Anti-Human PVRIG Single-Domain Antibodies

Anti-human PVRIG monoclonal single-domain antibodies were produced by immunizing camels. The immune antigen is his-tagged human PVRIG recombinant protein (h-PVRIG-his). Freund's adjuvant (Sigma, Lot No.: F5881/F5506) was used for emulsification, where Freund's complete adjuvant (CFA) CFA was used for primary immunization, and Freund's incomplete adjuvant (IFA) was used for remaining boost immunizations. The immunization injection time was on day 0, day 14, day 28 and day 42. Blood was collected on day 56 for a blood test, and camel serum was tested by the ELISA method to determine the antibody titer in the camel serum.

200 mL of camel peripheral blood was taken, PBMCs were isolated therefrom, and RNA was extracted from the cells using Trizol and reverse-transcribed into cDNA. The genes of the variable region of the single-domain antibodies were amplified by the PCR method and cloned into a phage vector, thereby establishing a phage library of the anti-human PVRIG single-domain antibodies.

The phage library was diluted and blocked with BSA, and co-incubated with magnetic bead Dynabeads (M-280, invitrogen). The phages were collected after negative screening and incubationafter negative screening and incubation. Dynabeads were coated and blocked by biotinylated his-tagged human PVRIG and incubated with a phage suspension collected after negative screening, and then the phages were eluted with pancreatin. After 3 rounds of screening, 400 clones obtained from the 3rd round of screening were selected and sequenced, where the heavy chain sequences of 5 single-domain antibodies are shown in Table 2, and the CDRs of different numbering schemes are shown in Table 3.

**Table 2. Sequences of heavy chain variable regions (HCVRs) of anti-PVRIG antibodies**

| Antibody No. | Amino acid sequence of heavy chain variable region | | Sequence No. |
|---|---|---|---|
| 20 | HCVR | DVQLVESGGGSVQAGGSLRLSCVASRYTSRTDCMGWF RQAPGKEHEGVAHIDSDGIPRYVDSVKGRFTISQDHAK NTLYLQMNSLKPEDSATYYCVVGFKFDDDYCAPNDW GQGTQVTVSS | SEQ ID NO: 2 |
| 30 | HCVR | HVQLVESGGGSVQAGGSLRLSCEASGYSYSGDCMGW FRRAPGKERDEGVATIDNAGRIKYADSVKGRFTISHGN GKYILYLQMNSLKPEDTDMYYCAAGWTFGGNCSPAD WGQGTQVTVSS | SEQ ID NO: 3 |
| 38 | HCVR | QVQLVESGGGSVQAGGSLRLSCAASPSTYGPSDMAWF RQAPGKQREGVATISAAGRLTYYTDSVRGRFTISRDNA KNTMYLQMNSLKPEDTAMYYCAADFAGGSSLFADYK YWGQGTQVTVSS | SEQ ID NO: 4 |
| 39 | HCVR | QVQLVESGGGSVQAGGSLRLSCAASRYTSRTDCMGWF RQAPGKEREGVAHIDSDGIPRYVESVKGRFTISQDHAK NTLYLQMNSLKPEDSATYYCVVGFKFGDYCAPNDWG QGTQVTVSS | SEQ ID NO: 5 |
| 151 | HCVR | HVQLVESGGGSVQAGGSLRLSCVASASGFTYRPYCMA WFRQAPGKEREAVAGIDIFGGTTYADSVKGRFTASRDN AGFSLFLQMNDLKPEDTAMYYCAAGDSPDGRCPPLGQ GLNYWGQGTQVTVSS | SEQ ID NO: 6 |

**Table 3. Sequences of heavy chain complementarity determining regions (CDRs) of anti-PVRIG antibodies**

| Antibody No. | CDR | Kabat numbering scheme | Chothia numbering scheme | IMGT numbering scheme | AbM numbering scheme |
|---|---|---|---|---|---|
| 20 | HCDR1 | TDCMG (SEQ ID NO: 7) | RYTSRTD (SEQ ID NO: 22) | RYTSRTDC (SEQ ID NO: 37) | RYTSRTDCMG (SEQ ID NO: 52) |
| | HCDR2 | HIDSDGIPRYVDSV KG (SEQ ID NO: 8) | DSDGI (SEQ ID NO: 23) | IDSDGIP (SEQ ID NO: 38) | HIDSDGIPR (SEQ ID NO: 53) |
| | HCDR3 | GFKFDDDYCAPND (SEQ ID NO: 9) | GFKFDDDYCAPND (SEQ ID NO: 24) | VVGFKFDDDYCAP ND (SEQ ID NO: 39) | GFKFDDDYCAPND (SEQ ID NO: 54) |
| 30 | HCDR1 | GDCMG (SEQ ID NO: 10) | GYSYSGD (SEQ ID NO: 25) | GYSYSGDC (SEQ ID NO: 40) | GYSYSGDCMG (SEQ ID NO: 55) |
| | HCDR2 | TIDNAGRIKYADSV KG (SEQ ID NO: 11) | DNAGR (SEQ ID NO: 26) | IDNAGRI (SEQ ID NO: 41) | TIDNAGRIK (SEQ ID NO: 56) |
| | HCDR3 | GWTFGGNCSPAD (SEQ ID NO: 12) | GWTFGGNCSPAD (SEQ ID NO: 27) | AAGWTFGGNCSPA D (SEQ ID NO: 42) | GWTFGGNCSPAD (SEQ ID NO: 57) |
| 38 | HCDR1 | PSDMA (SEQ ID NO: 13) | PSTYGPS (SEQ ID NO: 28) | PSTYGPSD (SEQ ID NO: 43) | PSTYGPSDMA (SEQ ID NO: 58) |
| | HCDR2 | TISAAGRLTYYTDS VRG (SEQ ID NO: 14) | SAAGRL (SEQ ID NO: 29) | ISAAGRLT (SEQ ID NO: 44) | TISAAGRLTY (SEQ ID NO: 59) |
| | HCDR3 | DFAGGSSLFADYK Y (SEQ ID NO: 15) | DFAGGSSLFADYK Y (SEQ ID NO: 30) | AADFAGGSSLFAD YKY (SEQ ID NO: 45) | DFAGGSSLFADYK Y (SEQ ID NO: 60) |
| 39 | HCDR1 | TDCMG (SEQ ID NO: 16) | RYTSRTD (SEQ ID NO: 31) | RYTSRTDC (SEQ ID NO: 46) | RYTSRTDCMG (SEQ ID NO: 61) |
| | HCDR2 | HIDSDGIPRYVESV KG (SEQ ID NO: 17) | DSDGI (SEQ ID NO: 32) | IDSDGIP (SEQ ID NO: 47) | HIDSDGIPR (SEQ ID NO: 62) |
| | HCDR3 | GFKFGDYCAPND (SEQ ID NO: 18) | GFKFGDYCAPND (SEQ ID NO: 33) | VVGFKFGDYCAPN D (SEQ ID NO: 48) | GFKFGDYCAPND (SEQ ID NO: 63) |
| 151 | HCDR1 | YRPYCMA (SEQ ID NO: 19) | ASGFTYRPY (SEQ ID NO: 34) | ASGFTYRPYC (SEQ ID NO: 49) | ASGFTYRPYCMA (SEQ ID NO: 64) |
| | HCDR2 | GIDIFGGTTYADSV KG (SEQ ID NO: 20) | DIFGG (SEQ ID NO: 35) | IDIFGGT (SEQ ID NO: 50) | GIDIFGGTT (SEQ ID NO: 65) |
| | HCDR3 | GDSPDGRCPPLGQ GLNY (SEQ ID NO: 21) | GDSPDGRCPPLGQ GLNY (SEQ ID NO: 36) | AAGDSPDGRCPPL GQGLNY (SEQ ID NO: 51) | GDSPDGRCPPLGQ GLNY (SEQ ID NO: 66) |

### Example 3: Preparation of Full-Length Anti-PVRIG Antibodies

The heavy chain variable regions of the 5 antibodies of Example 2 were each linked to the Fc region of the human IgG4 heavy chain to construct full-length anti-PVRIG antibodies. The Fc region of the heavy chain comprised a hinge region and carried S228P, F234A, L235A, and K447A mutations (Eu nomenclature system). The anti-PVRIG antibody CPA.7.021 shown in WO2016134333A1 was screened from a phage library of antibodies, and it was of an IgG1 subtype and was able to well bind to human PVRIG, but it didn't bind to cynomolgus monkey PVRIG. The heavy and light chain variable regions of CPA.7.021 were linked to the human IgG4 heavy chain constant region (with mutations S228P, F234A, L235A, and K447A) and the human Kappa light chain constant region, respectively, to construct a positive antibody Tab5. The full-length sequences of the 5 antibodies and the positive antibody are shown in Table 4.

**Table 4. Full-length sequences of heavy and light chains of fully human anti-PVRIG antibodies**

| Antibody No. | Amino acid sequences of full-length heavy/light chains | | Sequence No. |
|---|---|---|---|
| 20 | HC | DVQLVESGGGSVQAGGSLRLSCVASRYTSRTDCMGWFRQAPG KEHEGVAHIDSDGIPRYVDSVKGRFTISQDHAKNTLYLQMNSL KPEDSATYYCVVGFKFDDDYCAPNDWGQGTQVTVSSESKYGP PCPPCPAPEAAGGPSVFLFPPKPKDTLMISRTPEVTCVVVDVSQ EDPEVQFNWYVDGVEVHNAKTKPREEQFNSTYRVVSVLTVLH QDWLNGKEYKCKVSNKGLPSSIEKTISKAKGQPREPQVYTLPP SQEEMTKNQVSLTCLVKGFYPSDIAVEWESNGQPENNYKTTPP VLDSDGSFFLYSRLTVDKSRWQEGNVFSCSVMHEALHNHYTQ KSLSLSLGA | SEQ ID NO: 67 |
| 30 | HC | HVQLVESGGGSVQAGGSLRLSCEASGYSYSGDCMGWFRRAPG KERDEGVATIDNAGRIKYADSVKGRFTISHGNGKYILYLQMNSL KPEDTDMYYCAAGWTFGGNCSPADWGQGTQVTVSSESKYGP PCPPCPAPEAAGGPSVFLFPPKPKDTLMISRTPEVTCVVVDVSQ EDPEVQFNWYVDGVEVHNAKTKPREEQFNSTYRVVSVLTVLH QDWLNGKEYKCKVSNKGLPSSIEKTISKAKGQPREPQVYTLPP SQEEMTKNQVSLTCLVKGFYPSDIAVEWESNGQPENNYKTTPP VLDSDGSFFLYSRLTVDKSRWQEGNVFSCSVMHEALHNHYTQ KSLSLSLGA | SEQ ID NO: 68 |
| 38 | HC | QVQLVESGGGSVQAGGSLRLSCAASPSTYGPSDMAWFRQAPG KQREGVATISAAGRLTYYTDSVRGRFTISRDNAKNTMYLQMNS LKPEDTAMYYCAADFAGGSSLFADYKYWGQGTQVTVSSESKY GPPCPPCPAPEAAGGPSVFLFPPKPKDTLMISRTPEVTCVVVDVS QEDPEVQFNWYVDGVEVHNAKTKPREEQFNSTYRVVSVLTVL HQDWLNGKEYKCKVSNKGLPSSIEKTISKAKGQPREPQVYTLP PSQEEMTKNQVSLTCLVKGFYPSDIAVEWESNGQPENNYKTTP PVLDSDGSFFLYSRLTVDKSRWQEGNVFSCSVMHEALHNHYTQ KSLSLSLGA | SEQ ID NO: 69 |
| 39 | HC | QVQLVESGGGSVQAGGSLRLSCAASRYTSRTDCMGWFRQAPG KEREGVAHIDSDGIPRYVESVKGRFTISQDHAKNTLYLQMNSLK PEDSATYYCVVGFKFGDYCAPNDWGQGTQVTVSSESKYGPPC PPCPAPEAAGGPSVFLFPPKPKDTLMISRTPEVTCVVVDVSQED PEVQFNWYVDGVEVHNAKTKPREEQFNSTYRVVSVLTVLHQD WLNGKEYKCKVSNKGLPSSIEKTISKAKGQPREPQVYTLPPSQE EMTKNQVSLTCLVKGFYPSDIAVEWESNGQPENNYKTTPPVLD SDGSFFLYSRLTVDKSRWQEGNVFSCSVMHEALHNHYTQKSLS LSLGA | SEQ ID NO: 70 |
| 151 | HC | HVQLVESGGGSVQAGGSLRLSCVASASGFTYRPYCMAWFRQA PGKEREAVAGIDIFGGTTYADSVKGRFTASRDNAGFSLFLQMND LKPEDTAMYYCAAGDSPDGRCPPLGQGLNYWGQGTQVTVSSE SKYGPPCPPCPAPEAAGGPSVFLFPPKPKDTLMISRTPEVTCVVV DVSQEDPEVQFNWYVDGVEVHNAKTKPREEQFNSTYRVVSVL TVLHQDWLNGKEYKCKVSNKGLPSSIEKTISKAKGQPREPQVY TLPPSQEEMTKNQVSLTCLVKGFYPSDIAVEWESNGQPENNYK TTPPVLDSDGSFFLYSRLTVDKSRWQEGNVFSCSVMHEALHNH YTQKSLSLSLGA | SEQ ID NO: 71 |
| Tab5 | HC | EVQLVESGGGVVKPGGSLRLSCAASGFTFGTSSMNWVRQAPG KGLEWVAVISFDGTEIHYADSVKGRFTISRDNSKSTVFLQMNSL RPDDTALYYCAKGSGNIYFYSGMDVWGQGTTVTVSSASTKGP SVFPLAPCSRSTSESTAALGCLVKDYFPEPVTVSWNSGALTSGV HTFPAVLQSSGLYSLSSVVTVPSSSLGTKTYTCNVDHKPSNTKV DKRVESKYGPPCPPCPAPEAAGGPSVFLFPPKPKDTLMISRTPEV | SEQ ID NO: 72 |
| | | TCVVVDVSQEDPEVQFNWYVDGVEVHNAKTKPREEQFNSTYR VVSVLTVLHQDWLNGKEYKCKVSNKGLPSSIEKTISKAKGQPR EPQVYTLPPSQEEMTKNQVSLTCLVKGFYPSDIAVEWESNGQPE NNYKTTPPVLDSDGSFFLYSRLTVDKSRWQEGNVFSCSVMHEA LHNHYTQKSLSLSLGA | |
| | LC | DIQMTQSPSTLSASVGDRVTITCRAGQSISGWLAWFQQKPGKA PNLLIYETSTLESGVPSRFSGSGSGTEYTLTISSLQPDDFATYYCQ QYYSYPLTFGQGTKVEIKRTVAAPSVFIFPPSDEQLKSGTASVVC LLNNFYPREAKVQWKVDNALQSGNSQESVTEQDSKDSTYSLS STLTLSKADYEKHKVYACEVTHQGLSSPVTKSFNRGEC | SEQ ID NO: 73 |

| | | | |
|---|---|---|---|
| (Note: the underlined part is the Fc domain of the heavy chain) | | | |

The gene sequences encoding the above sequences were synthesized, digested with BamHI and XhoI, and inserted into a pcDNA3.1 expression vector (Life Technologies Cat. No. V790-20) through the BamHI/XhoI enzymatic digestion site. The expression vector and transfection reagent PEI (Polysciences, Inc. Cat. No. 23966) were transfected into HEK293 cells (Life Technologies Cat. No. 11625019) in a 1:2 ratio, and the cells were placed in a CO₂ incubator and incubated for 4-5 days. The expressed antibodies were isolated by centrifugation and purified by using a conventional method. Detection was performed and the antibodies of interest were obtained.

### Example 4: Experiment on Binding of Anti-PVRIG Antibodies to PVRIG Recombinant Proteins

ELISA assay was used to detect binding properties of anti-PVRIG antibodies. An his-tagged PVRIG recombinant protein was directly used for coating. After the antibody was added, the activity of the binding of the antibody to the antigen was detected by adding a secondary antibody (HRP-conjugated anti-primary antibody Fc antibody) and HRP substrate TMB.

A human, cynomolgus monkey, or mouse PVRIG protein (at a concentration of 1 µg/mL) was coated on a 96-well microplate at 100 µL per well, and incubated overnight at 4 °C. The plate was washed three times with wash buffer at 250 µL per well. For each wash, the plate was shaken for 10 s to ensure adequate cleaning. A blocking solution (PBS + 0.05% Tween20 + 1% BSA) was added at 300 µL/well, and the plate was incubated at room temperature for 1 h. The plate was washed three times with wash buffer at 250 µL per well. For each wash, the plate was shaken for 10 s to ensure adequate cleaning. Dilutions of the test anti-PVRIG antibodies were added at 100 µL/well. The plate was incubated at 37 °C for 1 h. The plate was washed three times with wash buffer at 250 µL per well. The HRP-labeled anti-human IgG secondary antibody (Sigma, A8667) was added at 100 µL per well. The plate was incubated at 37 °C for 1 h. The plate was washed three times with wash buffer at 250 µL per well. TMB was added at 100 µL/well, and the plate was incubated in the dark for 15 min. 0.16 M sulfuric acid was added at 50 µL/well. The OD values at 450 nm were read by using a Thermo MultiSkanFc microplate reader, and the EC₅₀ values of the binding of the anti-PVRIG antibodies to PVRIG were calculated. All antibodies had a relatively strong binding capacity to human or cynomolgus monkey PVRIG recombinant protein, but they did not bind to mouse PVRIG recombinant protein.

**Table 5. Results of experiment on binding of anti-PVRIG antibodies to PVRIG recombinant proteins of different species**

| Antibody No. | Human PVRIG-his ELISA EC₅₀ (nM) | Monkey PVRIG-his ELISA EC₅₀ (nM) | Mouse PVRIG-Fc ELISA EC₅₀ (nM) |
|---|---|---|---|
| 20 | 0.40 | 0.14 | No binding |
| 30 | 0.26 | 0.16 | No binding |
| 38 | 0.26 | 0.40 | No binding |
| 39 | 0.27 | 0.16 | No binding |
| 151 | 2.15 | 2.43 | No binding |
| Tab5 | 2.86 | No binding | No binding |
| IgG4 | No binding | No binding | No binding |

### Example 5: Experiment on Binding of Anti-PVRIG Antibodies to Cells Expressing PVRIG

A flow cytometer (FACS) was used to detect binding properties of anti-PVRIG antibodies. A cell strain overexpressing human or cynomolgus monkey PVRIG was constructed, and after the addition of the antibody, the activity of the binding of the antibody to the antigen was detected by the addition of a secondary antibody.

The expression plasmid carrying human or cynomolgus monkey PVRIG gene sequence was transfected into HEK293 cells, and the over-expressing stable transfection monoclonal cell strain was obtained by antibiotic screening and infinite dilution. Overexpressing cells were seeded into a 96-well plate at 2 × 10⁵ cells/per well. The cells were centrifuged at 300 g for 5 min, the supernatant was then removed, 100 µL of the test antibody was added, and the mixture was incubated at 4 °C for 1 h. The mixture was centrifuged, the supernatant was removed, the plate was washed 3 times with 200 µL of wash buffer (PBS + 2% FBS), and then 100 µL of an anti-human IgG secondary antibody (Invitrogen, A-11013) labeled with Alexa Fluor 488 diluted at 1:500 was added. The mixture was incubated at 4 °C for 1 h. The plate was centrifuged, and the supernatant was removed. The plate was washed 3 times with 200 µL of wash buffer (PBS + 2% FBS). The cells were resuspended in 100 µL of PBS and tested on a flow cytometer (BD FACS Calibur or BD FACS Canto_ II). All the antibodies showed relatively strong capacities to bind to human or cynomolgus monkey PVRIG expressed on the cell surfaces, and their binding capabilities were significantly stronger than that of the positive antibody Tab5, which did not even bind to cynomolgus monkey PVRIG at all.

**Table 6. Results of experiment on binding of anti-PVRIG antibodies to cells expressing PVRIG of different species**

| Antibody No. | Human PVRIG FACS EC₅₀ (nM) | Monkey PVRIG FACS EC₅₀ (nM) |
|---|---|---|
| 20 | N.A. | N.A. |
| 30 | N.A. | 0.02 |
| 38 | 0.24 | 0.34 |
| 39 | 0.004 | 6.97 |
| 151 | 0.01 | 2.23 |
| Tab5 | 2.13 | No binding |
| IgG4 | No binding | No binding |

| | | |
|---|---|---|
| (Note: N.A., not available, means that the binding is too strong, no dissociation of antibody is present even at low concentrations, and it is impossible to fit to get accurate EC₅₀.) | | |

### Example 6: Experiment on Blocking the Binding of PVRIG to PVRL2 by Anti-PVRIG Antibodies

In this experiment, by *in vitro* blocking experiment, the ability of the selected anti-PVRIG antibodies to block the binding of human PVRIG to its ligand human PVRL2 was detected. Specifically, a mouse IgG2a Fc-tagged human PVRIG recombinant protein (h-PVRIG-mIgG2a Fc) was coated on a 96-well microplate, an anti-PVRIG antibody was added to fully bind to and occupy an epitope, then his-tagged PVRL2 (PV2-H52E2, AcroBiosystem) was added, and the binding amount of PVRIG and PVRL2 was calculated by detecting the his tag. The IC₅₀ value of the anti-PVRIG antibody for blocking the PVRIG active site was calculated.

The h-PVRIG-mIgG2a Fc protein (at a concentration of 1 µg/mL) was coated on the 96-well microplate at 100 µL per well, and incubated overnight at 4 °C. The plate was washed three times with wash buffer at 250 µL per well. For each wash, the plate was shaken for 10 s to ensure adequate cleaning. A blocking solution was added at 300 µL/well, and the plate was incubated at room temperature for 1 h. The plate was washed three times with wash buffer at 250 µL per well. For each wash, the plate was shaken for 10 s to ensure adequate cleaning. 50 µL of diluted test anti-PVRIG antibody and 50 µL of his-tagged ligand PVRL2 were added to each well, and the plate was incubated at 37 °C for 1 h. The plate was washed three times with wash buffer at 250 µL per well. The HRP-labeled anti-his-tagged secondary antibody (Genscrpit) diluted at 1:2000 was added at 100 µL per well. The plate was incubated at 37 °C for 1 h. The plate was washed three times with wash buffer at 250 µL per well. TMB was added at 100 µL/well, and the plate was incubated in the dark for 15 min. 0.16 M sulfuric acid was added at 50 µL/well. OD values at 450 nm were measured using a Thermo MµLtiSkanFc microplate reader, and the IC₅₀ values of the anti-PVRIG antibodies blocking the binding of PVRIG to PVRL2 were calculated.

The results showed that all the antibodies tested had a strong effect in inhibiting the binding of human PVRIG to human PVRL2.

**Table 7. Experiment on blocking of human PVRIG/PVRL2 binding by antibodies**

| Antibody No. | ELISA IC₅₀ (nM) |
|---|---|
| 20 | 1.18 |
| 30 | 1.11 |
| 38 | 0.93 |
| 39 | 0.76 |
| 151 | 0.37 |
| Tab5 | 1.16 |
| IgG4 | No blocking |

### Example 7: Determination of Affinity of Anti-PVRIG Antibodies for PVRIG

A Protein A biosensor (Fortebio, #18-5010) was immersed in 200 µL of KB buffer (PBS, pH 7.4, 0.02% tween-20, 0.1% BSA) for 60 s for the wetting treatment. Then, the anti-PVRIG antibody was diluted to 10 µg/mL with the KB buffer, and the sensor was immersed in 200 µL of the solution until the reading was 1.2 nm. The sensor was immersed in the KB buffer for 100 s to elute excess antibody. The his-tagged human PVRIG was diluted in a 2-fold gradient to 64 nM-4 nM with the KB buffer. The sensor was immersed in the solution for 300 s for binding. The sensor was immersed in the KB buffer for 600 s for dissociation. The data were fitted in a dynamic 1: 1 binding mode. The affinity of anti-PVRIG antibodies to human PVRIG is shown in Table 8.

The results showed that all the antibodies tested had high affinity for human PVRIG.

**Table 8. Affinity of anti-PVRIG antibodies for human PVRIG**

| **Antibody No.** | **Kon (1/Ms)** | **Koff (1/s)** | **KD (M)** |
|---|---|---|---|
| 20 | 3.43E+05 | 8.07E-05 | 2.36E-10 |
| 30 | 2.84E+05 | 2.05E-04 | 7.23E-10 |
| 38 | 1.32E+05 | 2.87E-04 | 2.17E-09 |
| 39 | 2.42E+05 | 1.69E-04 | 6.96E-10 |
| 151 | 2.61E+05 | 5.22E-05 | 2.00E-10 |
| Tab5 | 7.37E+05 | 1.61E-05 | 2.19E-10 |

### Example 8: Experiment on Activity of Anti-PVRIG Antibodies in Reporter Gene Cells

Firstly, a plvx-OS8 (G418 resistance) plasmid was constructed and transfected into 293F cells, and G418 screening was performed. The expression of OS8 by clone cells was tested using a flow cytometer, and meanwhile the activation of Jurkat cells by OS8 was tested. Clones with moderate activation were selected, yielding a 293F-OS8 cell strain. A plvx-PVRL2 plasmid was constructed and used to infect 293F-OS8 cells, and clones with the highest level of PVRL2 expression were obtained by screening using a flow cytometer, thereby yielding a 293F-OS8-PVRL2 cell strain.

Secondly, a plvx-NFAT-Luc (Hygromycin resistance) was constructed and packaged into a lentivirus. The lentivirus was used to infect Jurkat E6.1 cells. Hygromycin was added, and clones with resistance were obtained by screening. The clones were stimulated with OKT3, and clones with moderate Luciferase signals were obtained by screening, yielding a Jurkat-NFAT-Luc cell line. Aplvx-PVRIG (Puromycin resistance) vector was constructed and packaged into a lentivirus, and the lentivirus was used to infect Jurkat-NFAT-Luc cells. Clones with the highest level of PVRIG expression were obtained by screening using a flow cytometer, thereby yielding a Jurkat-NFAT-Luc-PVRIG cell strain.

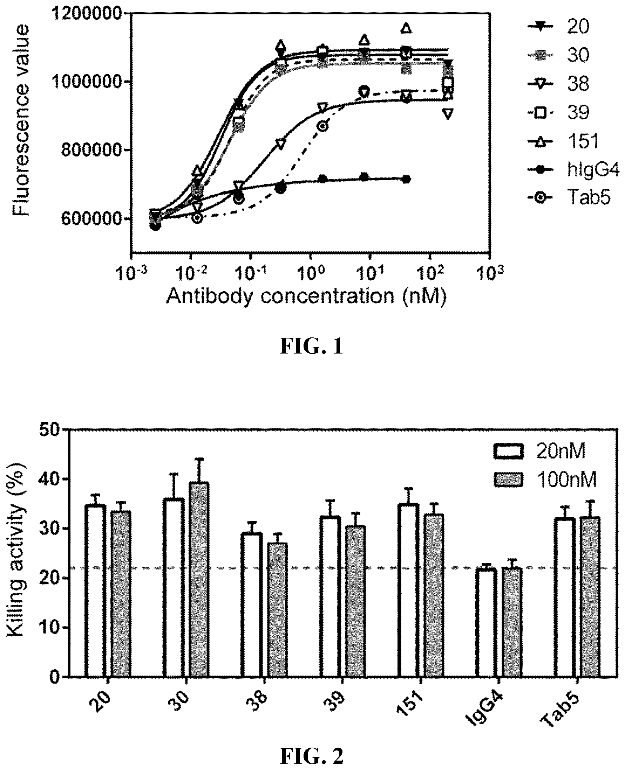

1E4 Jurkat-NFAT-Luc-PVRIG cells were incubated with a test antibody at 37 °C for 20 min. 1E5 293F-OS8-PVRL2 cells were added, and the mixture was incubated at 37 °C for 5 h. The mixture was centrifuged, the supernatant was removed, the Luciferase buffer (Promega, E6130) was added to lyse the cells, and the fluorescence value was detected. EC₅₀ values were calculated and used to evaluate the *in vitro* cell activity of the anti-PVRIG antibodies. The results of the experiment are shown in FIG. 1 and Table 9.

The results showed that all the antibodies tested had relatively strong ability to activate Luciferase in Jurkat cells, and the activity was 3.7-18.5 times that of the positive antibody, which shows that the antibodies can bind to PVRIG and block the binding of PVRL2 to PVRIG.

**Table 9. Results of experiment on activity of anti-PVRIG antibodies in reporter gene cells**

| Antibody strain No. | PVRIG reporter gene cell activity EC₅₀ (nM) |
|---|---|
| 20 | 0.04 |
| 30 | 0.06 |
| 38 | 0.20 |
| 39 | 0.06 |
| 151 | 0.04 |
| Tab5 | 0.74 |
| IgG4 | No binding |

### Example 9: NK Cell Killing Experiment of Anti-PVRIG Antibodies

PVRIG is expressed on NK cells, while PVRL2 is expressed in many tumor cells (including K562 cells). By blocking the binding of PVRL2 to PVRIG, the anti-PVRIG antibodies can relieve the inhibition of the activity of NK cells by tumor cells.

The cultured NK92 cell line (NK cells of a patient with human malignant non-Hodgkin's lymphoma) was washed twice with wash buffer (comprising RPMI 1640, 5% FBS and 10 ng/mL IL-2) and resuspended to a density of 2 × 10⁶ cells/mL. NK92 cells were added to a 96-well plate at 50 µL (1 × 10⁵ cells in total) per well. 50 µL of 20 nM or 100 nM test antibody was added, and the plate was incubated at 37 °C for 30 min. The plate was washed twice with wash buffer, and the cells were resuspended to a density of 2 × 10⁵/mL. Human chronic myeloid leukemia K562 cells were added at 50 µL (1 × 10⁴ cells in total) per well, so that the ratio of the number of NK92 cells to the number of K562 cells was 10:1. The plate was incubated at 37 °C for 4 h. The killing activity was measured using the CytoTox-Glo cytotoxicity system (Promega, G9292). First, 50 µL of AAF-Glo reagent was added. The mixture was incubated at room temperature for 15 min, and the fluorescence of K562 cells killed by NK92 cells was measured. 50 µL of a lysis buffer was added, the mixture was incubated at room temperature for 15 min to lyse all cells in the well, and then the fluorescence of all the cells was measured. Three control groups were prepared, including a sample containing only the culture solution (control group 1), a sample containing only NK92 cells (control group 2), and 150 µL of a sample containing only K562 cells (control group 3), and they were subjected to the same procedure.

The killing activity was calculated according to the following formula: killing activity (%) = {[(R - BG) - (T - BG) - (E - BG)] / [(TL - BGL) - (T - BG)]} × 100 where R represents the fluorescence value after addition of AAF-Glo; BG represents the fluorescence value of control group 1 after addition of AAF-Glo; E represents the fluorescence value of control group 2 after addition of AAF-Glo; and T represents the fluorescence value of control group 3 after addition of AAF-Glo; TL represents the fluorescence value of control group 3 after addition of lysis buffer, and BGL represents the fluorescence value of control group 1 after addition of lysis buffer.

The results of the experiment are shown in FIG. 2 and Table 10, which show that all the anti-PVRIG antibodies tested can significantly activate NK92 cells and kill K562 cells.

**Table 10. NK cell killing experiment of anti-PVRIG antibodies**

| Antibody No. | Killing activity (%) (mean ± standard deviation) | |
|---|---|---|
| | 20 nM antibody | 100 nM antibody |
| 20 | 35±2 | 33±2 |
| 30 | 36±5 | 39±5 |
| 38 | 29±2 | 27±2 |
| 39 | 32±3 | 30±3 |
| 151 | 35±3 | 33±2 |
| Tab5 | 32±2 | 32±3 |
| IgG4 | 22±1 | 22±2 |

### Example 10: Mixed Lymphocyte Reaction (MLR) Experiment of Anti-PVRIG Antibodies

PVRIG is expressed on T cells, while PVRL2 is expressed in dendritic cells (DC cells). By blocking the binding of PVRL2 to PVRIG, the anti-PVRIG antibodies can relieve the inhibition of T cells by dendritic cells and thus activate the T cells.

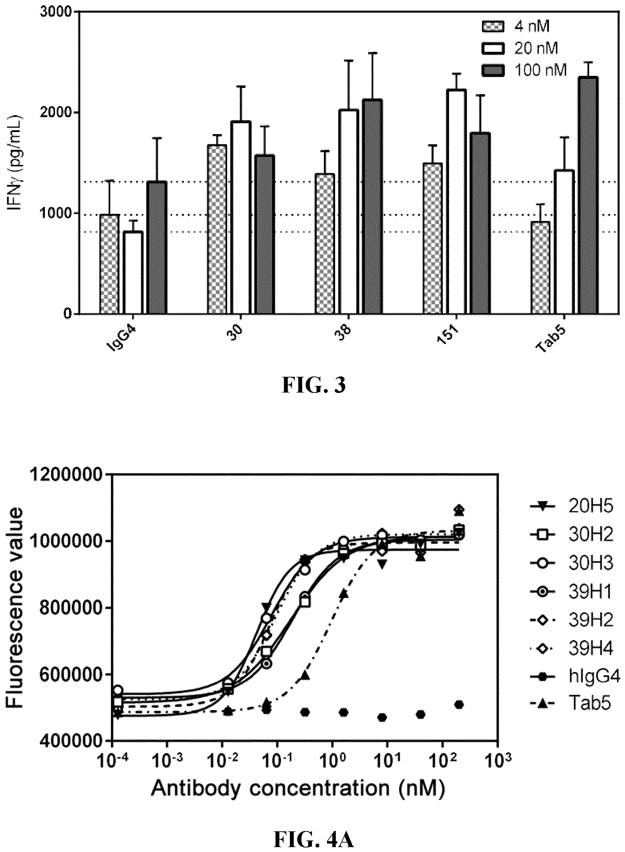

The mixed lymphocyte reaction means that when two unrelated individual lymphocytes with normal functions are co-cultured *in vitro,* the two lymphocytes can mutually stimulate the T cells of each other to proliferate due to different major histocompatibility antigens. PBMCs were isolated from peripheral blood derived from a first individual and cultured in a RPMI 1640 medium containing 10% FBS, cytokines were added at a final concentration of 50 ng/mL GM-CSF (Peprotech, 300-03-100UG) and 50 ng/mL IL-4 (Peprotech, 200-04-100UG), and a fresh medium containing the cytokines was added every 2-3 days; after 6 days of culturing, 1 µg/mL LPS (Sigma, L2880-25MG) was added, the mixture was incubated for 24 h, and DC cells obtained by differentiation and maturation were collected. PBMCs were isolated from peripheral blood of a second source, and then CD3⁺ T cells were isolated from the cells using the EasySep human CD3⁺ T cell isolation kit (Stemcell, 17952). The density of the CD3⁺ T cells and DCs was adjusted, and 1 × 10⁵ CD3⁺ T cells and 2 × 10⁴ DCs were added to each well. The test antibody was added, the mixture was incubated at 37 °C for 120 h, the supernatant was taken, and then the IFNγ content in the supernatant was detected by using an ELISA kit (R&D, DY202). The results of the experiment are shown in FIG. 3 and Table 11.

The results show that compared with the control antibody IgG4, all the anti-PVRIG antibodies tested can significantly activate T cells to secrete IFNγ. Meanwhile, at low doses (such as 4 nM and 20 nM), the antibodies of the present disclosure outperform the positive control Tab5.

**Table 11. IFN γ secretion level in mixed lymphocyte reaction of anti-PVRIG antibodies**

| Antibody No. | IFNγ (pg/mL) (mean ± standard deviation) | | |
|---|---|---|---|
| | 4 nM antibody | 20 nM antibody | 100 nM antibody |
| 30 | 1675±101 | 1911±347 | 1576±288 |
| 38 | 1388±232 | 2024±491 | 2126±465 |
| 151 | 1498±175 | 2224±162 | 1798±373 |
| Tab5 | 912±173 | 1425±330 | 2349±148 |
| IgG4 | 984±335 | 814±112 | 1309±437 |

### Example 11: Humanization of Anti-PVRIG Antibodies

Based on the VH typical structure of the camel single-domain antibodies 20, 30, 38, 39 and 151 obtained, the heavy chain variable region sequence was compared with an antibody GermLine database to obtain a human germline template with high homology. The framework regions of the camel single-domain antibodies were replaced with the heavy chain framework regions of the human germline template and the CDRs (according to the Kabat numbering scheme) were retained, and then they were recombined with the Fc region of human IgG (IgG4 Fc with S228P, F234A, L235A, and K447A mutations). Based on the three-dimensional structure of the camel single-domain antibody, the embedded residues, the residues directly interacting with the CDRs and the residues that had important influence on the conformation of the variable region were subjected to back mutation, and the chemically unstable amino acid residues in the CDRs were optimized to generate a series of humanized single-domain antibodies. The human germline templates and humanized antibody heavy chain variable region sequences for each single-domain antibody are shown in Tables 12-16.

**Table 12. Human germline template and humanized antibody sequences for antibody 20**

| Antibody No. | Amino acid sequence of heavy chain variable region | | Sequence No. |
|---|---|---|---|
| Template IGHV3-7 *01 | HCVR | EVQLVESGGGLVQPGGSLRLSCAASGFTFSSYWMSWV RQAPGKGLEWVANIKQDGSEKYYVDSVKGRFTISRDN AKNSLYLQMNSLRAEDTAVYYCAR | SEQ ID NO: 74 |
| 20H1 | HCVR | EVQLVESGGGLVQPGGSLRLSCAASGFTFSTDCMGWF RQAPGKGLEGVAHIDSDGIPRYVDSVKGRFTISRDNAK | SEQ ID NO: 75 |
| | | NSLYLQMNSLRAEDTAVYYCVVGFKFDEDYCAPNDW GQGTMVTVSS | |
| 20H2 | HCVR | EVQLVESGGGLVQPGGSLRLSCAASGFTSRTDCMGWF RQAPGKGLEGVAHIDSDGIPRYVDSVKGRFTISRDNAK NSLYLQMNSLRAEDTAVYYCVVGFKFDEDYCAPNDW GQGTMVTVSS | SEQ ID NO: 76 |
| 20H3 | HCVR | EVQLVESGGGLVQPGGSLRLSCAASGYTFSTDCMGWF RQAPGKGLEGVAHIDSDGIPRYVDSVKGRFTISQDHAK NSLYLQMNSLRAEDTAVYYCVVGFKFDEDYCAPNDW GQGTMVTVSS | SEQ ID NO: 77 |
| 20H4 | HCVR | EVQLVESGGGLVQPGGSLRLSCAASGYTSRTDCMGWF RQAPGKGLEGVAHIDSDGIPRYVDSVKGRFTISQDHAK NSLYLQMNSLRAEDTAVYYCVVGFKFDEDYCAPNDW GQGTMVTVSS | SEQ ID NO: 78 |
| 20H5 | HCVR | EVQLVESGGGLVQPGGSLRLSCAASGYTSRTDCMGWF RQAPGKEHEGVAHIDSDGIPRYVDSVKGRFTISQDHAK NSLYLQMNSLRAEDTAVYYCVVGFKFDEDYCAPNDW GQGTMVTVSS | SEQ ID NO: 79 |

According to Table 12, antibodies 20H1-20H5 comprise a CDR1 shown as TDCMG (SEQ ID NO: 7), a CDR2 shown as HIDSDGIPRYVDSVKG (SEQ ID NO: 8), and a CDR3 shown as GFKFDEDYCAPND (SEQ ID NO: 150).

**Table 13. Human germline template and humanized antibody sequences for antibody 30**

| Antibody No. | Amino acid sequence of heavy chain variable region | | Sequence No. |
|---|---|---|---|
| Template IGHV3-7 *01 | HCVR | EVQLVESGGGLVQPGGSLRLSCAASGFTFSSYWMSWV RQAPGKGLEWVANIKQDGSEKYYVDSVKGRFTISRDN AKNSLYLQMNSLRAEDTAVYYCAR | SEQ ID NO: 74 |
| 30H1 | HCVR | EVQLVESGGGLVQPGGSLRLSCAASGFTFSGDCMGWF RQAPGKGLEGVATIDNAGRIKYADSVKGRFTISRDNAK NSLYLQMNSLRAEDTAVYYCAAGWTFGGQCSPADWG QGTQVTVSS | SEQ ID NO: 80 |
| 30H2 | HCVR | EVQLVESGGGLVQPGGSLRLSCAASGFTFSGDCMGWF RQAPGKGLDEGVATIDNAGRIKYADSVKGRFTISRDNA KNSLYLQMNSLRAEDTAVYYCAAGWTFGGQCSPADW GQGTQVTVSS | SEQ ID NO: 81 |
| 30H3 | HCVR | EVQLVESGGGLVQPGGSLRLSCAASGYSYSGDCMGWF RQAPGKGLDEGVATIDNAGRIKYADSVKGRFTISRDNA KNSLYLQMNSLRAEDTAVYYCAAGWTFGGQCSPADW GQGTQVTVSS | SEQ ID NO: 82 |
| 30H4 | HCVR | EVQLVESGGGLVQPGGSLRLSCAASGFTFSGDCMGWF RQAPGKGLDEGVATIDNAGRIKYADSVKGRFTISHGNA KYILYLQMNSLRAEDTAVYYCAAGWTFGGQCSPADW GQGTQVTVSS | SEQ ID NO: 83 |
| 30H5 | HCVR | EVQLVESGGGLVQPGGSLRLSCAASGYSYSGDCMGWF RQAPGKGLDEGVATIDNAGRIKYADSVKGRFTISHGNA KYILYLQMNSLRAEDTAVYYCAAGWTFGGQCSPADW GQGTQVTVSS | SEQ ID NO: 84 |

According to Table 13, antibodies 30H1-30H5 comprise a CDR1 shown as GDCMG (SEQ ID NO: 10), a CDR2 shown as TIDNAGRIKYADSVKG (SEQ ID NO: 11), and a CDR3 shown as GWTFGGQCSPAD (SEQ ID NO: 151).

**Table 14. Human germline template and humanized antibody sequences for antibody 38**

| Antibody No. | Amino acid sequence of heavy chain variable region | | Sequence No. |
|---|---|---|---|
| Template IGHV3-30 *02 | HCVR | QVQLVESGGGVVQPGGSLRLSCAASGFTFSSYGMHWV RQAPGKGLEWVAFIRYDGSNKYYADSVKGRFTISRDNS KNTLYLQMNSLRAEDTAVYYCAK | SEQ ID NO: 85 |
| 38H2 | HCVR | EVQLVESGGGVVQPGGSLRLSCAASGFTFSPSDMAWF RQAPGKGLEGVATISAAGRLTYYTDSVRGRFTISRDNS KNTLYLQMNSLRAEDTAVYYCAADFAGGSSLFADYKY WGQGTMVTVSS | SEQ ID NO: 86 |
| 38H4 | HCVR | EVQLVESGGGVVQPGGSLRLSCAASPFTYGPSDMAWF RQAPGKGLEGVATISAAGRLTYYTDSVRGRFTISRDNS KNTLYLQMNSLRAEDTAVYYCAADFAGGSSLFADYKY WGQGTMVTVSS | SEQ ID NO: 87 |
| 38H7 | HCVR | EVQLVESGGGVVQPGGSLRLSCAASPSTYGPSDMAWF RQAPGKQREGVATISAAGRLTYYTDSVRGRFTISRDNS KNTMYLQMNSLRAEDTAVYYCAADFAGGSSLFADYK YWGQGTMVTVSS | SEQ ID NO: 88 |
| 38H8 | HCVR | EVQLVESGGGVVQPGGSLRLSCAASPSTYGPSDMAWF RQAPGKGLEGVATISAAGRLTYYTDSVRGRFTISRDNS KNTLYLQMNSLRAEDTAVYYCAADFAGGSSLFADYKY WGQGTMVTVSS | SEQ ID NO: 89 |
| 38H9 | HCVR | EVQLVESGGGVVQPGGSLRLSCAASPSTYGPSDMAWF RQAPGKGLEGVATISAAGRLTYYTDSVRGRFTISRDNS KNTMYLQMNSLRAEDTAVYYCAADFAGGSSLFADYK YWGQGTMVTVSS | SEQ ID NO: 90 |

**Table 15. Human germline template and humanized antibody sequences for antibody 39**

| Antibody No. | Amino acid sequence of heavy chain variable region | | Sequence No. |
|---|---|---|---|
| Template IGHV3-7 *01 | HCVR | EVQLVESGGGLVQPGGSLRLSCAASGFTFSSYWMSWV RQAPGKGLEWVANIKQDGSEKYYVDSVKGRFTISRDN AKNSLYLQMNSLRAEDTAVYYCAR | SEQ ID NO: 74 |
| 39H1 | HCVR | EVQLVESGGGLVQPGGSLRLSCAASGFTFSTDCMGWF RQAPGKGLEGVAHIDSDGIPRYVESVKGRFTISRDNAK NSLYLQMNSLRAEDTAVYYCVVGFKFGDYCAPNDWG QGTMVTVSS | SEQ ID NO: 91 |
| 39H2 | HCVR | EVQLVESGGGLVQPGGSLRLSCAASGFTSRTDCMGWF RQAPGKGLEGVAHIDSDGIPRYVESVKGRFTISRDNAK NSLYLQMNSLRAEDTAVYYCVVGFKFGDYCAPNDWG QGTMVTVSS | SEQ ID NO: 92 |
| 39H3 | HCVR | EVQLVESGGGLVQPGGSLRLSCAASGYTFSTDCMGWF RQAPGKGLEGVAHIDSDGIPRYVESVKGRFTISQDHAK NSLYLQMNSLRAEDTAVYYCVVGFKFGDYCAPNDWG QGTMVTVSS | SEQ ID NO: 93 |
| 39H4 | HCVR | EVQLVESGGGLVQPGGSLRLSCAASGYTSRTDCMGWF RQAPGKGLEGVAHIDSDGIPRYVESVKGRFTISQDHAK NSLYLQMNSLRAEDTAVYYCVVGFKFGDYCAPNDWG QGTMVTVSS | SEQ ID NO: 94 |
| 39H5 | HCVR | EVQLVESGGGLVQPGGSLRLSCAASGYTSRTDCMGWF RQAPGKEREGVAHIDSDGIPRYVESVKGRFTISQDHAK NSLYLQMNSLRAEDTAVYYCVVGFKFGDYCAPNDWG QGTMVTVSS | SEQ ID NO: 95 |

**Table 16. Human germline template and humanized antibody sequences for antibody 151**

| Antibody No. | Amino acid sequence of heavy chain variable region | | Sequence No. |
|---|---|---|---|
| Template IGHV3-7 *01 | HCVR | EVQLVESGGGLVQPGGSLRLSCAASGFTFSSYWMSWV RQAPGKGLEWVANIKQDGSEKYYVDSVKGRFTISRDN AKNSLYLQMNSLRAEDTAVYYCAR | SEQ ID NO: 74 |
| 151H2 | HCVR | EVQLVESGGGLVQPGGSLRLSCAASGFTYRPYCMAWF RQAPGKGLEAVAGIDIFGGTTYADSVKGRFTISRDNAK NSLYLQMNSLRAEDTAVYYCAAGDSPDGRCPPLGQGL NYWGQGTMVTVSS | SEQ ID NO: 96 |
| 151H4 | HCVR | EVQLVESGGGLVQPGGSLRLSCVASASGFTYRPYCMA WFRQAPGKGLEAVAGIDIFGGTTYADSVKGRFTISRDN AKNSLYLQMNSLRAEDTAVYYCAAGDSPDGRCPPLGQ GLNYWGQGTMVTVSS | SEQ ID NO: 97 |
| 151H7 | HCVR | HVQLVESGGGLVQPGGSLRLSCVASASGFTYRPYCMA WFRQAPGKEREAVAGIDIFGGTTYADSVKGRFTASRDN AGFSLYLQMNSLRAEDTAVYYCAAGDSPDGRCPPLGQ GLNYWGQGTMVTVSS | SEQ ID NO: 98 |
| 151H8 | HCVR | EVQLVESGGGLVQPGGSLRLSCVASASGFTYRPYCMA WFRQAPGKGLEAVAGIDIFGGTTYADSVKGRFTISRDN AGFSLYLQMNSLRAEDTAVYYCAAGDSPDGRCPPLGQ GLNYWGQGTMVTVSS | SEQ ID NO: 99 |
| 151H9 | HCVR | HVQLVESGGGLVQPGGSLRLSCVASASGFTYRPYCMA WFRQAPGKGLEAVAGIDIFGGTTYADSVKGRFTASRDN AGFSLYLQMNSLRAEDTAVYYCAAGDSPDGRCPPLGQ GLNYWGQGTMVTVSS | SEQ ID NO: 100 |

The heavy chain variable regions of the humanized antibodies described above were linked to the heavy chain Fc region of human IgG4 to construct full-length anti-PVRIG antibodies. The Fc region of the heavy chain comprised a hinge region and carried S228P, F234A, L235A, and K447A mutations.
>Fc region of human IgG4 heavy chain (S228P/F234A/L235A/K447A) 
ESKYGPPCPPCPAPEAAGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGA (SEQ ID NO: 101)
>Fc region of human IgG4 heavy chain (S228P/K447A)

ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK (SEQ ID NO: 153)

The antibodies were expressed and purified by conventional methods. It was verified that the antibodies of interest were obtained.

### Example 12: Experiment on Binding of Humanized Anti-PVRIG Antibodies to Cells Expressing PVRIG

Binding of the humanized anti-PVRIG antibodies to human or cynomolgus monkey PVRIG was detected by using a flow cytometer according to the method of Example 5. The results of the experiment are shown in Table 17.

**Table 17. Results of FACS experiment on binding of humanized anti-PVRIG single-domain antibodies to PVRIG of different species**

| Antibody No. | Human PVRIG FACS EC₅₀ (nM) | Monkey PVRIG FACS EC₅₀ (nM) |
|---|---|---|
| 20H1 | 0.019 | 0.658 |
| 20H2 | 0.006 | 0.338 |
| 20H3 | 0.006 | 1.500 |
| 20H4 | 0.008 | 0.591 |
| 20H5 | 0.005 | 0.007 |
| 30H1 | 0.024 | 0.374 |
| 30H2 | 0.003 | 0.005 |
| 30H3 | 0.004 | 0.003 |
| 38H2 | 0.088 | 0.137 |
| 38H4 | 0.062 | 0.095 |
| 38H7 | 0.040 | 0.049 |
| 38H8 | 0.065 | N.T. |
| 38H9 | 0.068 | N.T. |
| 39H1 | 0.004 | 0.251 |
| 39H2 | 0.006 | 0.019 |
| 39H3 | 0.005 | 0.348 |
| 39H4 | 0.005 | 0.006 |
| 39H5 | 0.006 | 0.208 |
| 151H4 | 0.240 | 0.035 |
| 151H7 | 0.002 | 0.467 |
| 151H8 | 0.006 | N.T. |
| 151H9 | 0.004 | 3.942 |
| Tab5 | 0.160 | No binding |
| IgG4 | No binding | No binding |

| | | |
|---|---|---|
| (Note: N.T., not tested.) | | |

### Example 13: Determination of Affinity of Humanized Anti-PVRIG Antibodies for PVRIG

The affinity of the humanized anti-PVRIG antibodies for human PVRIG was detected according to the method of Example 7. The results are shown in Table 18. All the antibodies listed in the Table have high affinity for human PVRIG.

**Table 18. Affinity of humanized anti-PVRIG antibodies for human PVRIG**

| **Antibody No.** | **Kon (1/Ms)** | **Koff (1/s)** | **KD (M)** |
|---|---|---|---|
| 20H5 | 1.93E+05 | 1.35E-05 | 6.98E-11 |
| 30H2 | 1.69E+05 | 3.25E-04 | 1.92E-09 |
| 30H3 | 1.48E+05 | 3.58E-04 | 2.41E-09 |
| 39H1 | 2.64E+05 | 8.65E-04 | 3.28E-09 |
| 39H2 | 1.80E+05 | 1.24E-04 | 6.92E-10 |
| 39H4 | 1.89E+05 | 9.11E-05 | 4.82E-10 |
| 151H7 | 1.57E+05 | 1.88E-04 | 1.20E-09 |

### Example 14: Experiment on Activity of Humanized Anti-PVRIG Antibodies in Reporter Gene Cells

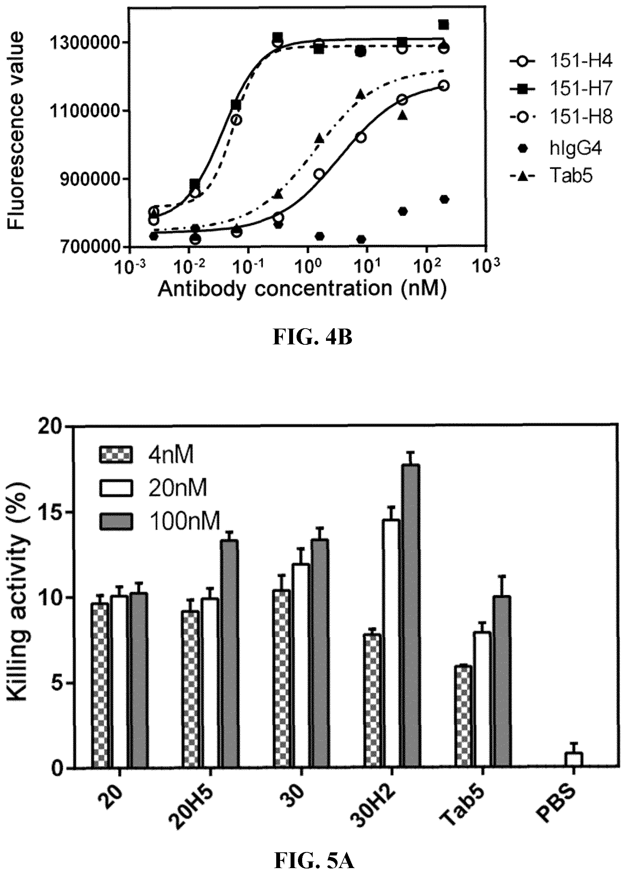

The activity of the humanized anti-PVRIG antibodies in reporter gene cells was detected according to the method of Example 8. The results of the experiment are shown in FIGs. 4A-4B and Table 19. All the antibodies listed in the Table have the ability to activate Jurkat cells.

**Table 19. Experiment on activity of humanized anti-PVRIG antibodies in reporter gene cells**

| Antibody No. | PVRIG reporter gene cell activity ECso (nM) |
|---|---|
| 20H5 | 0.042 |
| 30H2 | 0.176 |
| 30H3 | 0.078 |
| 39H1 | 0.191 |
| 39H2 | 0.074 |
| 39H4 | 0.094 |
| 151H4 | 3.549 |
| 151H7 | 0.038 |
| 151H8 | 0.058 |
| Tab5 | 1.380 |
| IgG4 | No activation |

### Example 15: Experiment on Activation of NK Cell Killing Ability by Humanized Anti-PVRIG Antibodies

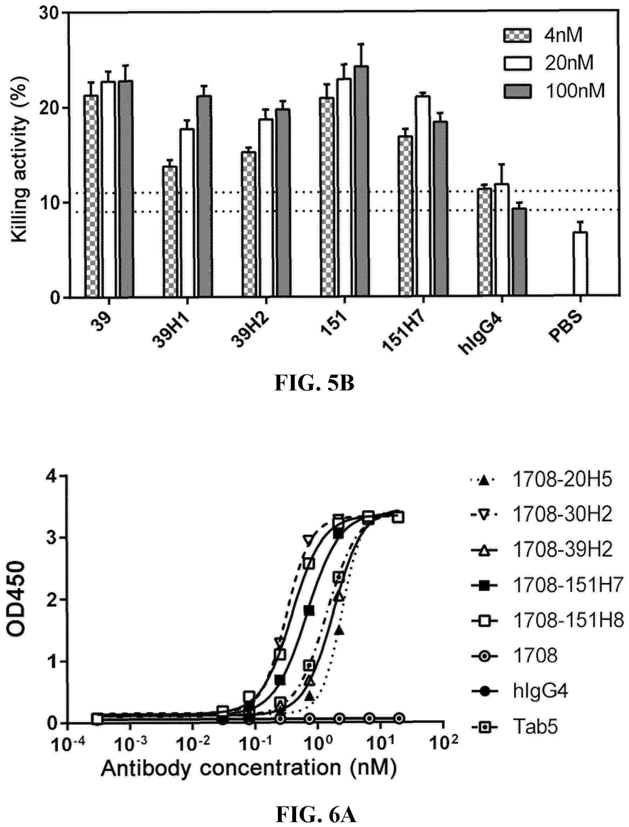

The ability of the humanized anti-PVRIG antibodies to activate NK cells was detected according to the method of Example 9. The results of the experiment are shown in FIGs. 5A-5B and Tables 20-21. The results show that the humanized anti-PVRIG antibodies of the present disclosure can significantly activate NK cells and promote the killing of target cells K562 by the NK cells.

**Table 20. NK cell killing experiment of humanized anti-PVRIG antibodies**

| Antibody No. | Killing activity (%) (mean ± standard deviation) | | |
|---|---|---|---|
| | 4 nM antibody | 20 nM antibody | 100 nM antibody |
| 20 | 9.6±0.5 | 10.1±0.5 | 10.2±0.6 |
| 20H5 | 9.2±0.7 | 9.9±0.6 | 13.3±0.5 |
| 30 | 10.4±0.9 | 11.9±0.9 | 13.3±0.7 |
| 30H2 | 7.7±0.3 | 14.5±0.8 | 17.7±0.7 |
| TabS | 5.9±0.1 | 7.9±0.6 | 10.0±1.2 |
| PBS | 0.8±0.6 | | |

**Table 21. NK cell killing experiment of humanized anti-PVRIG antibodies**

| Antibody No. | Killing activity (%) (mean ± standard deviation) | | |
|---|---|---|---|
| | 4 nM antibody | 20 nM antibody | 100 nM antibody |
| 39 | 21.3±1.4 | 22.7±1.1 | 22.8±1.6 |
| 39H1 | 13.7±0.7 | 17.7±0.9 | 21.2±1.1 |
| 39H2 | 15.3±0.5 | 18.7±1.0 | 19.7±0.9 |
| 151 | 20.9±1.5 | 22.9±1.5 | 24.2±2.3 |
| 151H7 | 16.9±0.8 | 21.0±0.4 | 18.4±0.9 |
| IgG4 | 11.2±0.4 | 11.7±2.1 | 9.2±0.6 |
| PBS | 6.6±1.1 | | |

### Example 16: Preparation of Anti-PVRIG/TIGIT Bispecific Antibodies

To explore the effect of differently configured anti-PVRIG/TIGIT bispecific antibodies on antibody functions, the anti-PVRIG single-domain antibody 151 was linked to the N-terminus or C-terminus of the heavy or light chain of the anti-TIGIT antibody 1708 via the linker GGGGSGGGGS (SEQ ID NO: 152). Four anti-PVRIG/TIGIT bispecific antibodies were formed and named 1708-151-1, 1708-151-2, 1708-151-3 and 1708-151-4, which corresponded to the cases where 151 was linked to the heavy chain N-terminal, heavy chain C-terminal, light chain N-terminal and light chain C-terminal of 1708, respectively. The anti-TIGIT antibody 1708 is of the human IgG4 subtype and has the mutation S228P (Eu naming system). Sequences of the anti-TIGIT antibody 1708 and the bispecific antibodies formed thereby together with 151 are shown in Table 22 below. Sequence information about anti-TIGIT antibodies is shown in Tables 23-24. The TIGIT antibody in WO2019062832A1 is incorporated herein by reference in its entirety.

**Table 22. Sequences of first and second polypeptide chains of anti-PVRIG/TIGIT bispecific antibodies**

| Antibody No. | Amino acid sequences of full-length heavy/light chains | | Sequence No. |
|---|---|---|---|
| 1708 (anti-TIGIT antibody) | First polypeptide chain | EVQLVQSGAEVKKPGASVKVSCKASGYTFTNYWMH WVRQAPGQGLEWMGRIDPDSTGSKYNEKFKTRVTMT RDTSTSTVYMELSSLRSEDTAVYYCAREGAYGYYFDY WGQGTLVTVSSASTKGPSVFPLAPCSRSTSESTAALGC LVKDYFPEPVTVSWNSGALTSGVHTFPAVLQSSGLYSL SSVVTVPSSSLGTKTYTCNVDHKPSNTKVDKRVESKY GPPCPPCPAPEFLGGPSVFLFPPKPKDTLMISRTPEVTC VVVDVSQEDPEVQFNWYVDGVEVHNAKTKPREEQFN STYRVVSVLTVLHQDWLNGKEYKCKVSNKGLPSSIEK TISKAKGQPREPQVYTLPPSQEEMTKNQVSLTCLVKGF YPSDIAVEWESNGQPENNYKTTPPVLDSDGSFFLYSRL TVDKSRWQEGNVFSCSVMHEALHNHYTQKSLSLSLG K | SEQ ID NO: 102 |
| | Second polypeptide chain | DIQMTQSPSSLSASVGDRVTITCRASENIYSYLAWYQQ KPGKSPKLLIYNARTLAEGVPSRFSGSGSGTDFTLTISS LQPEDFATYYCQYHSGSPLPFGGGTKVEIKRTVAAPSV FIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC | SEQ ID NO: 103 |
| 1708-151-1 (antibody 151 linked to N-terminus of heavy chain of 1708) | First polypeptide chain | HVQLVESGGGSVQAGGSLRLSCVASASGFTYRPYCMA WFRQAPGKEREAVAGIDIFGGTTYADSVKGRFTASRDN AGFSLFLQMNDLKPEDTAMYYCAAGDSPDGRCPPLGQ GLNYWGQGTQVTVSSGGGGSGGGGSEVQLVQSGAEV KKPGASVKVSCKASGYTFTNYWMHWVRQAPGQGLE WMGRIDPDSTGSKYNEKFKTRVTMTRDTSTSTVYMEL SSLRSEDTAVYYCAREGAYGYYFDYWGQGTLVTVSSA STKGPSVFPLAPCSRSTSESTAALGCLVKDYFPEPVTVS WNSGALTSGVHTFPAVLQSSGLYSLSSVVTVPSSSLGTK TYTCNVDHKPSNTKVDKRVESKYGPPCPPCPAPEFLGG PSVFLFPPKPKDTLMISRTPEVTCVVVDVSQEDPEVQFN WYVDGVEVHNAKTKPREEQFNSTYRVVSVLTVLHQD WLNGKEYKCKVSNKGLPSSIEKTISKAKGQPREPQVYT LPPSQEEMTKNQVSLTCLVKGFYPSDIAVEWESNGQPE NNYKTTPPVLDSDGSFFLYSRLTVDKSRWQEGNVFSCS VMHEALHNHYTQKSLSLSLGK | SEQ ID NO: 104 |
| | Second polypeptide chain | Same as the light chain of 1708 | SEQ ID NO: 103 |
| 1708-151-2 (151 linked to C terminus of heavy chain of 1708) | First polypeptide chain | EVQLVQSGAEVKKPGASVKVSCKASGYTFTNYWMHW VRQAPGQGLEWMGRIDPDSTGSKYNEKFKTRVTMTR DTSTSTVYMELSSLRSEDTAVYYCAREGAYGYYFDYW GQGTLVTVSSASTKGPSVFPLAPCSRSTSESTAALGCLV KDYFPEPVTVSWNSGALTSGVHTFPAVLQSSGLYSLSSV VTVPSSSLGTKTYTCNVDHKPSNTKVDKRVESKYGPPC PPCPAPEFLGGPSVFLFPPKPKDTLMISRTPEVTCVVVD VSQEDPEVQFNWYVDGVEVHNAKTKPREEQFNSTYR VVSVLTVLHQDWLNGKEYKCKVSNKGLPSSIEKTISKA KGQPREPQVYTLPPSQEEMTKNQVSLTCLVKGFYPSDI AVEWESNGQPENNYKTTPPVLDSDGSFFLYSRLTVDKS RWQEGNVFSCSVMHEALHNHYTQKSLSLSLGKGGGG SGGGGSHVQLVESGGGSVQAGGSLRLSCVASASGFTY RPYCMAWFRQAPGKEREAVAGIDIFGGTTYADSVKGRF TASRDNAGFSLFLQMNDLKPEDTAMYYCAAGDSPDGR CPPLGQGLNYWGQGTQVTVSS | SEQ ID NO: 105 |
| | Second polypeptide chain | Same as the light chain of 1708 | SEQ ID NO: 103 |
| 1708-151-3 (151 linked to N-terminus of light chain of 1708) | First polypeptide chain | Same as the heavy chain of 1708 | SEQ ID NO: 102 |
| | Second polypeptide chain | HVQLVESGGGSVQAGGSLRLSCVASASGFTYRPYCMA WFRQAPGKEREAVAGIDIFGGTTYADSVKGRFTASRDN AGFSLFLQMNDLKPEDTAMYYCAAGDSPDGRCPPLGQ GLNYWGQGTQVTVSSGGGGSGGGGSDIQMTQSPSSLS ASVGDRVTITCRASENIYSYLAWYQQKPGKSPKLLIYN ARTLAEGVPSRFSGSGSGTDFTLTISSLQPEDFATYYCQ YHSGSPLPFGGGTKVEIKRTVAAPSVFIFPPSDEQLKSGT ASVVCLLNNFYPREAKVQWKVDNALQSGNSQESVTE QDSKDSTYSLSSTLTLSKADYEKHKVYACEVTHQGLSS PVTKSFNRGEC | SEQ ID NO: 106 |
| 1708-151-4 (151 linked to | First polypeptide chain | Same as the heavy chain of 1708 | SEQ ID NO: 102 |
| C-terminus of light chain of 1708) | Second polypeptide chain | DIQMTQSPSSLSASVGDRVTITCRASENIYSYLAWYQQ KPGKSPKLLIYNARTLAEGVPSRFSGSGSGTDFTLTISSL QPEDFATYYCQYHSGSPLPFGGGTKVEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGECGGGGSGGGGSHVQLV ESGGGSVQAGGSLRLSCVASASGFTYRPYCMAWFRQA PGKEREAVAGIDIFGGTTYADSVKGRFTASRDNAGFSLF LQMNDLKPEDTAMYYCAAGDSPDGRCPPLGQGLNYW GQGTQVTVSS | SEQ ID NO: 107 |

**Table 23. Sequences of heavy and light chain CDRs of anti-TIGIT antibodies (Kabat numbering scheme)**

| Antibody | Heavy chain | | Light chain | |
|---|---|---|---|---|
| 1707 | HCDR1 | DYHMY (SEQ ID NO: 115) | LCDR1 | KASQDVGTSVA (SEQ ID NO: 118) |
| | HCDR2 | YISKGGISTYYPDTVKG (SEQ ID NO: 116) | LCDR2 | WASARHT (SEQ ID NO: 119) |
| | HCDR3 | QSSYDFAMDY (SEQ ID NO: 117) | LCDR3 | QQYSSYPLT (SEQ ID NO: 120) |
| 1708 | HCDR1 | NYWMH (SEQ ID NO: 121) | LCDR1 | RASENIYSYLA (SEQ ID NO: 124) |
| | HCDR2 | RIDPDSTGSKYNEKFKT (SEQ ID NO: 122) | LCDR2 | NARTLAE (SEQ ID NO: 125) |
| | HCDR3 | EGAYGYYFDY (SEQ ID NO: 123) | LCDR3 | QYHSGSPLP (SEQ ID NO: 126) |
| 1709 | HCDR1 | DYYMH (SEQ ID NO: 127) | LCDR1 | KASQNWTAVA (SEQ ID NO: 130) |
| | HCDR2 | LVYPYNDNTGYNRKFKG (SEQ ID NO: 128) | LCDR2 | SASNRYT (SEQ ID NO: 131) |
| | HCDR3 | GGPSNWNYFDY (SEQ ID NO: 129) | LCDR3 | QQYTLYPLT (SEQ ID NO: 132) |
| 1710 | HCDR1 | NYYMH (SEQ ID NO: 133) | LCDR1 | RTSENIFTYLA (SEQ ID NO: 136) |
| | HCDR2 | RIDPTSGATKYNDNFKG (SEQ ID NO: 134) | LCDR2 | NAKTFAE (SEQ ID NO: 137) |
| | HCDR3 | EGGFGYYFDY (SEQ ID NO: 135) | LCDR3 | QHHYGIPLP (SEQ ID NO: 138) |
| 1711 | HCDR1 | NYWIG (SEQ ID NO: 139) | LCDR1 | KSSQSLLYSRNQMNYLA (SEQ ID NO: 142) |
| | HCDR2 | DIYPGGAYTNYNEKFKD (SEQ ID NO: 140) | LCDR2 | WTSTRES (SEQ ID NO: 143) |
| | HCDR3 | GDYYDSSGRAMDY (SEQ ID NO: 141) | LCDR3 | QQYYSYPYT (SEQ ID NO: 144) |

**Table 24. Sequences of heavy chain VH and light chain VL of anti-TIGIT antibodies**

| Antibody | Sequences of heavy chain VHs and light chain VLs |
|---|---|
| 1708-VH1 | EVQLVQSGAEVKKPGASVKVSCKASGYTFTNYWMHWVRQAPGQGLEWMGRID PDSTGSKYNEKFKTRVTMTRDTSTSTVYMELSSLRSEDTAVYYCAREGAYGYYF DYWGQGTLVTVSS (SEQ ID NO: 145) |
| 1708-VH2 | EVQLVQSGAEVKKPGASVKVSCKASGYTFTNYWMHWVRQAPGQGLEWMGRID PDSTGSKYNEKFKTRVTMTVDTSTSTVYMELSSLRSEDTAVYYCAREGAYGYYF DYWGQGTLVTVSS (SEQ ID NO: 146) |
| 1708-VH3 | EVQLVQSGAEVKKPGASVKVSCKASGYTFTNYWMHWVRQAPGQGLEWIGRIDP DSTGSKYNEKFKTRVTMTVDTSTSTAYMELSSLRSEDTAVYYCAREGAYGYYFD YWGQGTLVTVSS (SEQ ID NO: 147) |
| 1708-VL1 | DIQMTQSPSSLSASVGDRVTITCRASENIYSYLAWYQQKPGKAPKLLIYNARTLAE GVPSRFSGSGSGTDFTLTISSLQPEDFATYYCQYHSGSPLPFGGGTKVEIK (SEQ ID NO: 148) |
| 1708-VL2 | DIQMTQSPSSLSASVGDRVTITCRASENIYSYLAWYQQKPGKSPKLLIYNARTLAE GVPSRFSGSGSGTDFTLTISSLQPEDFATYYCQYHSGSPLPFGGGTKVEIK (SEQ ID NO: 149) |

Transient transfection, expression and purification of the antibodies were carried out according to conventional methods. Identification was performed, and the full-length anti-PVRIG/TIGIT bispecific antibodies of the present disclosure were obtained. The expression level and purity of the bispecific antibodies are shown in Table 25. Coupling a nanobody to a common monoclonal antibody, whether via the N-terminus or the C-terminus of a heavy or light chain, results in good expression level and purity.

**Table 25. Expression level and purity of anti-PVRIG/TIGIT bispecific antibodies**

| Antibody No. | Expression level (mg/L) | SEC purity (%) |
|---|---|---|
| 1708-151-1 | 140 | 97.6 |
| 1708-151-2 | 108 | 95.7 |
| 1708-151-3 | 160 | 94.7 |
| 1708-151-4 | 158 | 96.2 |

### Example 17: Experiment on Binding of Anti-PVRIG/TIGIT Bispecific Antibodies to PVRIG and TIGIT and Blocking of Corresponding Ligands

### 1. Binding of bispecific antibodies with different configurations to human PVRIG and blocking of ligand PVRL2

The experiments were carried out according to the methods of Example 4, Example 5 and Example 6, and the results are shown in Table 26. The results show that the bispecific antibodies with different configurations are basically the same and have no difference in binding to human PVRIG recombinant protein and cells over-expressing human PVRIG and in blocking the binding of PVRL2 to PVRIG.

**Table 26. Binding of bispecific antibodies to PVRIG and blocking of ligand**

| Antibody No. | Binding to human PVRIG recombinant protein EC₅₀ (nM) | EC₅₀ (nM) of binding to cells overexpressing human PVRIG | Blocking binding of human PVRIG to human PVRL2 IC₅₀ (nM) |
|---|---|---|---|
| 1708-151-1 | 1.864 | 0.05 | 0.757 |
| 1708-151-2 | 2.391 | 0.21 | 0.824 |
| 1708-151-3 | 1.579 | 0.06 | 0.712 |
| 1708-151-4 | 1.671 | 0.49 | 0.743 |
| Tab5 | 1.857 | 3.15 | 0.797 |
| IgG4 | No binding | No binding | No blocking |

### 2. Binding of bispecific antibodies with different configurations to human TIGIT and blocking of ligand PVR

The experiments were carried out according to the methods of Example 4, Example 5 and Example 6 (corresponding receptors and ligands were replaced with human TIGIT and human PVR), and the results are shown in Table 27. The results show that the bispecific antibodies with different configurations and the anti-TIGIT antibody are basically the same and have no difference in binding to human TIGIT recombinant protein and cells over-expressing human TIGIT and in blocking the binding of TIGIT to its ligand PVR. The manner of linkage of the anti-PVRIG antibody 151 almost has no effect on the binding of anti-TIGIT antibody to TIGIT.

**Table 27. Binding of bispecific antibodies to TIGIT and blocking of ligand**

| Antibody No. | Binding to human TIGT recombinant protein EC₅₀ (nM) | EC₅₀ (nM) of binding to cells overexpressing human TIGIT | Blocking binding of human TIGIT to human PVR IC₅₀ (nM) |
|---|---|---|---|
| 1708-151-1 | 0.101 | 1.51 | 0.96 |
| 1708-151-2 | 0.090 | 1.27 | 1.04 |
| 1708-151-3 | 0.069 | 1.92 | 0.62 |
| 1708-151-4 | 0.054 | 1.00 | 0.69 |
| 1708 | 0.055 | 0.74 | 0.87 |
| IgG4 | No binding | No binding | No blocking |

With reference to the data in Tables 26-27, it is found that the anti-PVRIG antibody, whether linked to the N-terminus or C-terminus of the heavy or light chain of the anti-TIGIT antibody, maintains its binding to PVRIG and TIGIT and blocking of the ligand, and shows good expression level and purity.

### Example 18: Preparation of Humanized Anti-PVRIG/TIGIT Bispecific Antibodies

Different humanized anti-PVRIG antibodies (20H5, 30H2, 39H2, 151H7, and 151H8) were each linked to the N-terminus of the heavy chain of the anti-TIGIT antibody 1708 (i.e., using a bispecific antibody configuration similar to 1708-151-1) to construct diabodies, and the sequences are shown in Table 28.

**Table 28. Full-length sequences of first and second polypeptide chains of humanized anti-PVRIG/TIGIT bispecific antibodies**

| Antibody No. | Full-length amino acid sequences of first and second polypeptide chains | | Sequence No. |
|---|---|---|---|
| 1708-20H5 | First polypeptide chain | EVQLVESGGGLVQPGGSLRLSCAASGYTSRTDCMGWF RQAPGKEHEGVAHIDSDGIPRYVDSVKGRFTISQDHAK NSLYLQMNSLRAEDTAVYYCVVGFKFDEDYCAPNDW GQGTMVTVSSGGGGSGGGGSEVQLVQSGAEVKKPGA SVKVSCKASGYTFTNYWMHWVRQAPGQGLEWMGRI DPDSTGSKYNEKFKTRVTMTRDTSTSTVYMELSSLRSE DTAVYYCAREGAYGYYFDYWGQGTLVTVSSASTKGPS VFPLAPCSRSTSESTAALGCLVKDYFPEPVTVSWNSGA LTSGVHTFPAVLQSSGLYSLSSVVTVPSSSLGTKTYTCN VDHKPSNTKVDKRVESKYGPPCPPCPAPEFLGGPSVFL | SEQ ID NO: 108 |
| | | FPPKPKDTLMISRTPEVTCVVVDVSQEDPEVQFNWYV DGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNY KTTPPVLDSDGSFFLYSRLTVDKSRWQEGNVFSCSVMH EALHNHYTQKSLSLSLGK | |
| | Second polypeptide chain | Same as the light chain of 1708 | SEQ ID NO: 103 |
| 1708-30H2 | First polypeptide chain | EVQLVESGGGLVQPGGSLRLSCAASGFTFSGDCMGWF RQAPGKGLDEGVATIDNAGRIKYADSVKGRFTISRDNA KNSLYLQMNSLRAEDTAVYYCAAGWTFGGQCSPADW GQGTQVTVSSGGGGSGGGGSEVQLVQSGAEVKKPGA SVKVSCKASGYTFTNYWMHWVRQAPGQGLEWMGRI DPDSTGSKYNEKFKTRVTMTRDTSTSTVYMELSSLRSE DTAVYYCAREGAYGYYFDYWGQGTLVTVSSASTKGPS VFPLAPCSRSTSESTAALGCLVKDYFPEPVTVSWNSGA LTSGVHTFPAVLQSSGLYSLSSVVTVPSSSLGTKTYTCN VDHKPSNTKVDKRVESKYGPPCPPCPAPEFLGGPSVFL FPPKPKDTLMISRTPEVTCVVVDVSQEDPEVQFNWYV DGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNY KTTPPVLDSDGSFFLYSRLTVDKSRWQEGNVFSCSVMH EALHNHYTQKSLSLSLGK | SEQ ID NO: 109 |
| | Second polypeptide chain | Same as the light chain of 1708 | SEQ ID NO: 103 |
| 1708-39H2 | First polypeptide chain | EVQLVESGGGLVQPGGSLRLSCAASGFTSRTDCMGWF RQAPGKGLEGVAHIDSDGIPRYVESVKGRFTISRDNAK NSLYLQMNSLRAEDTAVYYCVVGFKFGDYCAPNDWG QGTMVTVSSGGGGSGGGGSEVQLVQSGAEVKKPGAS VKVSCKASGYTFTNYWMHWVRQAPGQGLEWMGRID PDSTGSKYNEKFKTRVTMTRDTSTSTVYMELSSLRSED TAVYYCAREGAYGYYFDYWGQGTLVTVSSASTKGPSV FPLAPCSRSTSESTAALGCLVKDYFPEPVTVSWNSGALT SGVHTFPAVLQSSGLYSLSSVVTVPSSSLGTKTYTCNVD HKPSNTKVDKRVESKYGPPCPPCPAPEFLGGPSVFLFPP KPKDTLMISRTPEVTCVVVDVSQEDPEVQFNWYVDGV EVHNAKTKPREEQFNSTYRVVSVLTVLHQDWLNGKE YKCKVSNKGLPSSIEKTISKAKGQPREPQVYTLPPSQEE MTKNQVSLTCLVKGFYPSDIAVEWESNGQPENNYKTT PPVLDSDGSFFLYSRLTVDKSRWQEGNVFSCSVMHEAL HNHYTQKSLSLSLGK | SEQ ID NO: 110 |
| | Second polypeptide chain | Same as the light chain of 1708 | SEQ ID NO: 103 |
| 1708-151H7 | First polypeptide chain | HVQLVESGGGLVQPGGSLRLSCVASASGFTYRPYCMA WFRQAPGKEREAVAGIDIFGGTTYADSVKGRFTASRDN AGFSLYLQMNSLRAEDTAVYYCAAGDSPDGRCPPLGQ GLNYWGQGTMVTVSSGGGGSGGGGSEVQLVQSGAEV KKPGASVKVSCKASGYTFTNYWMHWVRQAPGQGLE WMGRIDPDSTGSKYNEKFKTRVTMTRDTSTSTVYMEL SSLRSEDTAVYYCAREGAYGYYFDYWGQGTLVTVSSA STKGPSVFPLAPCSRSTSESTAALGCLVKDYFPEPVTVS WNSGALTSGVHTFPAVLQSSGLYSLSSVVTVPSSSLGTK TYTCNVDHKPSNTKVDKRVESKYGPPCPPCPAPEFLGG PSVFLFPPKPKDTLMISRTPEVTCVVVDVSQEDPEVQF NWYVDGVEVHNAKTKPREEQFNSTYRVVSVLTVLHQ | SEQ ID NO: 111 |
| | | DWLNGKEYKCKVSNKGLPSSIEKTISKAKGQPREPQV YTLPPSQEEMTKNQVSLTCLVKGFYPSDIAVEWESNGQ PENNYKTTPPVLDSDGSFFLYSRLTVDKSRWQEGNVFS CSVMHEALHNHYTQKSLSLSLGK | |
| | Second polypeptide chain | Same as the light chain of 1708 | SEQ ID NO: 103 |
| 1708-151H8 | First polypeptide chain | EVQLVESGGGLVQPGGSLRLSCVASASGFTYRPYCMA WFRQAPGKGLEAVAGIDIFGGTTYADSVKGRFTISRDN AGFSLYLQMNSLRAEDTAVYYCAAGDSPDGRCPPLGQ GLNYWGQGTMVTVSSGGGGSGGGGSEVQLVQSGAEV KKPGASVKVSCKASGYTFTNYWMHWVRQAPGQGLE WMGRIDPDSTGSKYNEKFKTRVTMTRDTSTSTVYMEL SSLRSEDTAVYYCAREGAYGYYFDYWGQGTLVTVSSA STKGPSVFPLAPCSRSTSESTAALGCLVKDYFPEPVTVS WNSGALTSGVHTFPAVLQSSGLYSLSSVVTVPSSSLGTK TYTCNVDHKPSNTKVDKRVESKYGPPCPPCPAPEFLGG PSVFLFPPKPKDTLMISRTPEVTCVVVDVSQEDPEVQF NWYVDGVEVHNAKTKPREEQFNSTYRVVSVLTVLHQ DWLNGKEYKCKVSNKGLPSSIEKTISKAKGQPREPQV YTLPPSQEEMTKNQVSLTCLVKGFYPSDIAVEWESNGQ PENNYKTTPPVLDSDGSFFLYSRLTVDKSRWQEGNVFS CSVMHEALHNHYTQKSLSLSLGK | SEQ ID NO: 112 |
| | Second polypeptide chain | Same as the light chain of 1708 | SEQ ID NO: 103 |

Transient transfection, expression and purification of the antibodies were carried out according to conventional methods. Identification was performed, and the diabodies of interest were obtained.

### Example 19: Binding of Humanized Anti-PVRIG/TIGIT Bispecific Antibodies to PVRIG and TIGIT and Blocking of Corresponding Ligands

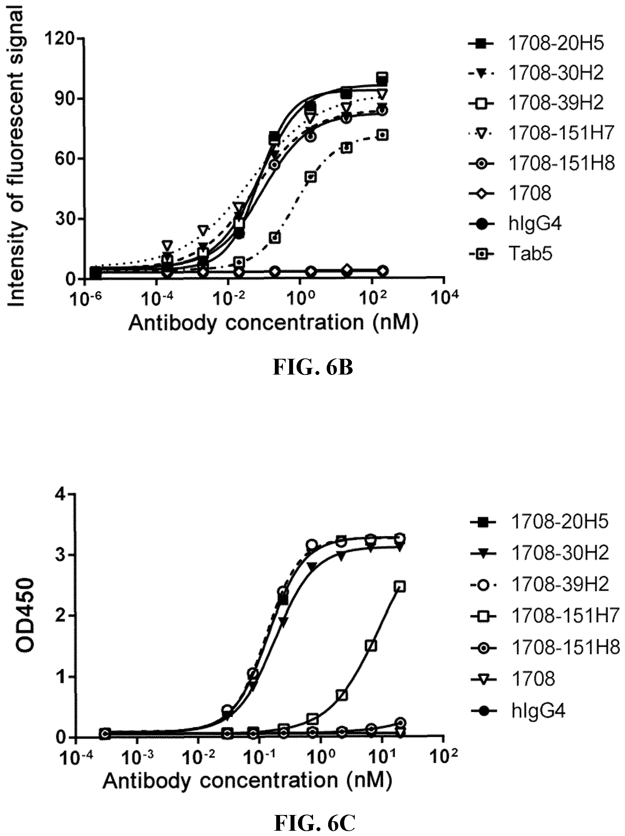

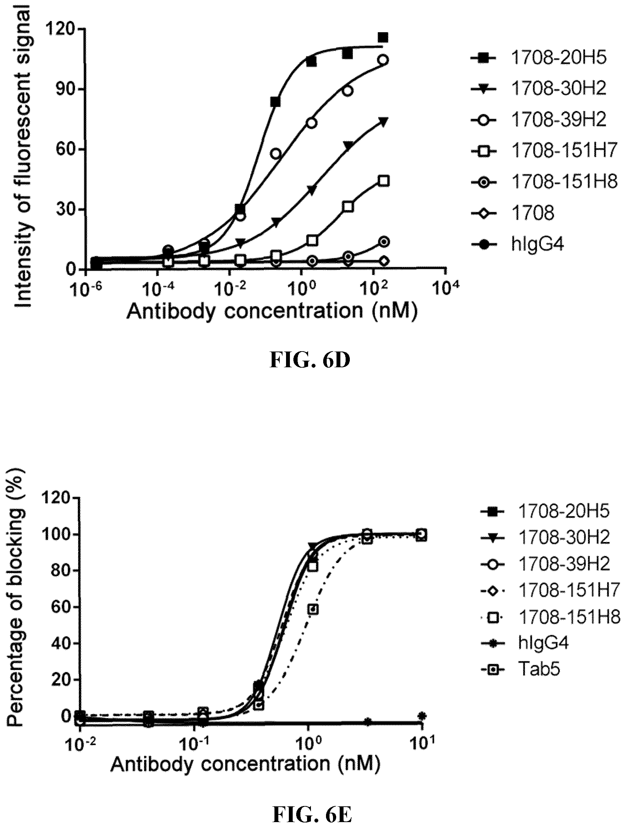

Binding of the humanized anti-PVRIG/TIGIT bispecific antibodies to human and cynomolgus monkey PVRIG and their blocking of the ligand of human PVRIG were detected according to the methods of Examples 4, 5 and 6. The results are shown in Table 29 and FIGs. 6A-6E. The results show that each humanized bispecific antibody can bind to human PVRIG and block the binding of PVRIG to PVRL2. 1708-151H8 shows weak binding to cynomolgus monkey PVRIG.

**Table 29. The binding of humanized bispecific antibodies to PVRIG and their blocking of the ligand**

| Antibody No. | Binding to human PVRIG recombinant protein EC₅₀ (nM) | Binding to cells overexpressing human PVRIG EC₅₀ (nM) | Binding to cynomolgus monkey PVRIG recombinant protein EC₅₀ (nM) | Binding to cells overexpressing cynomolgus monkey PVRIG EC₅₀ (nM) | Blocking binding of human PVRIG to human PVRL2 IC₅₀ (nM) |
|---|---|---|---|---|---|
| 1708-20H5 | 2.466 | 0.075 | 0.144 | 0.070 | 0.628 |
| 1708-30H2 | 0.320 | 0.056 | 0.184 | 3.540 | 0.552 |
| 1708-39H2 | 1.805 | 0.079 | 0.133 | 0.293 | 0.619 |
| 1708-151H7 | 0.679 | 0.043 | 9.442 | 11.470 | 0.598 |
| 1708-151H8 | 0.390 | 0.094 | N.A. | N.A. | 0.654 |
| 1708 | No binding | No binding | No binding | No binding | Not tested |
| Tab5 | 1.407 | 0.789 | No binding | No binding | 0.964 |
| IgG4 | No binding | No binding | No binding | No binding | No blocking |

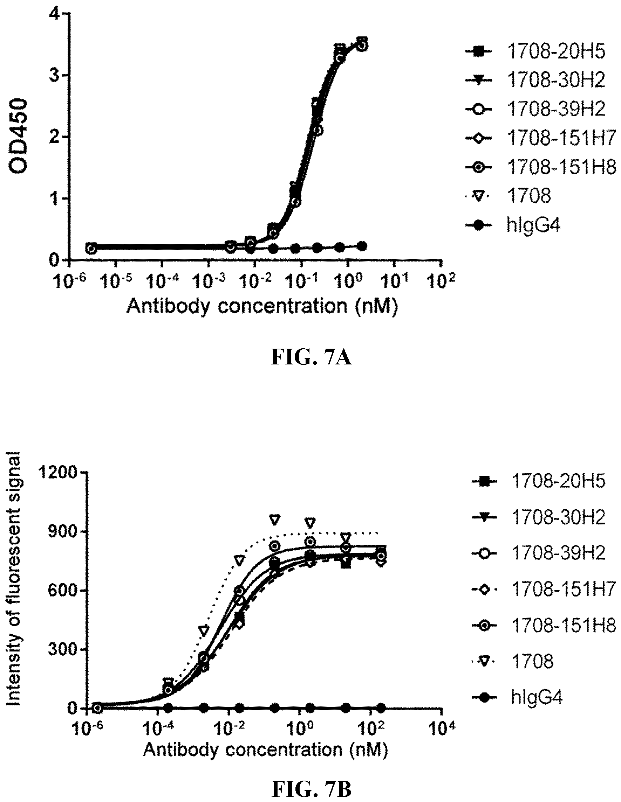

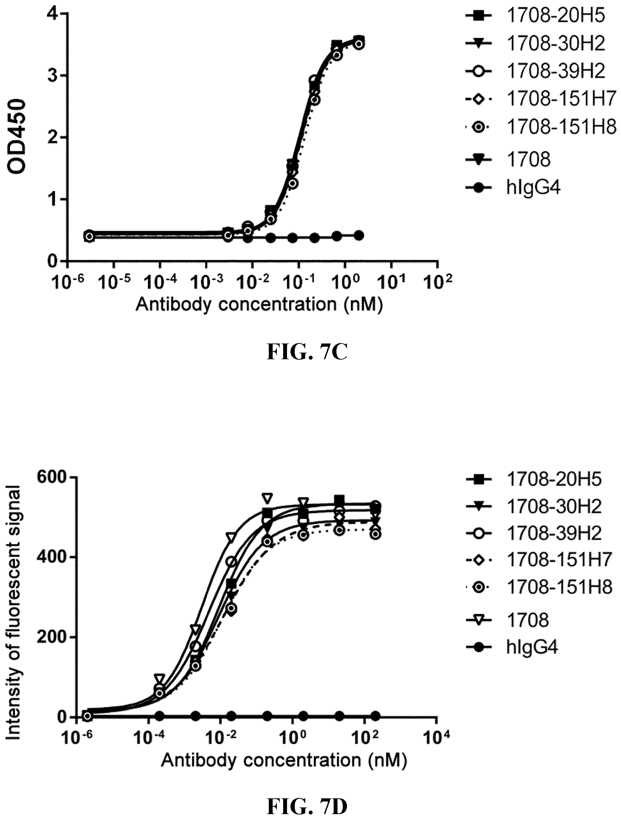

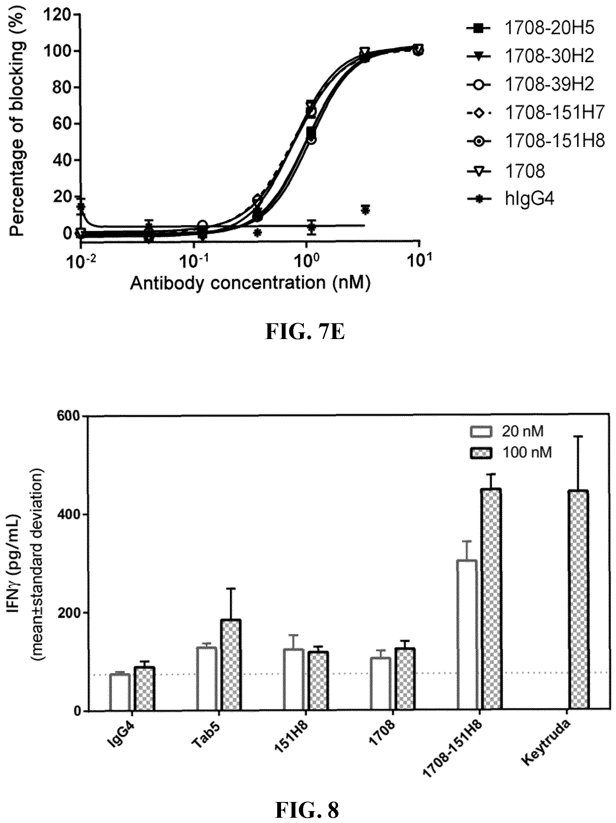

Similarly to Examples 4, 5 and 6, the binding of humanized anti-PVRIG/TIGIT bispecific antibodies to human and cynomolgus monkey TIGIT and blocking of binding of human TIGIT to ligand were detected, where PVRIG protein was replaced with TIGIT and PVRL2 was replaced with PVR. The results are shown in Table 30 and FIGs. 7A-7E. The results show that each diabody can bind to human and cynomolgus monkey TIGIT to block the binding of TIGIT to PVR.

**Table 30. The binding of humanized bispecific antibodies to TIGIT and their blocking of the ligand**

| Antibody No. | Binding to human TIGIT recombinant protein EC₅₀ (nM) | Binding to cells overexpressing human TIGIT EC₅₀ (nM) | Binding to cynomolgus monkey TIGIT recombinant protein EC₅₀ (nM) | Binding to cells overexpressing cynomolgus monkey TIGIT EC₅₀ (nM) | Blocking binding of human TIGIT to human PVR IC₅₀ (nM) |
|---|---|---|---|---|---|
| 1708-20H5 | 0.145 | 0.010 | 0.112 | 0.009 | 1.018 |
| 1708-30H2 | 0.153 | 0.010 | 0.114 | 0.009 | 1.014 |
| 1708-39H2 | 0.135 | 0.005 | 0.105 | 0.005 | 0.812 |
| 1708-151H7 | 0.160 | 0.012 | 0.119 | 0.012 | 0.773 |
| 1708-151H8 | 0.184 | 0.006 | 0.135 | 0.011 | 1.087 |
| 1708 | 0.133 | 0.0027 | 0.104 | 0.003 | 0.779 |
| Tab5 | No binding | No binding | No binding | No binding | Not tested |
| IgG4 | No binding | No binding | No binding | No binding | No blocking |

The affinity of the humanized bispecific antibodies for human PVRIG, cynomolgus monkey PVRIG and human TIGIT was detected using Biacore. Humanized bispecific antibodies were each captured on a Protein A biosensor chip (GE lifesciences, 29127557) of a Biacore instrument (Biacore X100, GE), and then human PVRIG antigen (AcroBiosystem, PVG-H52H4), cynomolgus monkey PVRIG antigen (SEQ ID NO: 1), or human TIGIT antigen (AcroBiosystem, TIT-H52H3) at a series of concentration gradients each flowed over the chip surface. The reaction signals were detected in real time using a Biacore instrument (Biacore X100, GE) to obtain association and dissociation curves. The data obtained from the experiment were fitted using the (1:1) Binding model with the BiacoreX100 evaluation software 2.0 GE to obtain affinity values (see Table 31).

**Table 31. Affinity of humanized bispecific antibodies for human PVRIG, cynomolgus monkey PVRIG and human TIGIT**

| Antibody No. | Antigen | kon (1/Ms) | koff (1/s) | KD (M) |
|---|---|---|---|---|
| 1708-20H5 | Human PVRIG | 1.67E+07 | 1.30E-04 | 7.82E-12 |
| 1708-30H2 | | 1.29E+07 | 9.12E-03 | 7.06E-10 |
| 1708-39H2 | | 7.90E+06 | 2.76E-04 | 3.49E-11 |
| 1708-151H7 | | 6.06E+06 | 7.57E-04 | 1.25E-10 |
| 1708-20H5 | Human TIGIT | 1.97E+06 | 1.50E-04 | 7.63E-11 |
| 1708-30H2 | | 3.08E+06 | 1.58E-04 | 5.12E-11 |
| 1708-39H2 | | 1.39E+06 | 1.13E-04 | 8.08E-11 |
| 1708-151H7 | | 1.40E+06 | 1.16E-04 | 8.28E-11 |
| 1708-20H5 | Cynomolgus monkey PVRIG | 2.56E+07 | 1.81E-01 | 7.05E-09 |
| 1708-30H2 | | 1.82E+07 | 5.81E-01 | 3.19E-08 |
| 1708-39H2 | | 8.48E+07 | 2.52E+00 | 2.97E-08 |

### Example 20: Mixed Lymphocyte Reaction (MLR) Experiment of Humanized Anti-PVRIG/TIGIT Bispecific Antibody

The ability of the humanized anti-PVRIG/TIGIT bispecific antibody to activate T cells was detected according to the method of Example 10. The results of the experiment are shown in FIG. 8 and Table 32. The results show that the humanized anti-PVRIG/TIGIT bispecific antibody 1708-151H8 has a significant capacity to activate T cells and promote the secretion of IFNγ by T cells. Importantly, the activity of the bispecific antibody is stronger than that of the anti-PVRIG antibody 151H8 alone or the anti-TIGIT antibody 1708 alone.

**Table 32. The mixed lymphocyte reaction IFNγ secretion levels for the humanized bispecific antibody**

| Antibody No. | IFNγ (pg/mL) (mean ± standard deviation) | |
|---|---|---|
| | 20 nM antibody | 100 nM antibody |
| IgG4 | 74±5 | 89±12 |
| 151H8 | 124±29 | 118±11 |
| 1708 | 106±16 | 125±16 |
| 1708-151H8 | 303±40 | 448±40 |
| Tab5 | 128±8.9 | 185±63 |
| Keytruda | Not tested | 444±111 |

### Example 21: Evaluation of Anti-Tumor Effect of Anti-PVRIG/TIGIT Bispecific Antibodies in a Mouse Subcutaneous Xenograft Tumor Model of Human Melanoma A375 Mixed with Human PBMCs

To further explore the role of the bispecific antibody subtype in animal efficacy, in addition to the bispecific antibodies of the IgG4 subtype described above, corresponding antibodies of the IgG1 subtype were also synthesized for use in animal efficacy test. Other antibody sequences used in this experiment and not described previously are shown in Table 33.

**Table 33. Full-length sequences of first and second polypeptide chains of humanized anti-PVRIG/TIGIT bispecific antibodies of type IgG1**

| Antibody No. | Full-length amino acid sequences of first and second polypeptide chains | | Sequence No. |
|---|---|---|---|
| 1708-IgG1 (same as 1708, except the heavy chain constant region subtype was changed to IgG1) | HC | EVQLVQSGAEVKKPGASVKVSCKASGYTFTNYW MHWVRQAPGQGLEWMGRIDPDSTGSKYNEKFKT RVTMTRDTSTSTVYMELSSLRSEDTAVYYCAREGA YGYYFDYWGQGTLVTVSSASTKGPSVFPLAPSSKS TSGGTAALGCLVKDYFPEPVTVSWNSGALTSGVH TFPAVLQSSGLYSLSSVVTVPSSSLGTQTYICNVNH KPSNTKVDKKVEPKSCDKTHTCPPCPAPELLGGPS VFLFPPKPKDTLMISRTPEVTCVVVDVSHEDPEVK FNWYVDGVEVHNAKTKPREEQYNSTYRVVSVLT VLHQDWLNGKEYKCKVSNKALPAPIEKTISKAKG QPREPQVYTLPPSREEMTKNQVSLTCLVKGFYPSD IAVEWESNGQPENNYKTTPPVLDSDGSFFLYSKLT VDKSRWQQGNVFSCSVMHEALHNHYTQKSLSLSP GK | SEQ ID NO: 113 |
| | LC | Same as the light chain of 1708 | SEQ ID NO: 103 |
| 1708-151-IgG 1 (same as 1708-151, except the heavy chain constant region subtype was changed to IgG1) | First polypeptide chain | HVQLVESGGGSVQAGGSLRLSCVASASGFTYRPY CMAWFRQAPGKEREAVAGIDIFGGTTYADSVKGR FTASRDNAGFSLFLQMNDLKPEDTAMYYCAAGDS PDGRCPPLGQGLNYWGQGTQVTVSSGGGGSGGG GSEVQLVQSGAEVKKPGASVKVSCKASGYTFTNY WMHWVRQAPGQGLEWMGRIDPDSTGSKYNEKF KTRVTMTRDTSTSTVYMELSSLRSEDTAVYYCARE GAYGYYFDYWGQGTLVTVSSASTKGPSVFPLAPS SKSTSGGTAALGCLVKDYFPEPVTVSWNSGALTSG VHTFPAVLQSSGLYSLSSVVTVPSSSLGTQTYICNV NHKPSNTKVDKKVEPKSCDKTHTCPPCPAPELLG GPSVFLFPPKPKDTLMISRTPEVTCVVVDVSHEDPE VKFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFYP SDIAVEWESNGQPENNYKTTPPVLDSDGSFFLYSK LTVDKSRWQQGNVFSCSVMHEALHNHYTQKSLS LSPGK | SEQ ID NO: 114 |
| | Second polypeptide chain | Same as the light chain of 1708 | SEQ ID NO: 103 |

NCG mice, female, aged 4-8 weeks, weighing about 18-22 g, purchased from Jiangsu GemPharmatech Co., Ltd. All the NCG mice were kept in an SPF animal house with an IVC constant-temperature and constant-pressure system.

A375 cells were cultured in DMEM medium containing 10% fetal bovine serum (FBS). A375 cells in the exponential growth phase were collected and resuspended in HBSS to a concentration appropriate for subcutaneous tumor inoculation into NCG mice. The A375 cells used for co-culture were treated with Mitomycin C for 2 h and washed three times with PBS. Peripheral blood was collected from a normal human, and human PBMCs were isolated by density gradient centrifugation and counted. The PBMCs were then resuspended in RPMI1640 medium (containing IL2 and 10% FBS) to a concentration of 3 × 10⁶ cells/mL and co-cultured with the Mitomycin C-treated A375 cells. After 6 days of co-culture, PBMCs were harvested, and meanwhile freshly digested A375 cells were harvested. Each mouse was inoculated with 5 × 10⁵ PBMCs and 4 × 10⁶ A375 cells; the inoculation volume was 0.2 mL/mouse (containing 50% Matrigel); the cells were inoculated subcutaneously into the right side of each female NCG mouse. The mice were randomly divided, according to the body weight, into groups for drug administration. The detailed administration method, dose of administration and route of administration are shown in Table 34, and the day of the grouping and administration was day 0. Given that the molecular weights of the anti-PVRIG antibody and anti-TIGIT antibody are different, these doses ensured that the anti-PVRIG antibody and anti-TIGIT antibody had the same initial molar concentration.

**Table 34. Administration regimen**

| **Group** | **Administration group** | **N** | **Dose (mg/kg)** | **Administration regimen** | **Route of administration** |
|---|---|---|---|---|---|
| 1 | hIgG1 | 7 | 30 | Q2D | i.p. |
| 2 | 151-IgG4 | 7 | 16.1 | Q2D | i.p. |
| 3 | 1708-IgG1 | 7 | 30 | Q2D | i.p. |
| 4 | 151-IgG4 + 1708-IgG1 | 7 | 16.1 | Q2D | i.p. |
| | | | 30 | Q2D | i.p. |
| 5 | 1708-151 IgG1 | 7 | 35.8 | Q2D | i.p. |
| 6 | 1708-151 IgG4 | 7 | 35.8 | Q2D | i.p. |

| | | | | | |
|---|---|---|---|---|---|
| (Note: N: number of animals used; i.p.: intraperitoneal injection; Q2D: once every two days; volume for administration: adjusted according to body weight of tumor-bearing mouse (0.1 mL/10 g).) | | | | | |

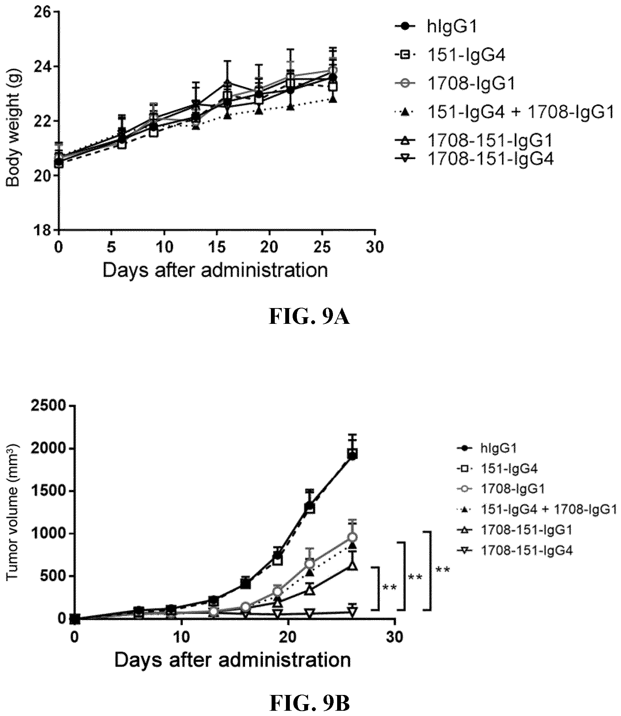

After the start of administration, the body weight and tumor volume of the mice were measured 2 times a week. The results are shown in Tables 35-36 and FIGs. 9A-9B.

**Table 35. Effect of anti-PVRIG/TIGIT bispecific antibodies on body weight of mice**

| Group | Average body weight on day 0 (g) | Average body weight on day 26 (g) | Rate of change in body weight (%) |
|---|---|---|---|
| | (mean ± standard deviation) | (mean ± standard deviation) | (mean ± standard deviation) |
| 1 | 20.5±0.42 | 23.60±0.64 | 15.21±2.88 |
| 2 | 20.43±0.4 | 23.77±0.74 | 16.54±2.57 |
| 3 | 20.65±0.48 | 23.85±0.45 | 15.71±2.08 |
| 4 | 20.69±0.51 | 22.81±0.78 | 10.14±1.45 |
| 5 | 20.65±0.56 | 23.54±1.14 | 13.77±3.26 |
| 6 | 20.65±0.48 | 23.8±0.76 | 15.28±2.58 |

**Table 36. The anti-tumor effects of the anti-PVRIG/TIGIT bispecific antibodies in the human A375 tumor mouse model**

| Group | Tumor volume at day 0 (mm³) | Tumor volume at day 26 (mm³) (mean ± standard deviation) | TGI (%) | T/C (%) | p value |
|---|---|---|---|---|---|
| 1 | 0±0 | 1913.62±188.23 | -- | -- | -- |
| 2 | 0±0 | 1942.70±223.36 | -1.52 | 101.52 | 0.916 |
| 3 | 0±0 | 958.83±204.39 | 49.89 | 50.11 | <0.001××× |
| 4 | 0±0 | 876.21±243.70 | 54.21 | 45.79 | <0.001××× |
| 5 | 0±0 | 629.64±163.74 | 67.10 | 32.90 | <0.001××× |
| 6 | 0±0 | 79.99±36.57 | 95.82 | 4.18 | <0.001××× |

| | | | | | |
|---|---|---|---|---|---|
| (Note: ×*P* < 0.05, ××*P* < 0.01, and ×××*P* < 0.001 are considered to have significant differences compared with the control group (hIgG1).) | | | | | |

At the end of the experiment (day 26 post-administration), the anti-PVRIG antibody 151 single drug group showed no significant difference compared with the control group. In the anti-TIGIT antibody 1708-IgG1 single drug group, the anti-PVRIG antibody 151 and anti-TIGIT antibody 1708-IgG1 combination group, and the 1708-151-IgG1 diabody group, the tumor volume was reduced. The 1708-151-IgG4 diabody group could even completely inhibit tumor growth, exhibiting significant difference from the other groups (see FIG. 9B).

The mice were randomly divided, according to the body weight, into groups for drug administration. The detailed administration method, dose of administration and route of administration are shown in Table 37, and the day of the grouping and administration was day 0.

**Table 37. Administration regimen**

| **Group** | **Administration group** | **Number** | **Dose (mg/kg)** | **Administration regimen** | **Route of administration** |
|---|---|---|---|---|---|
| 1 | hIgG4 | 7 | 35.8 | Q2D | i.p. |
| 2 | 1708-30H2 IgG4 | 7 | 12 | Q2D | i.p. |
| 3 | 1708-151H7 IgG4 | 7 | 12 | Q2D | i.p. |

| | | | | | |
|---|---|---|---|---|---|
| (Note: N: number of animals used; i.p.: intraperitoneal injection; Q2D: once every two days; volume for administration: adjusted according to body weight of tumor-bearing mouse (0.1 mL/10 g).) | | | | | |

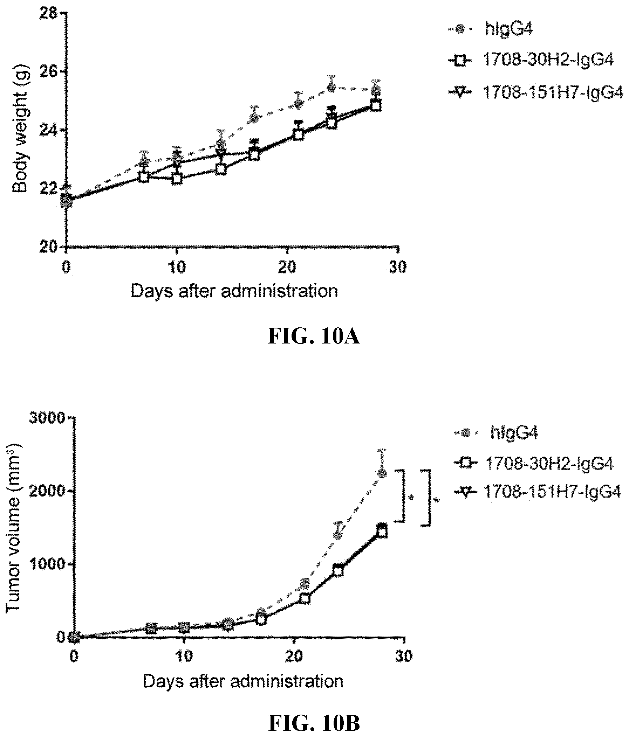

After the start of administration, the body weight and tumor volume of the mice were measured 2 times a week. The results are shown in Tables 38-39 and FIGs. 10A-10B.

**Table 38. Effect of anti-P RIG/TIGIT bispecific antibodies on body weight of mice**

| Group | Average body weight on day 0 (g) | Average body weight on day 28 (g) | Rate of change in body weight (%) |
|---|---|---|---|
| | (mean ± standard deviation) | (mean ± standard deviation) | (mean ± standard deviation) |
| 1 | 21.51±0.53 | 25.38±0.32 | 18.38±3.27 |
| 2 | 21.57±0.55 | 24.82±0.46 | 15.34±2.18 |
| 3 | 21.63±0.47 | 24.87±0.36 | 15.15±1.38 |

**Table 39. Anti-tumor effect of anti-PVRIG/TIGIT bispecific antibodies in mouse human-derived A375 tumor model**

| Group | Tumor volume at day 0 (mm³) | Tumor volume at day 28 (mm³) (mean ± standard deviation) | TGI (%) | T/C (%) | p value |
|---|---|---|---|---|---|
| 1 | 0±0 | 2239.26±322.87 | -- | -- | -- |
| 2 | 0±0 | 1435.36±117.61 | 35.90 | 64.10 | <0.05× |
| 3 | 0±0 | 1468.96±67.07 | 34.40 | 65.60 | <0.05× |

| | | | | | |
|---|---|---|---|---|---|
| (Note: ×*P* < 0.05, ××*P* < 0.01, and ×××*P* < 0.001 are considered to have significant differences compared with the control group (hIgG1).) | | | | | |

At the end of the experiment (day 28 post administration), both the 1708-30H2 IgG4 and 1708-151H7 IgG4 diabody groups were effective in inhibiting tumor growth at low doses compared with the control group, and exhibited significant difference from the control group (see FIG. 10A and FIG. 10B).

### Preparation Examples-Anti-PVRIG/TIGIT Bispecific Antibody Formulations

### The equipment, antibodies, and methods used in the preparation of formulations and detection are shown below.

### 1. SEC molecular exclusion chromatography:

This is a method for analyzing the separation of a solute by the relative relationship between the pore size of the gel pores and the size of the polymer sample molecule coil. SEC% (SEC monomer content percentage) = A monomer / A total × 100% (A monomer represents the peak area of the main peak monomer in the sample, and A total represents the sum of all peak areas).

Instrument for SEC determination: Agilent HPLC 1260; column: waters, XBridge BEH200Å SEC (7.8 × 300 mm 3.5 µm).

### 2. NR-CE (also known as CE-SDS (NR)) capillary gel electrophoresis:

This is a method of moving the gel into a capillary as a supporting medium for electrophoresis and performing separation according to the molecular weight of the sample under a certain voltage.

Non-reduced CE purity percentage = A main peak/A total × 100% (A main peak is the peak area of the main peak in the sample, and A total is the sum of all peak areas). Instrument for CE determination: Beckman capillary electrophoresis apparatus, model: plus800.

### 3. iCIEF imaged capillary isoelectric focusing electrophoresis:

This is a technique for separation according to the difference in isoelectric points (pI) of proteins.

iCIEF neutral peak content percentage = neutral peak area/total area × 100% (total area represents the sum of areas of acidic, neutral and basic peaks).

Instrument for iCIEF determination: simple protein, model: muarice.

### 4. Osmotic pressure determination:

The freezing point method is used for determining the osmotic pressure. On the basis of the directly proportional relation between the freezing point depression value and the molar concentration of the solution, the freezing point of a solution is determined by using a high-sensitivity temperature-sensing element, and then converted into the osmotic pressure through electric quantity.

Instrument for osmotic pressure determination: Loser, model: OM815.

### 5. Protein concentration determination:

The anti-PVRIG/TIGIT bispecific antibody used in the following preparation examples was 1708-30H2. The concentration of the bispecific antibody was calculated based on the protein concentration.

Instrument for protein concentration determination: ultraviolet-visible spectrophotometer; model: Nano Drop oneC; optical path length: 1 mm.

### Preparation Example 1: Buffer System and pH Value Screening for Anti-PVRIG/TIGIT Bispecific Antibody Formulations

The anti-PVRIG/TIGIT bispecific antibody (1708-30H2) formulations were prepared at a protein concentration of about 50 mg/mL using the following buffer systems:
1) 10 mM acetic acid-sodium acetate (AA for short), pH 5.0;
2) 10 mM AA, pH 5.5;
3) 10 mM succinic acid-sodium succinate (SA for short), pH 6.0;
4) 10 mM histidine-histidine hydrochloride (His-HCl for short), pH 5.5;
5) 10 mM His-HCl, pH 6.0;
6) 10 mM His-HCl, pH 6.5;
7) 10 mM disodium hydrogen phosphate-sodium dihydrogen phosphate (PB for short), pH 8.0;
8) 10 mM tromethamine-hydrochloride (Tris-HCl for short), pH 8.0; and
9) 10 mM Tris-HCl, pH 8.5.

The prepared formulations were subjected to sterile filtration and filled into containers, which were plugged and capped. The formulation samples were forcedly degraded at a high temperature of 40 °C, and the stability of the formulations was examined by taking appearance, SEC, CE-SDS (NR), and iCIEF as evaluation indexes.

The results of the experiment are shown in Table 40. The results show that after the formulation samples were stored at a high temperature of 40 °C for one month:
- in terms of appearance, a large number of particles appeared in the formulation of the SA buffer system group, which was inferior to formulations of other buffer system groups;
- in terms of purity, for the formulation samples with buffer system pH ≥ 8.0, the SEC monomers and CE-SDS (NR) were reduced greatly, ranging from 3.3% to 5.6% and 7.1% to 14.9%, respectively. In addition, for the formulation samples with buffer system pH = 5.0 or pH ≥ 8.0, the iCIEF neutral peak purity was reduced by more than 30%.

**Table 40. Experiment results of buffer system and pH value screening for anti-PVRIG/TIGIT antibody formulations**

| Buffer system No. | Storage conditions | Appearance | SEC% | | CE-SDS (NR) % | iCIEF% | |
|---|---|---|---|---|---|---|---|
| | | | Aggregate | Monomer | | Acidic peak | Neutral peak |
| 1 | 0h | Clear and transparent | 1.8 | 98.1 | 98.1 | 32.5 | 54.4 |
| | 40°CM1 | Particles | 2.8 | 95.7 | 93.7 | 38.6 | 23.3 |
| 2 | 0h | Clear and transparent | 1.7 | 98.2 | 98.0 | 33.1 | 53.4 |
| | 40°CM1 | Particles | 2.3 | 96.0 | 93.1 | 40.1 | 28.9 |
| 3 | 0h | Clear and slightly opalescent | 1.7 | 98.1 | 98.0 | 33.2 | 53.0 |
| | 40°CM1 | A large number of particles | 3.1 | 95.6 | 94.8 | 42.1 | 34.1 |
| 4 | 0h | Clear and slightly opalescent | 1.7 | 98.1 | 97.8 | 32.1 | 53.8 |
| | 40°CM1 | Particles | 2.4 | 96.1 | 94.2 | 37.4 | 28.8 |
| 5 | 0h | Clear and slightly opalescent | 1.6 | 98.3 | 97.9 | 31.8 | 54.0 |
| | 40°CM1 | Particles | 2.3 | 96.5 | 95.3 | 41.4 | 33.7 |
| 6 | 0h | Clear and slightly opalescent | 1.7 | 98.2 | 97.9 | 320 | 53.4 |
| | 40°CM1 | Particles | 2.4 | 96.6 | 95.4 | 46.5 | 33.6 |
| 7 | 0h | Clear and slightly opalescent | 1.5 | 98.4 | 97.5 | 33.7 | 53.0 |
| | 40°CM1 | Particles | 4.6 | 94.3 | 82.6 | 75.9 | 12.6 |
| 8 | 0h | Clear and slightly opalescent | 1.1 | 98.8 | 97.5 | 32.9 | 53.0 |
| | 40°CM1 | Particles | 3.4 | 95.5 | 90.4 | 64.8 | 19.7 |
| 9 | 0h | Clear and slightly opalescent | 1.0 | 98.9 | 97.5 | 33.2 | 52.7 |
| | 40°CM1 | Particles | 5.5 | 93.3 | 89.3 | 72.8 | 150 |

| | | | | | | | |
|---|---|---|---|---|---|---|---|
| Note: M represents a month, and 40 °C M1 represents storage at a constant temperature of 40 °C for 1 month | | | | | | | |

### Preparation Example 2: Protein Concentration Screening for Anti-PVRIG/TIGIT Bispecific Antibody Formulations

The formulations were prepared at protein concentrations of 50 mg/mL for low concentration and 100 mg/mL for high concentration, respectively, using the 10 mM His-HCl pH 6.0 buffer system. The prepared formulations were subjected to sterile filtration and filled into containers, which were plugged and capped. The samples were forcedly degraded at a high temperature of 40 °C, and the stability of the formulations was examined by taking SEC, CE-SDS (NR), and iCIEF as evaluation indexes.

The results of the experiment are shown in Table 41 and show that after the formulation samples were stored at a high temperature of 40 °C for 1 month, the SEC, CE-SDS (NR), and iCIEF values of the formulations at the high protein concentration were slightly lower in terms of the purity of the formulations at different concentrations.

**Table 41. Experimental results of stability of formulations at different protein concentrations**

| Protein concentration | conditions | SEC% | | CE-SDS (NR) % | iCIEF% | |
|---|---|---|---|---|---|---|
| | | Aggregate | Monomer | | Acidic peak | Neutral peak |
| 50 mg/mL | D0 | 1.6 | 98.3 | 97.9 | 31.8 | 54.0 |
| | 40°CM1 | 2.3 | 96.5 | 95.3 | 41.4 | 33.7 |
| 100 mg/mL | D0 | 1.6 | 98.3 | 97.9 | 31.8 | 54.0 |
| | 40°CM1 | 2.9 | 95.8 | 94.2 | 41.7 | 32.9 |

### Preparation Example 3: Surfactant Type and Concentration Screening for Anti-PVRIG/TIGIT Bispecific Antibody Formulations

The anti-PVRIG/TIGIT antibody (1708-30H2) formulations comprising 50 mg/mL antibody, 80 mg/mL sucrose, and surfactants at different concentrations were prepared using the 10 mM His-HCl pH 6.5 buffer system. The specific surfactant types and concentrations are as follows:
1) 0.2 mg/mL polysorbate 80;
2) 0.4 mg/mL polysorbate 80;
3) 0.6 mg/mL polysorbate 80;
4) 0.4 mg/mL polysorbate 20;
5) 0.4 mg/mL poloxamer 188.

The prepared formulations were subjected to sterile filtration and filled into containers, which were plugged and capped. The samples were forcedly degraded at a high temperature of 40 °C, and the stability of the formulations was examined by taking appearance, SEC, CE-SDS (NR), and iCIEF as evaluation indexes.

The results of the experiment are shown in Table 42. The results of the experiment show that the formulation in each experimental group had good stability.

**Table 42. Experimental results of stability of formulations comprising different surfactants**

| Surfactant No. | conditions | Appearance | SEC% | | CE-SDS (NR) % | iCIEF% | |
|---|---|---|---|---|---|---|---|
| | | | Aggregate | Monomer | | Acidic peak | Neutral peak |
| 1 | D0 | Clear and transparent | 0.7 | 99.3 | 98.3 | 28.9 | 56.8 |
| | 40°CM1 | Clear and transparent | 2.6 | 97.2 | 96.0 | 49.2 | 31.9 |
| 2 | D0 | Clear and transparent | 0.6 | 99.4 | 98.1 | 29.4 | 56.2 |
| | 40°CM1 | Clear and transparent | 2.9 | 96.9 | 95.1 | 49.2 | 32.5 |
| 3 | D0 | Clear and transparent | 0.6 | 99.4 | 98.3 | 29.4 | 56.5 |
| | 40°CM1 | Clear and transparent | 3.2 | 96.6 | 95.0 | 50.7 | 33.1 |
| 4 | D0 | Clear and transparent | 0.5 | 99.5 | 98.1 | 30.5 | 56.6 |
| | 40°CM1 | Clear and transparent | 1.6 | 98.3 | 96.3 | 46.3 | 33.7 |
| 5 | D0 | Clear and transparent | 0.6 | 99.4 | 98.2 | 29.1 | 56.6 |
| | 40°CM1 | Clear and transparent | 1.9 | 98.0 | 95.7 | 47.7 | 32.4 |

### Preparation Example 4: Sugar Concentration Screening for Anti-PVRIG/TIGIT Bispecific Antibody Formulations

The anti-PVRIG/TIGIT antibody (1708-30H2) formulations comprising 50 mg/mL antibody, 0.4 mg/mL polysorbate 80, and sucrose at different concentrations were prepared using the 10 mM His-HCl pH 5.5 buffer system. The specific sucrose concentrations are as follows:
1) 75 mg/mL sucrose;
2) 80 mg/mL sucrose.

The osmotic pressure of the prepared formulations was determined.

The results of the experiment are shown in Table 43. The results show that the osmotic pressure was in the isotonic range (280-320 mOsm) when the concentration of sucrose was 75 mg/mL to 80 mg/mL.

**Table 43. Experimental results of osmotic pressure of formulations at different sucrose concentrations**

| Sucrose No. | Sucrose concentration (mg/mL) | Osmotic pressure value of formulation (mOsm) |
|---|---|---|
| 1 | 75 | 290 |
| 2 | 80 | 300 |

### Preparation Example 5: Stability Test of Anti-PVRIG/TIGIT Bispecific Antibody in Different Buffer Systems

The anti-PVRIG/TIGIT antibody (1708-30H2) formulations comprising 50 mg/mL antibody, 80 mg/mL sucrose, and 0.4 mg/mL polysorbate 80 were prepared using the following buffer systems:
1) 10 mM AA, pH 5.5;
2) 10 mM His-HCl, pH 5.5.

The prepared formulations were subjected to sterile filtration and filled into containers, which were plugged and capped. The samples were forcedly degraded at a high temperature of 40 °C, and the stability of the formulations was examined by taking appearance, SEC, CE-SDS (NR), and iCIEF as evaluation indexes.

The results of the experiment are shown in Table 44 and show that after the formulation samples were stored at a high temperature of 40 °C for 1 month, the appearance, SEC, and CE-SDS (NR) of the formulations taking His-HCl and AA as buffer systems had no significant difference, but the iCIEF values showed that the neutral peak purity of the His-HCl buffer system group was higher than that of AA.

**Table 44. Experimental results of stability of formulations in different buffer systems**

| Buffer system No. | conditions | Appearance | SEC% | | CE-SDS (NR) % | iCIEF% | |
|---|---|---|---|---|---|---|---|
| | | | Aggregate | Monomer | | Acidic peak | Neutral peak |
| 1 | D0 | Clear and transparent | 0.4 | 99.6 | 98.2 | 31.6 | 55.5 |
| | 40°CM1 | Clear and transparent | 2.6 | 97.1 | 95.3 | 47.8 | 23.3 |
| 2 | D0 | Clear and transparent | 0.6 | 99.4 | 98.2 | 29.0 | 56.8 |
| | 40°CM1 | Clear and transparent | 2.9 | 96.8 | 95.3 | 42.9 | 27.3 |

### Preparation Example 6: Stability of Anti-PVRIG/TIGIT Bispecific Antibody Formulations at Different pH Values

The anti-PVRIG/TIGIT antibody (1708-30H2) formulations comprising 50 mg/mL antibody, 80 mg/mL sucrose, and 0.4 mg/mL polysorbate 80 were prepared using the following buffer systems:
1) 10 mM His-HCl, pH 5.5;
2) 10 mM His-HCl, pH 6.0;
3) 10 mM His-HCl, pH 6.5.

The prepared formulations were subjected to sterile filtration and filled into containers, which were plugged and capped. The samples were forcedly degraded at a high temperature of 40 °C, and the stability of the formulations was examined by taking appearance, SEC, CE-SDS (NR), and iCIEF as evaluation indexes.

The results of the experiment are shown in Table 45. The results of the experiment show that the formulation in each group had good stability when the pH of the His-HCl buffer was 5.5 to 6.5.

**Table 45. Experimental results of stability of formulations at different pH**

| Buffer system No. | conditions | Appearance | SEC% | | CE-SDS (NR) % | iCIEF% | |
|---|---|---|---|---|---|---|---|
| | | | Aggregate | Monomer | | Acidic peak | Neutral peak |
| 1 | D0 | Clear and transparent | 0.6 | 99.4 | 98.2 | 29.0 | 56.8 |
| | 40°CM1 | Clear and transparent | 2.9 | 96.8 | 95.3 | 42.9 | 27.3 |
| 2 | D0 | Clear and transparent | 0.6 | 99.4 | 98.2 | 29.8 | 56.4 |
| | 40°CM1 | Clear and transparent | 2.8 | 97.0 | 95.5 | 45.8 | 31.5 |
| 3 | D0 | Clear and transparent | 0.6 | 99.4 | 98.1 | 29.4 | 56.2 |
| | 40°CM1 | Clear and transparent | 2.9 | 96.9 | 95.1 | 49.2 | 32.5 |

### Preparation Example 7: Stability Test of Anti-PVRIG/TIGIT Bispecific Antibody Formulations

The anti-PVRIG/TIGIT antibody (1708-30H2) formulations comprising 50 mg/mL antibody, 0.4 mg/mL polysorbate 80, and 80 mg/mL sucrose were prepared using the 10 mM His-HCl pH 6.0 buffer system. The prepared formulations were subjected to sterile filtration and filled into containers, which were plugged and capped. The samples were stored at 25 °C and 2 °C to 8 °C for 6 months, and the stability of the formulations was examined by taking appearance, SEC, CE-SDS (NR), and iCIEF as evaluation indexes. The results of the experiment are shown in Table 46. The results show that the preferred formulations had good stability.

**Table 46. Experimental results of stability of formulations**

| conditions | Appearance | SEC% | | CE-SDS (NR) % | iCIEF% | |
|---|---|---|---|---|---|---|
| | | Aggregate | Monomer | | Acidic peak | Neutral peak |
| D0 | Clear and transparent | 0.6 | 99.4 | 98.2 | 29.8 | 56.4 |
| 25°CM6 | Clear and transparent | 2.2 | 97.6 | 97.0 | 42.3 | 39.6 |
| 2 °C to 8 °C M6 | Clear and transparent | 0.8 | 99.1 | 97.3 | 30.0 | 56.5 |

### Preparation Example 8: pH Drift Experiment of Anti-PVRIG/TIGIT Bispecific Antibody Formulations

Three batches of anti-PVRIG/TIGIT antibody (1708-30H2) formulations comprising 50 mg/mL antibody, 0.4 mg/mL polysorbate 80, and 80 mg/mL sucrose were prepared using the 10 mM His-HCl pH 6.0 buffer system. The pH values of the 3 batches of finished formulation products were measured and compared with the pH values of the buffers to examine the pH drift.

The results of the experiment are shown in Table 47. The results show that there was no statistically significant change in the pH drift of the formulations.

**Table 47. Experimental results of pH measurement of formulations**

| Batch No. | pH value of formulation | pH drift value |
|---|---|---|
| 1 | 6.07 | 0.07 |
| 2 | 6.07 | 0.07 |
| 3 | 6.07 | 0.07 |

### Preparation Example 9: Stability Test of Different Surfactants in Anti-PVRIG/TIGIT Bispecific Antibody Formulations

The anti-PVRIG/TIGIT antibody (1708-30H2) formulations comprising 50 mg/mL antibody, 80 mg/mL sucrose, and different types of surfactants were prepared using the 10 mM His-HCl pH 6.0 buffer system. The specific surfactant types and concentrations are as follows:
1) 0.4 mg/mL polysorbate 80;
2) 0.4 mg/mL polysorbate 20;
3) 0.4 mg/mL poloxamer 188.

The prepared formulations were subjected to sterile filtration and filled into containers, which were plugged and capped. The samples were stored at 2 °C to 8 °C for 12 months, and the stability of the formulations was examined by taking appearance, SEC, and iCIEF as evaluation indexes.

The results of the experiment are shown in Table 48. The results show that the formulations described above had good stability.

**Table 48. Experimental results of effects of different surfactants on stability of formulations**

| Surfactant No. | conditions | Appearance | SEC% | | iCIEF% | |
|---|---|---|---|---|---|---|
| | | | Aggregate | Monomer | Acidic peak | Neutral peak |
| 1 | D0 | Clear and transparent | 0.3 | 99.7 | 18.4 | 54.5 |
| | 2 °C to 8 °C M12 | Clear and transparent | 0.6 | 99.2 | 18.9 | 53.3 |
| 2 | D0 | Clear and transparent | 0.3 | 99.7 | 18.1 | 54.6 |
| | 2 °C to 8 °C M12 | Clear and transparent | 0.6 | 99.2 | 18.9 | 53.4 |
| 3 | D0 | Clear and transparent | 0.3 | 99.7 | 18.3 | 54.5 |
| | 2 °C to 8 °C M12 | Clear and transparent | 0.5 | 99.3 | 18.9 | 53.5 |

## Claims

1. A pharmaceutical composition comprising an anti-PVRIG/TIGIT bispecific antibody and a buffer, wherein:
the anti-PVRIG/TIGIT bispecific antibody comprises a first antigen-binding domain specifically binding to PVRIG and a second antigen-binding domain specifically binding to TIGIT;
the buffer is a histidine buffer or an acetate buffer;
preferably, the histidine buffer is a histidine-histidine hydrochloride buffer, or
preferably, the acetate buffer is an acetic acid-sodium acetate buffer.

2. The pharmaceutical composition according to claim 1, wherein the pharmaceutical composition has a pH of 5.0 to 6.5;
preferably, the pharmaceutical composition has a pH of 5.5 to 6.5;
more preferably, the pharmaceutical composition has a pH of 6.0±0.2.

3. The pharmaceutical composition according to claim 1 or 2, wherein the anti-PVRIG/TIGIT bispecific antibody is at a concentration of 1 mg/mL to 150 mg/mL; preferably, the anti-PVRIG/TIGIT bispecific antibody is at a concentration of 1 mg/mL to 100 mg/mL;
more preferably, the anti-PVRIG/TIGIT bispecific antibody is at a concentration of 50±5 mg/mL.

4. The pharmaceutical composition according to any one of claims 1 to 3, wherein the pharmaceutical composition comprises a surfactant;
preferably, the surfactant is polysorbate or poloxamer;
more preferably, the surfactant is polysorbate 80.

5. The pharmaceutical composition according to claim 4, wherein the surfactant is at a concentration of 0.05 mg/mL to 1.0 mg/mL;
preferably, the surfactant is at a concentration of 0.2 mg/mL to 0.6 mg/mL;
more preferably, the surfactant is at a concentration of 0.4±0.1 mg/mL.

6. The pharmaceutical composition according to any one of claims 1 to 5, wherein the pharmaceutical composition comprises an osmotic pressure regulator;
preferably, the osmotic pressure regulator is selected from one or more of the group consisting of sucrose, trehalose, sorbitol, arginine, glycine, and sodium chloride;
more preferably, the osmotic pressure regulator is sucrose.

7. The pharmaceutical composition according to claim 6, wherein the osmotic pressure regulator is at a concentration of 70 mg/mL to 100 mg/mL;
preferably, the osmotic pressure regulator is at a concentration of 70 mg/mL to 90 mg/mL;
more preferably, the osmotic pressure regulator is at a concentration of 80±5 mg/mL.

8. The pharmaceutical composition according to any one of claims 1 to 7, wherein the buffer is at a concentration of 5 mM to 100 mM;
preferably, the buffer is at a concentration of 10 mM to 30 mM;
more preferably, the buffer is at a concentration of 10±5 mM.

9. The pharmaceutical composition according to any of claims 1 to 8, wherein:
the first antigen-binding domain specifically binding to PVRIG comprises at least one immunoglobulin single variable domain, and the immunoglobulin single variable domain comprises a CDR1, a CDR2, and a CDR3, wherein the CDR1, CDR2, and CDR3 comprise the amino acid sequences of a CDR1, a CDR2, and a CDR3 set forth in any one of SEQ ID NOs: 3 and 80-84, respectively;
preferably, the CDR1, CDR2, and CDR3 of the immunoglobulin single variable domain are defined according to the Kabat numbering scheme, wherein the CDR1 comprises the amino acid sequence of SEQ ID NO: 10, the CDR2 comprises the amino acid sequence of SEQ ID NO: 11, and the CDR3 comprises the amino acid sequence of SEQ ID NO: 151;
more preferably, the immunoglobulin single variable domain comprises the amino acid sequence of SEQ ID NO: 81.

10. The pharmaceutical composition according to any one of claims 1 to 9, wherein the second antigen-binding domain specifically binding to TIGIT comprises a heavy chain variable region (VH) and a light chain variable region (VL), wherein:
the VH comprises a HCDR1, a HCDR2, and a HCDR3, wherein the HCDR1 comprises the amino acid sequence of SEQ ID NO: 121, the HCDR2 comprises the amino acid sequence of SEQ ID NO: 122, and the HCDR3 comprises the amino acid sequence of SEQ ID NO: 123; and
the VL comprises a LCDR1, a LCDR2, and a LCDR3, wherein the LCDR1 comprises the amino acid sequence of SEQ ID NO: 124, the LCDR2 comprises the amino acid sequence of SEQ ID NO: 125, and the LCDR3 comprises the amino acid sequence of SEQ ID NO: 126;
preferably, the VH comprises the amino acid sequence of SEQ ID NO: 145, and the VL comprises the amino acid sequence of SEQ ID NO: 149;
more preferably, the second antigen-binding domain specifically binding to TIGIT comprises a heavy chain and a light chain, wherein the heavy chain comprises the amino acid sequence of SEQ ID NO: 102, and the light chain comprises the amino acid sequence of SEQ ID NO: 103;
most preferably, the anti-PVRIG/TIGIT bispecific antibody comprises a first polypeptide chain and a second polypeptide chain, wherein the first polypeptide chain comprises the amino acid sequence of SEQ ID NO: 109, and the second polypeptide chain comprises the amino acid sequence of SEQ ID NO: 103.

11. The pharmaceutical composition according to any one of claims 1 to 10, comprising the following components:
(a) 1 mg/mL to 100 mg/mL of the anti-PVRIG/TIGIT bispecific antibody;
(b) 0.2 mg/mL to 0.6 mg/mL polysorbate 80;
(c) 70 mg/mL to 90 mg/mL sucrose; and
(d) 10 mM to 30 mM histidine buffer; the pharmaceutical composition has a pH of 5.5 to 6.5;
preferably, the pharmaceutical composition comprises the following components:
(a) 50 mg/mL to 100 mg/mL of the anti-PVRIG/TIGIT bispecific antibody;
(b) 0.2 mg/mL to 0.6 mg/mL polysorbate 80;
(c) 75 mg/mL to 80 mg/mL sucrose; and
(d) 10 mM histidine-histidine hydrochloride buffer; the pharmaceutical composition has a pH of 5.5 to 6.5;
more preferably, the pharmaceutical composition comprises the following components:
(a) 50±5 mg/mL of the anti-PVRIG/TIGIT bispecific antibody, wherein the anti-PVRIG/TIGIT bispecific antibody comprises a first polypeptide chain having the amino acid sequence set forth in SEQ ID NO: 109 and a second polypeptide chain having the amino acid sequence set forth in SEQ ID NO: 103;
(b) 0.4±0.1 mg/mL polysorbate 80;
(c) 80±5 mg/mL sucrose; and
(d) 10±5 mM histidine-histidine hydrochloride buffer; the pharmaceutical composition has a pH of 6.0±0.2.

12. A lyophilized formulation, wherein the lyophilized formulation can be reconstituted to form the pharmaceutical composition according to any one of claims 1 to 11.

13. A method for preparing a lyophilized formulation, comprising the step of lyophilizing the pharmaceutical composition according to any one of claims 1 to 11.

14. A lyophilized formulation, wherein the formulation is obtained by the method according to claim 13.

15. The pharmaceutical composition according to any one of claims 1 to 11, being a formulation for intravenous, subcutaneous, intraperitoneal, or intramuscular injection, preferably a formulation for intravenous injection.

16. A method for treating a disease, comprising:
administering to a subject a therapeutically effective amount of the pharmaceutical composition according to any one of claims 1 to 11 and 15 or the lyophilized formulation according to claim 12 or 14;
preferably, the disease is a proliferative disease, infection, or sepsis;
more preferably, the proliferative disease is a tumor;
most preferably, the tumor is selected from any one of the following: lung cancer, prostate cancer, breast cancer, head and neck cancer, esophagus cancer, gastric cancer, colorectal cancer, bladder cancer, cervical cancer, endometrial cancer, ovarian cancer, liver cancer, melanoma, renal cancer, squamous cell cancer, and hematological cancer.
